(19) Europäisches Patentamt
European Patent Office
Office européen des brevets

(11) Publication number: **0 114 002**
**B1**

(12) # EUROPEAN PATENT SPECIFICATION

(45) Date of publication of patent specification: **04.03.87**

(21) Application number: **83308029.4**

(22) Date of filing: **29.12.83**

(51) Int. Cl.[4]: **C 07 D 495/06,**
A 61 K 31/415, C 07 C 109/02
// C07C149/40, C07D335/16
,(C07D495/06, 335:00, 231:00)

(54) **Benzothiopyrano(4,3,2,-cd)indazoles, their pharmaceutical compositions and methods for their production.**

(30) Priority: **06.01.83 US 456162**
**12.12.83 US 559402**

(43) Date of publication of application:
**25.07.84 Bulletin 84/30**

(45) Publication of the grant of the patent:
**04.03.87 Bulletin 87/10**

(84) Designated Contracting States:
**AT BE CH DE FR GB IT LI LU NL SE**

(56) References cited:
**US-A-3 505 341**
**US-A-4 197 249**

(73) Proprietor: **WARNER-LAMBERT COMPANY**
**201 Tabor Road**
**Morris Plains New Jersey 07950 (US)**

(72) Inventor: **Elslager, Edward F.**
**4081 Thornoaks Drive**
**Ann Arbor, MI 48104 (US)**
Inventor: **Werbel, Leslie M.**
**1570 Covington Drive**
**Ann Arbor, MI 48103 (US)**
Inventor: **Ortwine, Daniel F.**
**663 Springbrook Court**
**Saline, MI 48176 (US)**
Inventor: **Worth, Donald F.**
**1156 Ravenwood**
**Ann Arbor, MI 48103 (US)**
Inventor: **Showalter, Howard D.H.**
**900 Patricia**
**Ann Arbor, MI 48103 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 114 002 B1

Courier Press, Leamington Spa, England.

**Description**

TECHNICAL FIELD

The invention relates to novel substituted benzothiopyrano[4,3,2-*cd*]indazoles, to methods for their production, to pharmaceutical compositions comprising the compounds, and to methods of treatment using the compounds in dosage form. The compounds of the invention have pharmacological properties and are useful antibacterial agents, antifungal agents, and antitumour agents.

BACKGROUND OF THE INVENTION

The benzothiopyrano[4,3,2-*cd*]indazole ring system

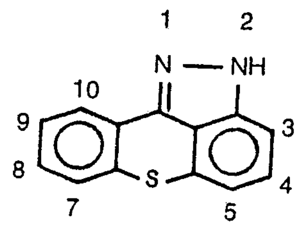

is known from F. Ullman and Otto van Glenck, *Ber., 49;* 2489 (1916) and from US Patent 3,505,341 to Elslager, Worth and Howells. There is no prior art in which this ring system has a nitrogen-containing functionality in the 5-position, nor is there any mention of 7,8,9,10-(OH or O-alkyl).

SUMMARY OF THE INVENTION

The invention in one aspect relates to benzothiopyrano[4,3,2-*cd*]indazole compounds having, in free base form, the structural formula 1:

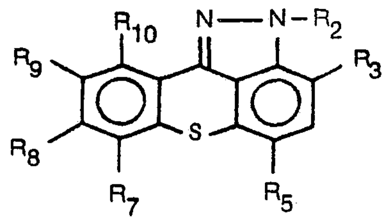

1

and to the pharmaceutically acceptable salts thereof, wherein the substituents $R_3$, $R_7$, $R_8$, $R_9$ and $R_{10}$ represent hydrogen, hydroxy or alkoxy of from 1 to 4 carbon atoms, hereinafter referred to as lower alkoxy; wherein $R_2$ is ANR′R″ wherein A is a straight or branched alkylene chain of from 2 to 5 carbon atoms, optionally substituted with hydroxyl; wherein R′ and R″ are hydrogen or straight or branched alkyl of from 1 to 4 carbon atoms, optionally substituted with hydroxyl, R′ and R″ taken together also representing

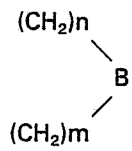

wherein n and m are each an integer from 2 to 3 and B is a direct bond or O, S, or N‴ wherein R‴ is hydrogen or straight or branched alkyl of from 1 to 4 carbon atoms, optionally substituted with hydroxyl; and wherein $R_5$ is nitro, $NH_2$,

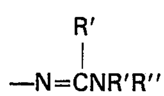

NHR′, $NR'R_2$ or NR′R⁗ wherein R⁗ is from 1 to 4 carbon acyl, chloroacetyl, or

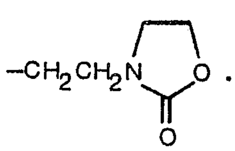

The compounds of the invention form pharmaceutically acceptable salts with both organic and inorganic acids. Examples of suitable acids for salt formation are hydrochloric, sulfuric, phosphoric, acetic, citric, oxalic, malonic, salicyclic, malic, fumaric, succinic, ascorbic, maleic, methanesulfonic, isethionic,

lactic, gluconic, glucuronic, sulfamic, benzoic, tartaric, pamoic, and the like. The salts are prepared by contacting the free base form with an equivalent amount of the desired acid in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention.

The compounds of the invention can exist in unsolvated as well as solvated forms, including hydrated forms. In general, the solvated forms with pharmaceutically acceptable solvents such as water, ethanol, and the like are equivalent to the unsolvated forms for purposes of the invention.

The term halogen as used herein is intended to include fluorine, chlorine, bromine, and iodine.

The invention in another aspect relates to benzothiopyrano[4,3,2-*cd*]indazole compounds having the structural formula 2:

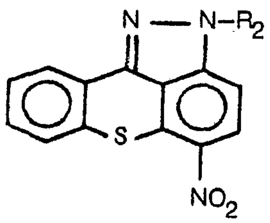

2

and the pharmaceutically acceptable salts thereof; wherein $R_2$ has the above meaning.

The invention in another embodiment relates to benzothiopyrano[4,3,2-*cd*]indazole compounds having the structural formual 2b:

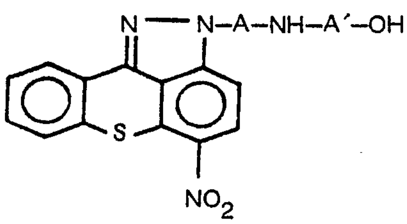

2b

and the pharmaceutically acceptable salts thereof; wherein A has the above meaning and A′ is a straight or branched alkylene chain of 2 to 5 carbon atoms.

The invention in another aspect relates to benzothiopyrano[4,3,2-*cd*]indazole compounds having the structural formula 3:

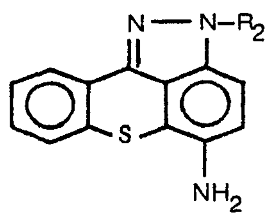

3

and the pharmaceutically acceptable salts thereof; wherein $R_2$ has the above meaning.

The invention in another aspect relates to benzothiopyrano[4,3,2-*cd*]indazole compounds having the structural formula 4a:

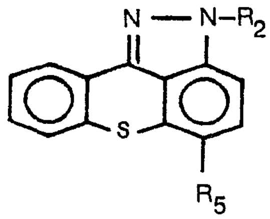

4a

and the pharmaceutically acceptable salts thereof; wherein $R_2$ and $R_5$ have the above meaning.

The invention in still another aspect relates to benzothiopyrano[4,3,2-*cd*]indazole compounds having the structural formula 5a:

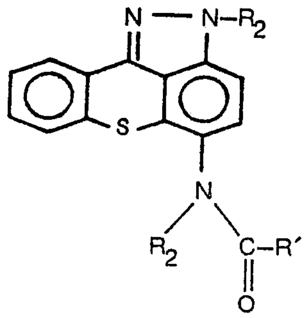

5a

and the pharmaceutically acceptable salts thereof; wherein $R_2$ and R' have the above meaning.

The invention in another embodiment relates to benzothiopyrano[4,3,2-*cd*]indazole compounds having the structural formula 6:

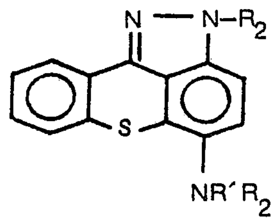

6

and the pharmaceutically acceptable salts thereof; wherein R' and $R_2$ have the above meaning.

The invention in another embodiment relates to benzothiopyrano[4,3,2-*cd*]indazole compounds having the structural formula 11a:

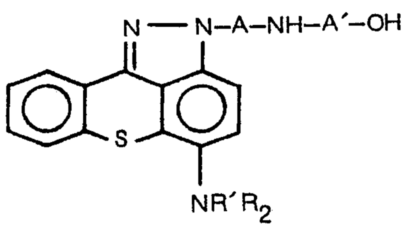

11a

and the pharmaceutically acceptable salts thereof; wherein A, A', R', and $R_2$ have the above meaning.

The invention in another embodiment relates to benzothiopyrano[4,3,2-*cd*]indazole compounds having the structural formula 16:

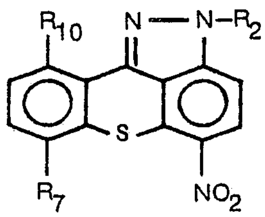

16

and the pharmaceutically acceptable salts thereof; wherein $R_2$ has the above meaning, and $R_7$ and $R_{10}$ are hydrogen, hydroxy or lower alkoxy, at least one of $R_7$ and $R_{10}$ being hydroxy or lower alkoxy.

The invention in another embodiment relates to benzothiopyrano[4,3,2-*cd*]indazole compounds having the structural formula 17:

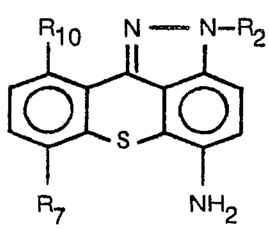

17

and the pharmaceutically acceptable salts thereof; wherein $R_2$ has the above meaning, and $R_7$ and $R_{10}$ are hydrogen, hydroxy or lower alkoxy, at least one of $R_7$ and $R_{10}$ being hydroxy or lower alkoxy.

The invention in another embodiment relates to benzothiopyrano[4,3,2-*cd*]indazole compounds having the structural formula 18:

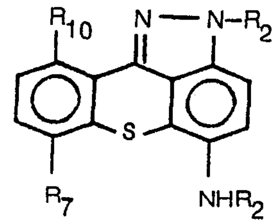

18

and the pharmaceutically acceptable salts thereof; wherein $R_2$ has the above meaning, and $R_7$ and $R_{10}$ are hydrogen, hydroxy or lower alkoxy, at least one of $R_7$ and $R_{10}$ being hydroxy or lower alkoxy.

The invention in another embodiment relates to benzothiopyrano[4,3,2-*cd*]indazole compounds having the structural formula 19:

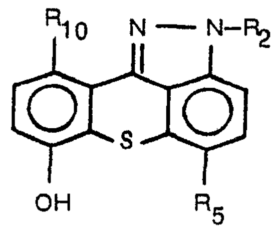

19

and the pharmaceutically acceptable salts thereof; wherein $R_2$ has the above meaning and $R_5$ is nitro amino or $NHR_2$.

The invention in another embodiment relates to benzothiopyrano[4,3,2-*cd*]indazole compounds having the structural formula 24:

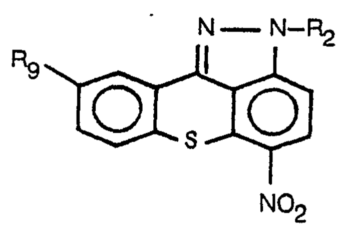

24

and the pharmaceutically acceptable salts thereof; wherein $R_2$ has the above meaning and $R_9$ is lower alkoxy.

The invention in another embodiment relates to benzothiopyrano[4,3,2-*cd*]indazole compounds having the structural formula 25:

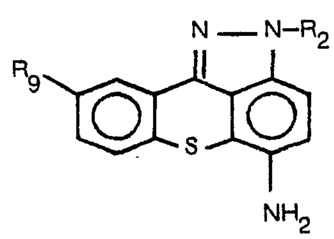

25

and the pharmaceutically acceptable salts thereof; wherein $R_2$ has the above meaning and $R_9$ is lower alkoxy.

The invention in another embodiment relates to benzothiopyrano[4,3,2-*cd*]indazole compounds having the structural formula 26:

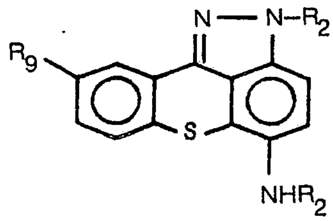

26

and the pharmaceutically acceptable salts thereof; wherein $R_2$ has the above meaning and $R_9$ is lower alkoxy.

The invention in another embodiment relates to benzothiopyrano[4,3,2-*cd*]indazole compounds having the structural formula 27:

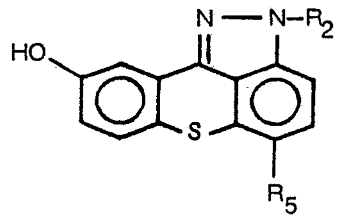

27

and the pharmaceutically acceptable salts thereof; wherein $R_2$ and $R_5$ have the above meaning.

In preferred embodiments of this invention, $R_2$ is diethylaminoethyl and

$R_5$ is $-NHCH_2CH_2NHCH_2CH_2OH$, or $R_2$ is $-CH_2CH_2NHCH_2CH_2OH$

and $R_5$ is $-NHCH_2CH_2NHCH_2CH_2OH$

or $R_2$ is diethylaminoethyl and $R_5$ is aminoethylamino. Moreover, preferred processes of this invention in which $R_2$ and $R_5$ have these definitions are described.

The invention in another aspect relates to compounds that are most preferred for their pharmacological properties, these compounds having the following names:

2-[[2-[[2-[2-(diethylamino)ethyl]-2*H*[1]benzothiopyrano[4,3,2-*cd*]indazol-5-yl]amino]ethyl]amino]ethanol;

2-[[2-[5-[[2-[(2-hydroxyethyl)amino]ethyl]amino]-2*H*[1]benzothiopyrano[4,3,2-*cd*]indazol-2-yl]ethyl]amino]ethanol; and

N-[2-[2-(diethylamino)ethyl]-2*H*[1]benzothiopyrano[4,3,2-*cd*]indazol-5-yl]-1,2-ethanediamine; and the pharmaceutically acceptable salts thereof.

The invention relates to further preferred species having the following names:

*N,N*-diethyl-5-nitro-2*H*[1]benzothiopyrano[4,3,2-*cd*]indazole-2-ethanamine;

5-nitro-2*H*[1]benzothiopyrano[4,3,2-*cd*]indazole-2-ethanamine;

2-[[2-(5-nitro-2*H*[1]benzothiopyrano[4,3,2-*cd*]indazol-2-yl)ethyl]amino]ethanol;

5-amino-*N,N*-diethyl-2*H*[1]benzothiopyrano[4,3,2-*cd*]indazole-2-ethanamine;

*N,N*-diethyl-9-methoxy-5-nitro-2*H*[1]benzothiopyrano[4,3,2-*cd*]indazole-2-ethanamine;

*N*-[2-[2-(diethylamino)ethyl]-2-*H*[1]benzothiopyrano[4,3,2-*cd*]indazol-5-yl]-1,3-propanediamine;

5-amino-*N,N*-dimethyl-2*H*[1]benzothiopyrano[4,3,2-*cd*]indazole-2-propanamine;

*N*'-[2-[2-(diethylamino)ethyl]-2*H*[1]benzothiopyrano[4,3,2-*cd*]indazol-5-yl]-*N,N*-dimethyl-methanimidamide;

3-[2-[[2-[2-(diethylamino)ethyl]-2*H*[1]benzothiopyrano[4,3,2-*cd*]indazol-5-yl]amino]ethyl]-2-oxazolidinone;

*N,N*-dimethyl-5-nitro-2*H*[1]benzothiopyrano[4,3,2-*cd*]indazole-2-propanamine;

2-[[2-(5-amino-2*H*[1]benzothiopyrano[4,3,2-*cd*]indazol-2-yl)ethyl]amino]ethanol;

2-[[2-[5-[[2-(diethylamino)ethyl]amino]-2*H*[1]benzothiopyrano[4,3,2-*cd*]indazol-2-yl]ethyl]amino]ethanol;

2-[[2-[[2-[2-(2-aminoethyl)-2*H*[1]benzothiopyrano[4,3,2-*cd*]indazol-5-yl]amino]ethyl]amino]ethanol;

5-Amino-2-[2-(diethylamino)ethyl]-2*H*[1]benzothiopyrano[4,3,2-*cd*]indazol-9-ol;

2-[2-(diethylamino)ethyl]-5-[[2-[(2-hydroxyethyl)amino]ethyl]amino]-2*H*[1]benzothiopyrano[4,3,2-*cd*]indazol-9-ol;

*N*-[2-[3-(dimethylamino)propyl]-2*H*[1]benzothiopyrano[4,3,2-*cd*]indazol-5-yl]-1,2-ethanediamine.
3-[2-[[2-[2-(diethylamino)ethyl]-9-hydroxy-2*H*[1]benzothiopyrano[4,3,2-*cd*]indazol-5-yl];
2-[2-(Diethylamino)ethyl]-5-[[2-[(2-hydroxyethyl]aminoethyl]amino-2*H*[1]benzothiopyrano[4,3,2-*cd*]indazol-9-ol;
*N,N*-Diethyl-7-methoxy-5-nitro-2*H*[1]benzothiopyrano[4,3,2-*cd*]indazole-2-ethanamine;
5-Amino-*N,N*-diethyl-7-methoxy-2*H*[1]benzothiopyrano[4,3,2-*cd*]indazole-2-ethanamine;
*N*-[2-[2-(Diethylamino)ethyl]-7-methoxy-2*H*[1]benzothiopyrano[4,3,2-*cd*]indazol-5-yl]-1,2-ethanediamine, hydrobromide salt;
5-[2-(Aminoethyl)amino]-2-[2-(diethylamino)ethyl]-2*H*[1]benzothiopyrano[4,3,2-*cd*]indazol-7-ol, hydrobromide salt;
5-Amino-2-[2-(diethylamino)ethyl]-2*H*[1]benzothiopyrano[4,3,2-*cd*]indazol-7-ol;
N-N-Diethyl-8-methoxy-5-nitro-2*H*[1]benzothiopyrano[4,3,2-*cd*]indazole-2-ethanamine;
5-Amino-*N,N*-diethyl-8-methoxy-2*H*[1]benzothiopyrano[4,3,2-*cd*]indazole-2-ethanamine;
*N*-[2-[2-(Diethylamino)ethyl]-8-methoxy-2*H*[1]benzothiopyrano[4,3,2-*cd*]indazol-5-yl]-1,2-ethanediamine, hydrobromide salt;
5-[2-(Aminoethyl)amino]-2-[2-diethylamino)ethyl]-2*H*[1]benzothiopyrano[4,3,2-*cd*]indazol-8-ol, hydrobromide salt;
5-Amino-2-[2-(diethylamino)ethyl]-2*H*[1]benzothiopyrano[4,3,2-*cd*]indazol-8-ol;
*N,N*-Diethyl-10-methoxy-5-nitro-2*H*[1]benzothiopyrano[4,3,2-*cd*]indazole-2-ethanamine;
5-Amino-*N,N*-diethyl-10-methoxy-2*H*[1]benzothiopyrano[4,3,2-*cd*]indazole-2-ethanamine;
*N*-[2-[2-(Diethylamino)ethyl]-10-methoxy-2*H*[1]benzothiopyrano[4,3,2-*cd*]indazol-5-yl]-1,2-ethanediamine, hydrobromide salt;
5-[2-(Aminoethyl)amino]-2-[2-diethylamino)ethyl]-2*H*[1]benzothiopyrano[4,3,2-*cd*]indazol-10-ol, hydrobromide salt;
5-Amino-2-[2-(diethylamino)ethyl]-2*H*[1]benzothiopyrano[4,3,2-*cd*]indazol-10-ol;
*N,N*-Diethyl-3-methoxy-5-nitro-2*H*[1]benzothiopyrano[4,3,2-*cd*]indazole-2-ethanamine;
5-Amino-*N,N*-diethyl-3-methoxy-2*H*[1]benzothiopyrano[4,3,2-*cd*]indazole-2-ethanamine;
*N*-[2-[2-(Diethylamino)ethyl]-3-methoxy-2*H*[1]benzothiopyrano[4,3,2-*cd*]indazol-5-yl]-1,2-ethanediamine, hydrobromide salt;
5-[2-(Aminoethyl)amino]-2-[2-diethylamino)ethyl]-2*H*[1]benzothiopyrano[4,3,2-*cd*]indazol-3-ol, hydrobromide salt;
5-Amino-2-[2-(diethylamino)ethyl]-2*H*[1]benzothiopyrano[4,3,2-*cd*]indazol-3-ol;
*N,N*-Diethyl-3,9-dimethoxy-5-nitro-2*H*[1]benzothiopyrano[4,3,2-*cd*]indazole-2-ethanamine;
5-Amino-*N,N*-diethyl-3,9-dimethoxy-2*H*[1]benzothiopyrano[4,3,2-*cd*]indazole-2-ethanamine;
*N*-[2-[2-(Diethylamino)ethyl]-3,9-dimethoxy-2*H*[1]benzothiopyrano[4,3,2-*cd*]indazol-5-yl]-1,2-ethanediamine, hydrobromide salt;
5-[2-(Aminoethyl)amino]-2-[2-diethylamino)ethyl]-2*H*[1]benzothiopyrano[4,3,2-*cd*]indazol-3,9-diol, hydrobromide salt;
5-Amino-2-[2-(diethylamino)ethyl]-2*H*[1]benzothiopyrano[4,3,2-*cd*]indazol-3,9-diol;
and the pharmaceutically acceptable salts thereof.

Certain of the compounds of the invention are also useful as intermediates in the preparation of the preferred species; more specifically, those compounds of structure 1 wherein $R_5$ represents $NO_2$ or $NH_2$ and those compounds such as those of structures 7, 8, 9 and 10 described below which contain protecting groups.

PROCESS FOR PREPARING THE COMPOUNDS

The invention in one process aspect comprises a process for preparing compounds having the structural formula 2:

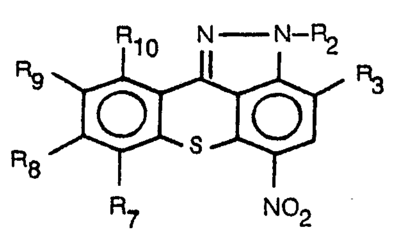

2

by reacting a 1-halo-4-nitro-9*H*-thioxanthen-9-one [such as that described by S. Archer and C. M. Suter, *J. Am. Chem. Soc., 74;* 4296 (1952)] and $R_2$-substituted hydrazine having the structural formula $H_2NNHR_2$, wherein $R_2$, $R_3$, $R_7$, $R_8$, $R_9$, and $R_{10}$ have the above meaning. The reaction conditions can be varied widely. The reaction is usually carried out in a solvent at temperatures between about 25 to about 140°C. A suitable solvent is xylene, pyridine or DMF.

The requisite hydrazines are prepared by reaction of hydrazine with the appropriate alkyl halide, $XR_2$, wherein $R_2$ has the above meaning [*J. Med. Chem., 7;* 403 (1964)], or other methods known in the art as described below.

The invention in another aspect comprises a process for preparing compounds having the structural formula 3:

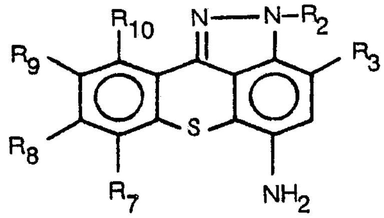

3

by subjecting a compound having the structural formula 2:

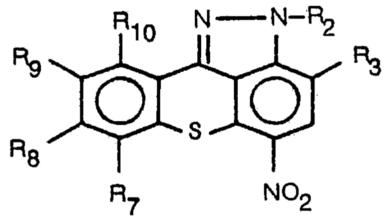

2

to reduction, wherein $R_2$, $R_3$, $R_7$, $R_8$, $R_9$, and $R_{10}$ have the above meaning. The reduction is carried out by suitable means, preferably by hydrogenation at room temperature using a palladium/charcoal catalyst in a solvent such as methanol or acetic acid, acetic acid being preferred.

The invention in another aspect comprises a process for preparing compounds having the structural formula 4:

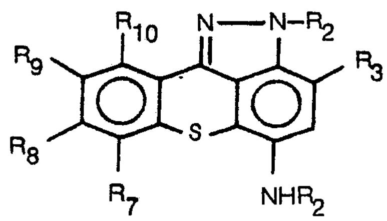

4

by reacting a compound having the structural formula 3:

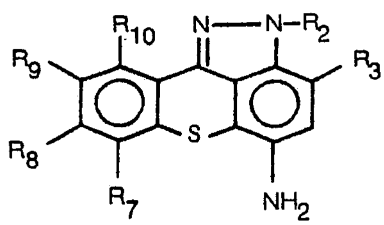

3

with a haloalkylamine having the formula $XR_2$, wherein $R_2$, $R_3$, $R_7$, $R_8$, $R_9$, and $R_{10}$ have the above meaning and X is a halogen. Where $R_2$ includes a sensitive group, this is appropriately protected prior to the reaction by a protective group. The reaction is carried out in the absence of solvent or in a suitable unreactive solvent such as $CHCl_3$ or DMF. In the absence of solvent, the reaction temperature may be about 150°C. With solvent, reflux temperature is used, for example, from about 60 to about 150°C. Bases such as $Et_3N$ or $K_2CO_3$ may be employed as acid scavengers but are not essential.

In another aspect, the invention comprises a process for preparing compounds having the structural formula 4:

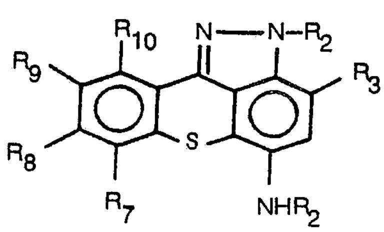

4

by reacting a compound having the formula 3:

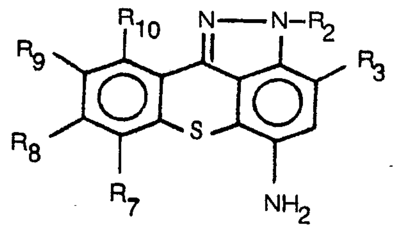

3

with the appropriately substituted aldehyde or acetal, ketone or ketal at reflux temperature, for example, from about 65 to about 140°C, and reducing the resulting Schiff base with a suitable reducing agent such as $NaBH_4$ or $NaBH_3CN$; wherein $R_2$, $R_3$, $R_7$, $R_8$, $R_9$, and $R_{10}$ have the above meaning.

In still another aspect, the invention comprises a process for preparing compounds having the structural formula 5:

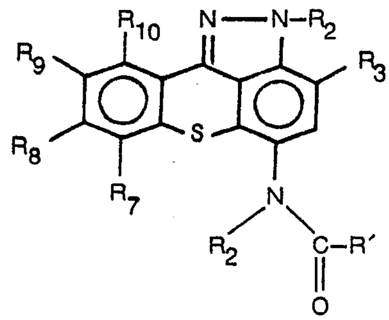

5

by monoacylating a compound having the structural formula 4:

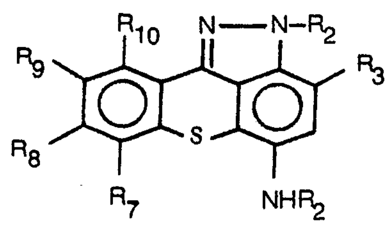

4

with a reactive derivative of an acylating agent of formula R′COOH such as acid halides, anhydrides and the like, wherein $R_2$, $R_3$, $R_7$, $R_8$, $R_9$, $R_{10}$ and R′ have the above meaning. Where R′ includes a sensitive group such as a hydroxy group, this is appropriately protected prior to acylation by a protective group such as benzyl group. The reaction can be carried out in any suitable way, for example, by reacting the compounds in a solvent such as pyridine at suitable temperature, for example, from about 25 to about 115°C.

In one preferred embodiment, the invention comprises a process for preparing benzothiopyrano[4,3,2-*cd*]indazole compounds having the structural formula 1:

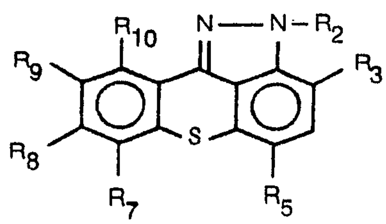

1

which comprises reacting an optionally substituted 1-chloro-4-nitro-9*H*-thioxanthen-9-one and an $R_2$-substituted hydrazine to form the compounds having the structural formula 1 where $R_5$ is nitro and, if desired, converting said compounds by reduction to compounds having the stuctural formula 1 where $R_5$ is $NH_2$ and if further desired converting said compounds to compounds having the structural formula 1 where $R_5$ is $NHR_2$ by alkylation with a haloalkylamine having the formula $XR_2$, optionally after covering sensitive groups with protective groups; or by monoacylating with a reactive derivative of an acylating agent of formula $R^aCOOH$ and reducing the acylated product, optionally after covering sensitive groups with protecting groups; and if further desired, converting said compounds wherein $R_5$ is $NHR_2$ to corresponding compounds wherein $R_5$ is $NR'R_2$ by further acylation and reduction; and if desired, removing the protecting groups by hydrolysis or reduction; and isolating the product in free base or acid addition salt form; wherein X is chloro or bromo and $R_2$, $R_3$, R′, $R_5$, $R_7$, $R_8$, $R_9$, and $R_{10}$ have the above meaning and wherein $R^a$ is a straight or branched alkyl having from one to three carbon atoms.

In another preferred embodiment, the invention comprises a process for preparing benzothiopyrano[4,3,2-*cd*]indazole compounds having in free base form the structural formula:

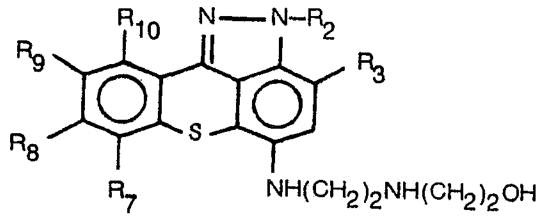

by reacting a compound having the structural formula:

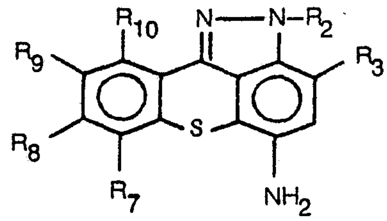

wherein $R_3$, $R_7$, $R_8$, $R_9$, and $R_{10}$ are hydrogen, hydroxyl, lower alkoxy, or benzyloxy, or *p*-halo or *p*-methoxy substituted benzyloxy, with 3-(β-haloethyl)-2-oxazolidinone to obtain an oxazolidinone compound having the structural formula:

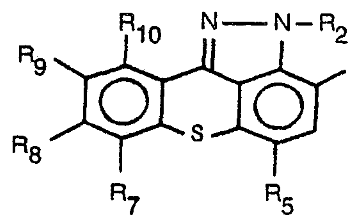

as defined above wherein $R_5$ is an N-[2-(2-oxo-3-oxazolidinyl)ethyl]amino group and subjecting the latter compound to alkaline hydrolysis to obtain a compound having the structural formula:

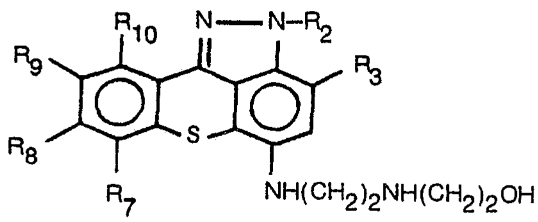

if necessary, removing any benzyloxy groups by hydrogenolysis, and isolating the product in free base or acid addition salt form. The invention also contemplates the embodiment in which one or more of $R_3$, $R_7$, $R_8$, $R_9$, and $R_{10}$ are benzyloxy or alkoxy wherein the benzyloxy and alkoxy groups are removed by hydrolysis or treatment with boron tribromide.

In another embodiment, the invention comprises a process for preparing compounds having the structural formula 6:

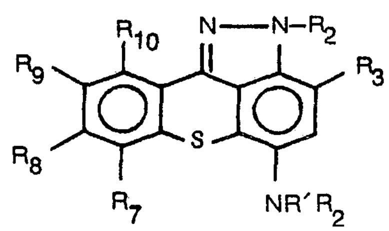

6

by subjecting a compound having the structural formula 5:

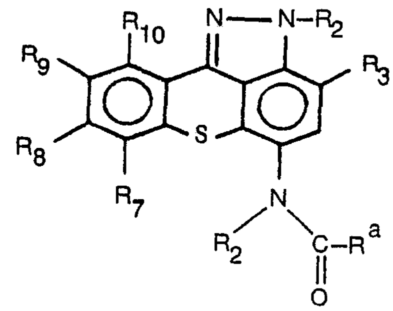

to reduction, wherein $R^a$, $R_2$, $R_3$, $R_7$, $R_8$, $R_9$, and $R_{10}$ have the above meaning. One uses a suitable reducing agent, preferably in an ethereal solvent such as ether, dioxane, THF or ethylene glycol dimethyl ether. Typically $LiAlH_4$ is employed, but other reducing agents, for example, aluminum derivatives such as $AlH_3$ may be used.

In another embodiment, the invention comprises a process for preparing compounds having the formula 11:

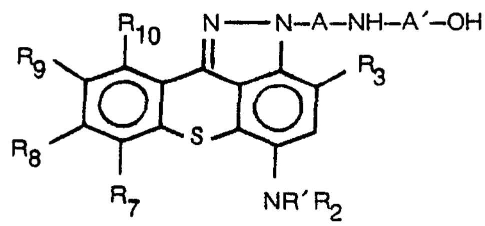

11

by removing the protecting groups from a compound having the structural formula 10:

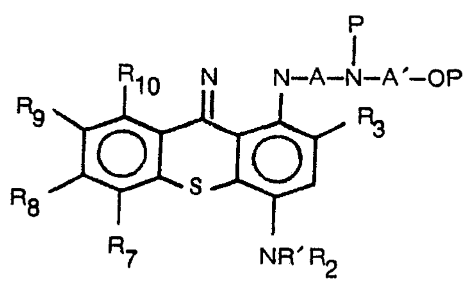

10

wherein A, A', R', $R_2$, $R_3$, $R_7$, $R_8$, $R_9$ and $R_{10}$ have the above meaning and P is a protective group such as acyl or benzyl. Removal of the protective groups is accomplished in any suitable way. For example, acyl groups are removed by hydrolysis; benzyl groups are removed by reduction. When acyl is used as a protecting group with compounds containing two or more protic groups separated by two or three carbon atoms, cyclic intermediates having the structural formula 2a:

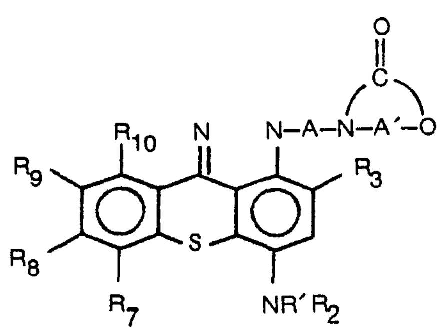

2a

are contemplated where A and A' have the above meaning.

The process and the method of preparing compounds such as structure 11 are illustrated as follows:

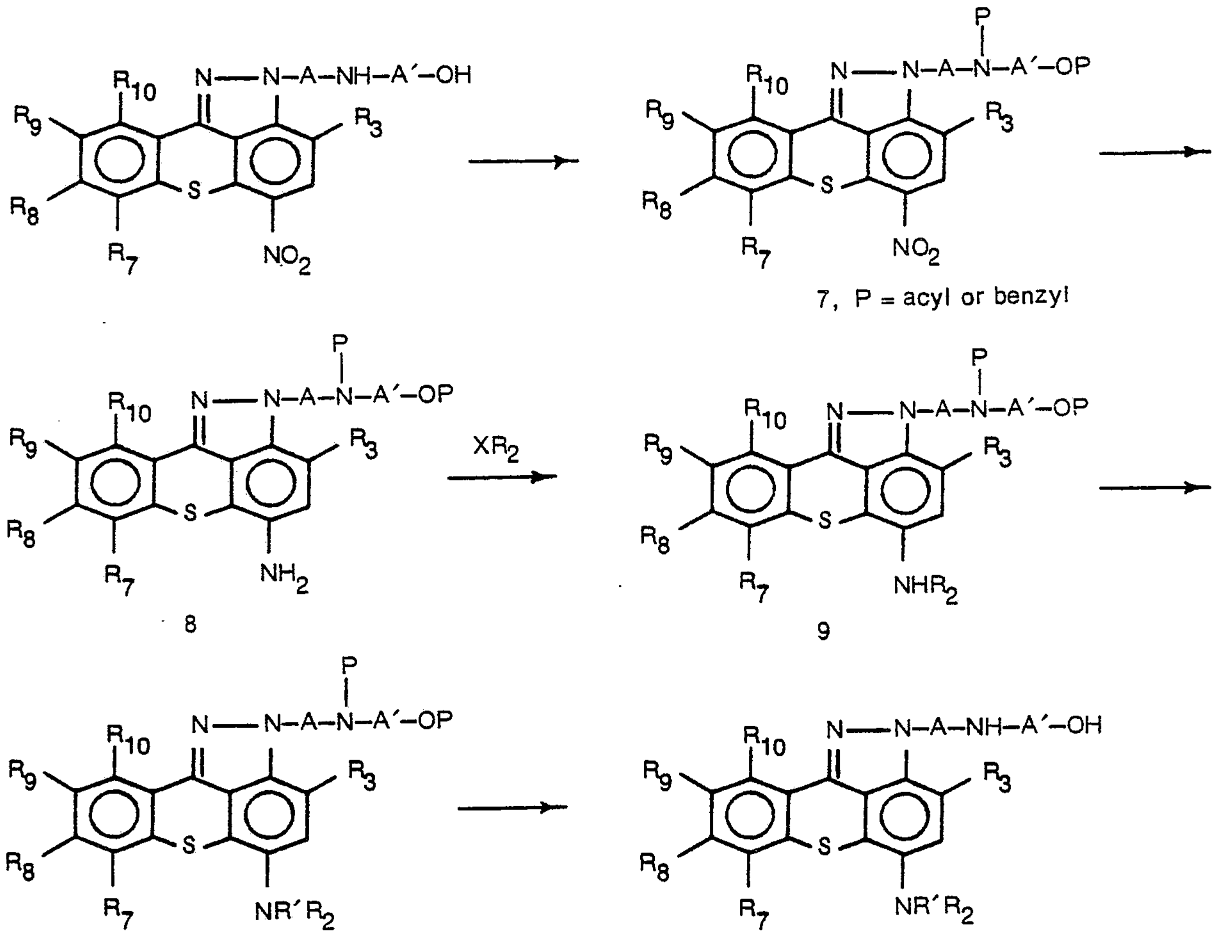


7, P = acyl or benzyl

8

9

10

11

The protecting groups may be installed by reacting the starting material with an acyl anhydride or benzyl halide or diphenyl carbonate. Alternatively, a hydrazine precursor already bearing protective groups may be utilized as, for example, $H_2NNH—A—N(P)—A'—OP$. The reaction steps to give a compound having the formula 10 are each carried out in a manner analogous to that described above for the production of a compound having one of the formulas 3, 4, 4a, or 6, provided however that, when P is benzyl, the reduction to a compound having the formula 8 is accomplished using Raney nickel as the catalyst in a suitable solvent such as MeOH, THF, or MeOH/THF combination rather than acetic acid.

In another embodiment, the invention comprises a process for preparing compounds having the structural formula 16:

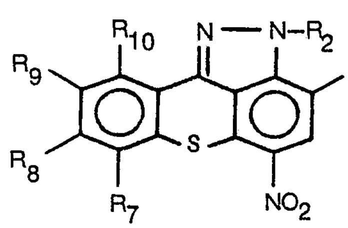

16

by reacting a compound having the structural formula 15:

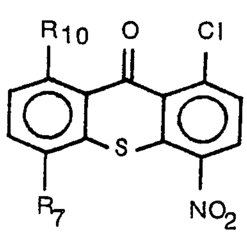

15

with a substituted hydrazine having the structural formula $H_2NNHR_2$, wherein $R_2$ has the above meaning, $R_7$ and $R_{10}$ are alkoxy of from 1 to 4 carbon atoms, benzyloxy or *p*-halo or *p*-methoxy-substituted benzyloxy, and $R_{10}$ may also be hydroxyl. The addition of the hydrazine proceeds best, according to the invention, in DMF at room temperature.

In another embodiment, the invention comprises a process for preparing compounds having the structural formula 17:

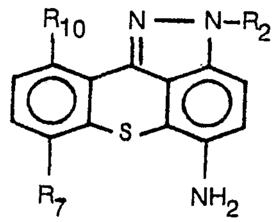

17

by subjecting a compound having the structural formula 16:

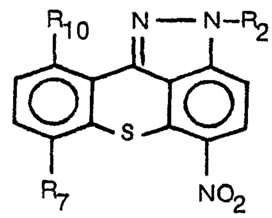

16

to reduction, wherein $R_2$ has the above meaning, $R_7$ and $R_{10}$ are alkoxy of from 1 to 4 carbon atoms, benzyloxy or *p*-halo or *p*-methoxy-substituted benzyloxy, and $R_{10}$ may also be hydroxyl, preferably by hydrogenation in AcOH using palladium/charcoal as the catalyst for the alkoxy substituted compounds and in MeOH using Raney nickel as the catalyst for the benzyloxy substituted compounds. If $R_2$ of compounds having the formula 16 contains reactive groups such as NH, $NH_2$, or OH, these groups may be protected using conditions described above for compounds having the formula 2.

In another embodiment, the invention comprises a process for preparing compounds having the structural formula 18:

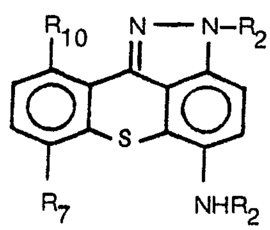

18

by N-alkylating a compound having the structural formula 17:

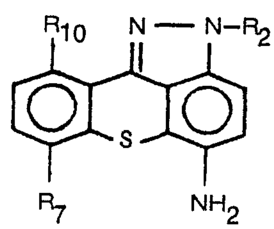

17

wherein $R_2$ has the above meaning, $R_7$ and $R_{10}$ are alkoxy of from 1 to 4 carbon atoms, benzyloxy, or *p*-halo- or *p*-methoxy-substituted benzyloxy, and $R_{10}$ may also be hydroxyl.

In another embodiment, the invention comprises a process for preparing compounds having the structural formula 19:

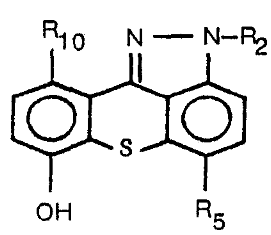

19

from a compound having the structural formula 16:

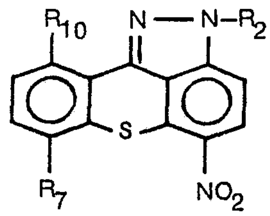

16

wherein $R_2$ has the above meaning, $R_5$ is nitro, $R_7$ and $R_{10}$ are alkoxy of from 1 to 4 carbon atoms, benzyloxy, or *p*-halo- or *p*-methoxy-substituted benzyloxy, and one of $R_7$ and $R_{10}$ may be hydroxyl. The conversion is accomplished suitably with $BBr_3$ in dichloromethane at temperatures in the range from about 25 to about 60°C for compounds wherein $R_7$ and $R_{10}$ are alkoxy or by hydrogenolysis for compounds wherein $R_7$ and $R_{10}$ are benzyloxy.

In another embodiment, the invention comprises a process for preparing certain compounds having the structural formula 19:

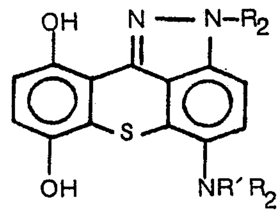

19

by acylating a compound having the structural formula 18:

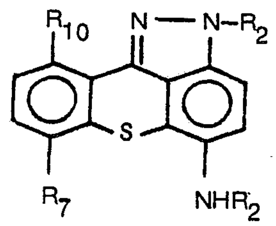

18

reducing the resulting acylated compound $LiAlH_4$ and treating the $BBr_3$, wherein $R_2$ has the above meaning, $R_7$ and $R_{10}$ are benzyloxy or alkxoy from 1 to 4 carbon atoms and one of $R_7$ and $R_{10}$ may also be hydroxyl.

For the preparation of starting material, intermediate 12, prepared by know procedures [P. K. Bannerjee and D. M. Chaudhury, *J. Indian Chem. Soc., 86:* 257 (1959)], is diazotised with sodium nitrite, treated with the potassium salt of ethylxanthic acid, and hydrolized with HCl to give compound 13. Reaction with 2,4-dichloronitrobenzene affords compound 14, which is ring closed in a trifluoroacetic acid/trifluoroacetic anhydride mixture to give intermediate 15. Alternatively, compound 14 may be chlorinated with thionyl chloride and ring closed with $AlCl_3$ in nitrobenzene at 70°C to give the intermediate 15a. Alternatively, the preparation of starting material 15 where $R_7$ and $R_{10}$ are alkoxy from 2 to 4 carbon atoms, benzyloxy, or *p*-halo- or *p*-methoxybenzyloxy is accomplished as follows: 2,5-dihydroxybenzoic acid is either alkylated or benzylated with a suitable alkyl or benzyl halide, nitrated, and reduced utilizing methods known to those skilled in the art to give 2-amino-3,6-[(dialkoxy, dibenzyloxy, or di(*p*-halo- or p-methoxy)-benzyloxy]benzoic acid. This intermediated is then reacted as described above for intermediate 12 to produce starting material 15 where $R_7$ and $R_{10}$ are alkoxy from 2 to 4 carbon atoms, benzyloxy, or *p*-halo- or *p*-methoxy-benzyloxy. The reactions are illustrated as follows:

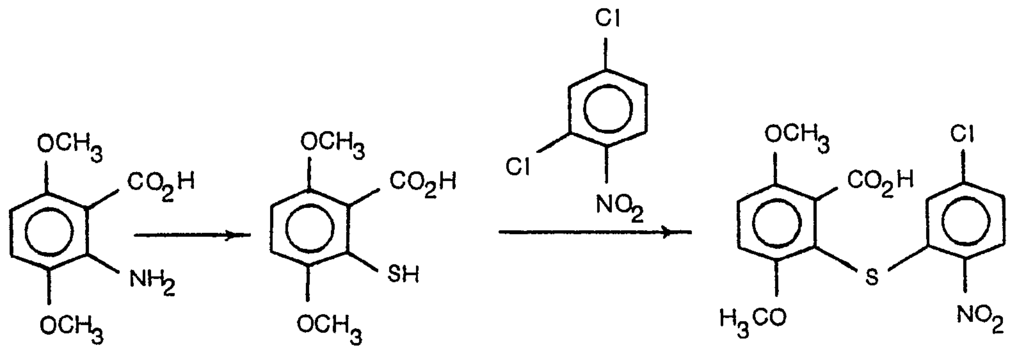

12 13 14

1. $SOCl_2$
2. $AlCl_3$

15a or 15b

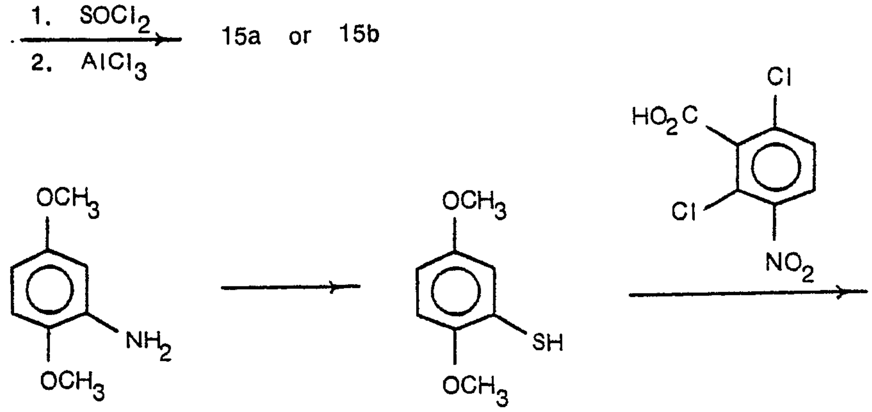

12a 13a

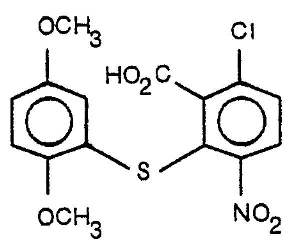

14a

1. $SOCl_2$
2. $AlCl_3$

15a or 15b

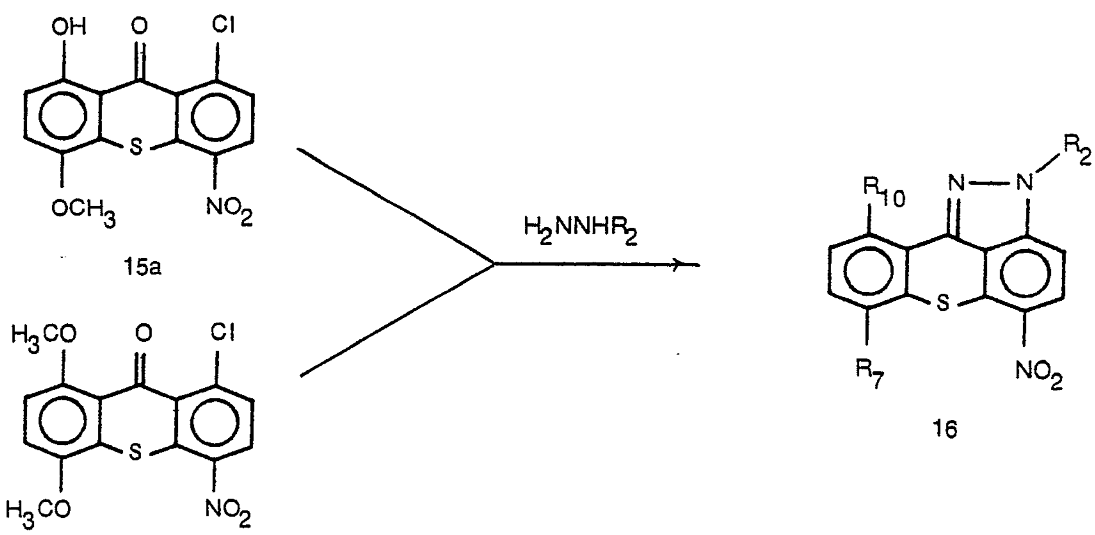


15a

15b

16

In still another embodiment, the invention comprises a process for preparing compound having the structural formula 24:

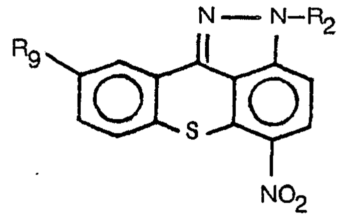

24

by reacting a compound having the structural formula 23:

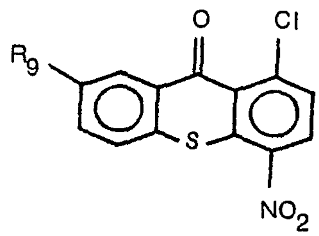

23

with a substituted hydrazine having the structural formula $H_2NNHR_2$; wherein $R_2$ has the above meaning and $R_9$ is alkoxy of from 1 to 4 carbon atoms and may also be benzyloxy of halo- or methoxy-substituted benzyloxy.

Alternatively, a process for preparing compounds of structural formula 16 comprises reacting a compound of formula 13a with 2,6-dichloro-3-nitrobenzoic acid to produce compound 14a which is subsequently ring-closed to produce the intermediates 15a or 15b. The starting material, 2,6-dichloro-3-nitrobenzoic acid, is prepared by the method of Lehmstadt and Schrader, *Ber. 70b:* 1526 (1937).

The process and the method of preparing the starting material are illustrated as follows:

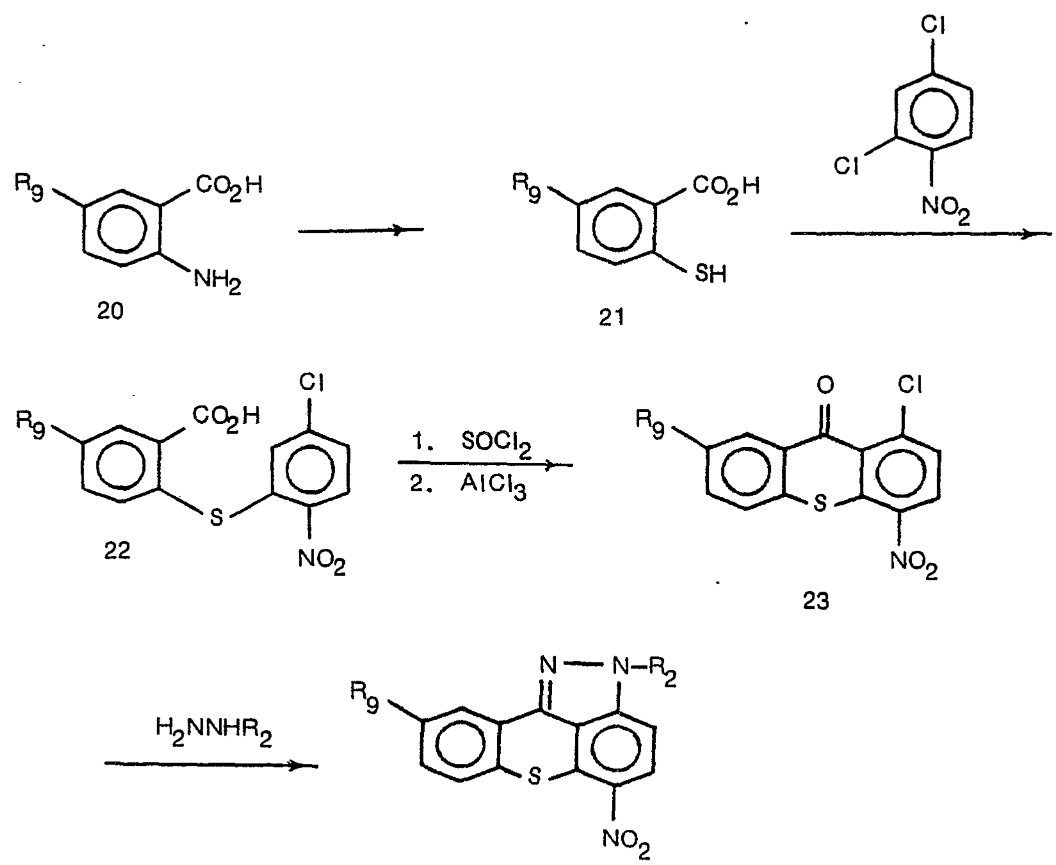


20 21 22 23 24

For the preparation of the starting materials 23, compound 20 [prepared by known procedures: N. B. Chapman, G. M. Gibson, and F. G. Mann, *J. Chem. Soc.,* 890 (1947), and German patent 2,525,050], is diazotized with sodium nitrite, treated with the potassium salt of ethylxanthic acid, and hydrolyzed with HCl to give compound 21. Reaction of 21 with 2,4-dichloro-nitrobenzene affords compound 22, which is chlorinated with thionyl chloride and ring closed with $AlCl_3$ in nitrobenzene at 70°C to give the intermediate 23. Addition of the hydrazine according to the invention, proceeds to give a compound having the formula 24. Suitably, the reaction can be carried out in xylene at 70°C in the presence of $K_2CO_3$.

In still another embodiment, the invention comprises a process for preparing compounds having the structural formula 25:

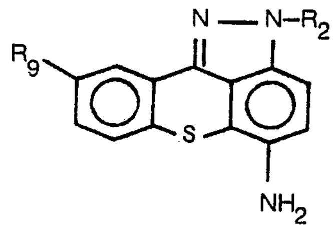

25

by subjecting to reduction a compound having the structural formula 24:

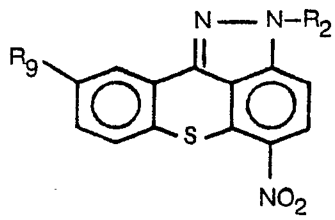

24

wherein $R_2$ has the above meaning and $R_9$ is alkoxy of from 1 to 4 carbon atoms and may also be benzyloxy or halo- or methoxy-substituted benzyloxy. Preferably, the reduction is carried out in AcOH using Pd/C as the catalyst for the methoxy substituted compounds and in MeOH using Raney nickel as the catalyst for the benzyloxy substituted compounds.

The invention also comprises a process for preparing compounds having the structural formula 26:

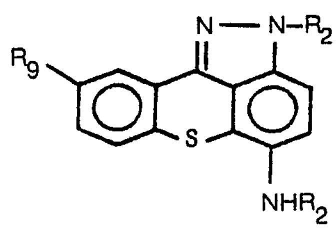

26

by alkylating a compound having the structural formula 25:

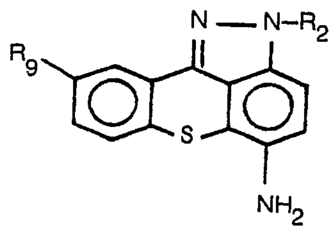

25

wherein $R_2$ has the above meaning and $R_9$ is alkoxy of from 1 to 4 carbon atoms and may also be benzyloxy or *p*-halo- or *p*-methoxy-substituted benzyloxy.

The invention also comprises a process for preparing compounds having the structural formula 27:

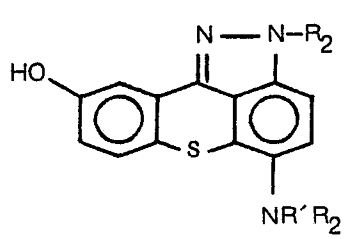

27

by acylating a compound having the structural formula 26:

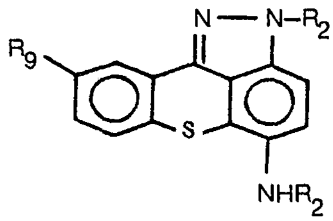

26

reducing the resulting acylated compound with $LiAlH_4$, and dealkylating as above or debenzylating as below, wherein $R_2$ has the above meaning and $R_9$ is alkoxy of from 1 to 4 carbon atoms and may also be benzyloxy or *p*-halo- or *p*-methoxy-substituted benzyloxy.

In another embodiment, the invention comprises a process for preparing compounds having the structural formula 28:

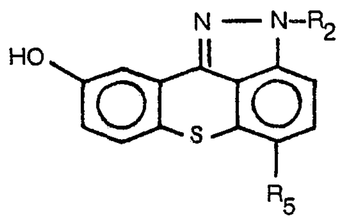

28

from a compound having the structural formula 24:

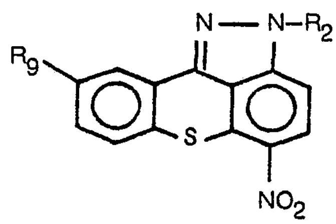

24

wherein $R_2$ has the above meaning and, $R_5$ is nitro and $R_9$ is alkoxy of from 1 to 4 carbon atoms and may also be benzyloxy or *p*-halo- or *p*-methoxy-substituted benzyloxy. The conversion is suitably accomplished by hydrolysis with acid such as 48% HBr for compounds wherein $R_9$ is alkoxy or by hydrogenolysis for compounds wherein $R_9$ is benzyloxy.

Purification of compounds or products obtained by the methods of the invention is accomplished in any suitable way, preferably by column chromatography or crystallization.

The invention in its composition aspect relates to a pharmaceutical composition comprising a compound having structural formula 1 and the pharmaceutically acceptable salt thereof in combination with a pharmaceutically acceptable carrier.

The invention in another aspect relates to a pharmaceutical composition comprising a compound having structural formula 2 and the pharmaceutically acceptable salts thereof in combination with a pharmaceutically acceptable carrier.

The invention in another aspect relates to a pharmaceutical compositions comprising a compound having structural formula 4a and the pharmaceutically acceptable salts thereof in combination with a pharmaceutically acceptable carrier.

The invention in another pharmaceutical aspect relates to a pharmaceutical composition comprising a compound having structural formula 6 and the pharmaceutically acceptable salts thereof in combination with a pharmaceutically acceptable carrier.

The compounds of this invention may be used in a method for treating microbial infections in a mammal which comprises administering a sufficient amount of a compound having structural formula 1 and the pharmaceutically acceptable salts thereof in combination with a pharmaceutically acceptable carrier, to a mammal in need thereof.

The compounds of this invention may also be used in a method for treating leukemia in a mammal which comprises administering a sufficient amount of compound having structural formula 1 and the pharmaceutically acceptable salts thereof in combination with a pharmaceutically acceptable carrier, to a mammal in need thereof.

The compounds of this invention may also be used in a method for treating leukemia in a mammal which comprises administering a sufficient amount of a compound having structural formula 4a and the pharmaceutically acceptable salts thereof in combination with a pharmaceutically acceptable carrier, to a mammal in need thereof.

The compounds of this invention may be used in a method for treating solid tumours in a mammal which comprises administering a sufficient amount of a compound having structural formula 1 and the

pharmaceutically acceptable salts thereof in combination with a pharmaceutically acceptable carrier, to a mammal in need thereof.

The compounds of this invention may also be used in a method for treating solid tumours in a mammal which comprises administering a sufficient amount of a compound having structural formula 4a and the pharmaceutically acceptable salts thereof in combination with a pharmaceutically acceptable carrier, to a mammal in need thereof.

The compounds of this invention may also be used in a method for treating solid tumours in a mammal which comprises administering a sufficient amount of a compound having structural formula 6 and the pharmaceutically acceptable salts thereof in combination with a pharmaceutically acceptable carrier, to a mammal in need thereof.

PHYSICAL AND PHARMACOLOGICAL PROPERTIES OF THE COMPOUNDS

The benzothiopyrano[4,3,2-*cd*]indazole compounds of the invention range in color from beige to orange. They are crystalline solids that are stable under normal atmospheric conditions. The compounds typically have melting points in the range of about 100 to about 250°C.

The compounds are useful as pharmacological agents for the treatment of bacterial and fungal infections in warm-blooded animals. The activity of representative compounds of the invention was established by test protocols described below.

In addition to their usefulness as antibacterial and antifungal agents, compounds of the invention display in vitro and in vivo antitumor activity.

TEST PROTOCOLS

In Vitro

One test protocol is the in vitro proliferating human colon adenocarcinoma (HCA) cell screen. In this test, HCT—8 cells (HCA cell line received from Yale University) are trypsinized using trypsin-EDTA. A single cell suspension is achieved by passing the cells through a 26 gauge needle with a 20 cc syringe. A cell suspension is prepared using RPMI 1640 + 10% FCS + 50) g/ml gentamicin sulfate with a cell concentration of approximately 30,000 cells/ml. The cell suspension is dispensed in Linbro 24-well plates; 1 ml/well. The plates are incubated for approximately 48 hours at 37°C in a 5% $CO_2$ atmosphere. At this time test compounds are added in the appropriate concentration. Five µl of the 200 µg/ml stock solution is added to each well in a primary test. Ten µl of the appropirate dilution is added to each well for a titration test. The plates are re-incubated an additional 60 to 65 hours at 37°C in a 5% $CO_2$ atmosphere. The cells are lysed using a mix of cationic surfactant, glacial acetic acid, and sodium chloride. Two ml of the lysed cell suspension from each well is added to 8 ml of diluent. The number of nuclei is determined using a Coulter counter (ZBI model), and a percent growth for each drug concentration is calculated. From this, an $ID_{50}$ molar concentration of a compound (that results in 50% inhibition of growth) is determined.

Another test protocol uses L1210 cells, a murine leukemia cell line, grown in RPMI 1640 supplemented with 5% fetal bovine serum and gentamicin (50 µg/ml). Drug dilutions are prepared in the appropriate solvent and 20 µl of each dilution are added to 24-well Linbro tissue culture plates, followed by the addition of 2.0 ml of cell suspension containing $3 \times 10^4$ cells per ml. Solvent and medium controls are included in each test. After incubation at 37°C for three days in 5% $CO_2$, the contents of each well are removed and the cells counted in a ZBI Coulter counter. Percent growth are calculated relative to the controls and the levels of drug activity are expressed as $ID_{50}$ in moles per liter.

Still another test protocol is the in vitro antibacterial/antifungal (ABF) test. Compounds are tested for antimicrobial activity in an agar-disk diffusion assay, a standard microbiological technique for testing antibiotics. After incubation of each culture with a test compound, a zone of inhibition is determined. The zone diameter (mm) of active compounds ranges from a minimum of 13.5 mm to as high as 60 mm, with a greater diameter reflecting higher activity. For convenience, values are reported for two gram-negative bacteria (*Aerobacter aerogenes* 0126 and *Escherichia coli* 04863), two gram-positive bacteria (Bacillus subtilis 04555 and *Streptococcus faecalis* 05045 utilizing AM—09 medium), and one mycelial fungus (*Penicillium avellaneum* M2988).

In Vivo

Another test protocol is the in vivo lymphocytic leukemia P388 test. The animals used are either male or female $CD_2F_1$ mice. There are six or seven animals per test group. The tumor transplant is by intraperitoneal injection of dilute ascitic fluid containing cells of lymphocytic leukemia P388. The test compounds are administered intraperitoneally once daily for five consecutive days at various doses following tumor inoculation. The animals are weighed and survivors are recorded on a regular basis for 30 days. A compound is designated "toxic" if, at a given dose, all animals died prior to four days after the first injection of drug. A ratio of survival time for treated (T)/control (C) animals is calculated. A criterion for efficacy is a ratio T/C times 100 greater than or equal to 125. See *Cancer Chemotherapy Reports*, Part 3, 3; 1 (1972) for a comprehensive discussion of the protocol.

The test protocol procedures gave results listed in Tables 1 and 2 for representative compounds of the invention.

TABLE 1

Chemical, Antitumor, Antibacterial, and Antifungal Data on 2,5-(Disubstituted)-2H-[1]benzothiopyrano[4,3,2-cd]indazoles

| $R_2$ | $R_5$ | Formula | mp, °C | L1210 in vitro $ID_{50}$ (M) | HCA—3 in vitro $ID_{50}$ (M) | P388 in vivo Dose (mg/kg) | P388 in vivo T/C × 100 | ABF Zone Diameter (cont. in mg/ml) A. Aerogenes | E. Coli | B. Subtilis | S. Faecalis | P. Avellaneum |
|---|---|---|---|---|---|---|---|---|---|---|---|---|
| $CH_2CH_2N(CH_2H_5)_2$ | $HO_2$ | $C_{19}H_{20}N_4O_2S$ | 150—152 | $5.6 \times 10^{-8}$ | $2.0 \times 10^{-7}$ | 50.0<br>25.0 | 170<br>138 | 0 (3) | 14 (1) | 14 (3) | 14 (3) | 15 (3) |
| $CH_2CH_2N(C_2H_5)_2$ | $NH_2$ | $C_{19}H_{22}N_4S$ | 100—102 | $1.3 \times 10^{-7}$ | $1.1 \times 10^{-6}$ | 25.00<br>12.50 | 162<br>144 | 0 (3) | 0 (3) | 0 (3) | 14 (1) | 14 (1) |
| $CH_2CH_2N(C_2H_5)_2$ | $NHCOCH_3$ | $C_{21}H_{24}N_4OS$<br>$\cdot HCl \cdot O.lH_2$ | 208—211 | $2.4 \times 10^{-7}$ | $6.0 \times 10^{-7}$ | 12.50 | 170 | 16 (3) | 16 (3) | 16 (0.5) | 15 (3) | 0 (3) |
| $CH_2CH_2N(C_2H_5)_2$ | $N{=}CHN(CH_3)_2$ | $C_{22}H_{27}N_5S$ | 108—109 | $1.0 \times 10^{-7}$ | $2.4 \times 10^{-7}$ | 50.00<br>25.00 | 206<br>145 | 0 (3) | 0 (3) | 16 (1) | 0 (3) | 16 (3) |
| $CH_2CH_2N(C_2H_5)_2$ | $NHCH_2CH_2N(C_2H_5)_2$ | $C_{25}H_{35}N_5S$<br>$\cdot 2HCl$ | 234—236 | $1.4 \times 10^{-7}$ | $4.5 \times 10^{-7}$ | 25.00 | 155 | 14 (0.5) | 18 (0.1) | 15 (0.1) | 15 (3) | 17 (3) |
| $CH_2CH_2NHCH_2CH_2OH$ | $NO_2$ | $C_{17}H_{16}N_4O_3S \cdot HCl$ | 293—295 dec | $2.0 \times 10^{-8}$ | $1.0 \times 10^{-7}$ | 25.00<br>12.50<br>6.25 | 197<br>150<br>148 | 16 (0.1) | 16 (0.1) | 18 (0.5) | 15 (0.5) | 0 (3) |
| $CH_2CH_2NHCH_2CH_2OH$ | $NH_2$ | $C_{17}H_{18}N_4OS$<br>$\cdot 1.6HCl \cdot 0.2H_2O$ | 285 dec | $1.7 \times 10^{-7}$ | $5.8 \times 10^{-7}$ | 25.00<br>12.50<br>6.25<br>3.12 | 194<br>159<br>143<br>135 | 16 (0.5) | 17 (0.5) | 18 (0.5) | 20 (1) | 15 (3) |
| $CH_2CH_2N(C_2H_5)_2$ | $CH_2CH_2$ 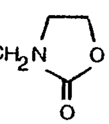 | $C_{24}H_{29}N_5O_2S$ | 90—94 | $9.5 \times 10^{-7}$ | $1.8 \times 10^{-6}$ | 100.00<br>84.38<br>63.28<br>50.00<br>47.45 | 218<br>193<br>165<br>140<br>141 | 0 (3) | 0 (3) | 13 (3) | 14 (0.5) | 0 (3) |

TABLE 1 (cont.)

| $R_2$ | $R_5$ | Formula | mp, °C | L1210 in vitro $ID_{50}$ (M) | HCA—3 in vitro $ID_{50}$ (M) | P388 in vivo Dose (mg/kg) | T/C × 100 | ABF Zone Diameter (cont. in mg/ml) A. Aero-genes | E. Coli | B. Sub-tilis | S. Fae-calis | P. Avell-aneum. |
|---|---|---|---|---|---|---|---|---|---|---|---|---|
| $CH_2CH_2N(C_2H_5)_2$ | $NHCH_2CH_2NHCH_2CH_2OH$ | $C_{23}H_{31}N_5OS$ $\cdot 2.7HCl \cdot 0.7H_2O$ | 223—228 | $8.2 \times 10^{-8}$ | $2.0 \times 10^{-7}$ | 12.50<br>6.25<br>3.12 | 280 Cures<br>219<br>209 Cures | 15 (0.1) | 26 (0.5) | 18 (0.5) | 16 (0.5) | 0 (3) |
| $CH_2CH_2NHCH_2CH_2OH$ | $NHCH_2CH_2N(C_2H_5)_2$ | $C_{23}H_{31}H_5OS$ $\cdot 2.9HCl \cdot 1.9H_2O$ | 208—212 | $2.4 \times 10^{-7}$ | $4.5 \times 10^{-7}$ | 25.00<br>12.50<br>6.25<br>3.12 | 147<br>135<br>137<br>134 | — | — | — | — | — |
| $CH_2CH_2NHCH_2CH_2OH$ | $NHCH_2CH_2NHCH_2CH_2OH$ | $C_{21}H_{27}N_5O_2S$ $\cdot 2.9HCl \cdot 0.5H_2O$ | 222—225 | $4.0 \times 10^{-7}$ | $4.8 \times 10^{-7}$ | 12.50<br>12.00<br>6.25<br>6.00<br>3.12<br>3.00<br>1.56<br>1.50<br>0.78<br>0.75 | 271 Cures<br>270 Cures<br>195 Cures<br>218<br>176<br>187<br>180<br>155<br>153<br>146 | 14 (0.5) | 14 (0.5) | 15 (0.5) | 0 (3) | 0 (3) |
| $CH_2CH_2NHCH_0H_2OH$ | $NHCH_2CH_2$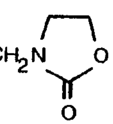 | $C_{22}H_{25}N_5O_3S$ $\cdot HCl \cdot 0.5H_2O$ | 180—182 | $5.7 \times 10^{-7}$ | $1.4 \times 10^{-6}$ | — | — | 14 (1) | 15 (3) | 16 (1) | 17 (1) | 0 (3) |
| $CH_2CH_2N(CH_3)_2$ | $NO_2$ | $C_{17}N_{16}N_4O_2S$ $\cdot CH_3SO_3H$ | 266 dec | $7.9 \times 10^{-8}$ | $9.7 \times 10^{-8}$ | 12.50 | 158 | 15 (1) | 14 (0.1) | 15 (3) | 15 (3) | 15 (0.1) |
| $CH_2CH_2CH_2N(CH_3)_2$ | $NO_2$ | $C_{18}H_{18}N_4O_2S$ $\cdot HCl \cdot 0.3H_2O$ | 309 dec | $1.9 \times 10^{-7}$ | $2.1 \times 10^{-7}$ | 100.00<br>50.00<br>50.00<br>25.00<br>50.00<br>25.00 | 141<br>135<br>174<br>121<br>153<br>126 | — | — | — | — | — |

TABLE 1 (cont.)

| $R_2$ | $R_5$ | Formula | mp, °C | L1210 in vitro $ID_{50}$ (M) | HCA—3 in vitro $ID_{50}$ (M) | P388 in vivo Dose (mg/kg) | P388 in vivo T/C × 100 | ABF Zone Diameter (cont. in mg/ml) A. Aerogenes | E. Coli | B. Subtilis | S. Faecalis | P. Avellaneum |
|---|---|---|---|---|---|---|---|---|---|---|---|---|
| $CH_2CH_2N(CH_2)_2$ | $NH_2$ | $C_{17}H_{18}N_4S$<br>$\cdot 2HCl \cdot 1.1H_2O$ | 273 dec | $8.0 \times 10^{-8}$ | $2.8 \times 10^{-7}$ | 12.50<br>6.25 | 160<br>126 | 14 (0.1) | 16 (0.5) | 15 (0.5) | 20 (1) | 17 (0.5) |
| $CH_2CH_2CH_2N(CH_3)_2$ | $NH_2$ | $C_{18}N_{20}N_4S$<br>$\cdot 2HCl \cdot 1H_2O$ | 264 dec | $2.0 \times 10^{-6}$ | $1.8 \times 10^{-6}$ | 200.00<br>100.00<br>50.00<br>100.00<br>50.00 | 169<br>182<br>121<br>197<br>129 | 16 (0.5) | 15 (3) | 0 (3) | 15 (3) | 16 (3) |
| $CH_2CH_2NH_2$ | $NO_2$ | $C_{15}H_{12}N_4O_2S$ | 199—203 | — | $2.0 \times 10^{-7}$ | — | — | 18 (0.1) | 15 (0.1) | 17 (0.1) | 0 (3) | 0 (3) |
| $CH_2CH_2NH_2$ | $NO_2$ | $C_{15}H_{12}N_4O_2S$ | >300 | — | — | 25.00<br>12.50 | 187<br>136 | — | — | — | — | — |
| $CH_2CH_2N(C_2H_5)_2$ | $NHCOCH_2Cl$ | $C_{21}H_{23}ClN_4OS$ | 204 dec | $1.9 \times 10^{-7}$ | $3.8 \times 10^{-7}$ | 50.00<br>25.00 | 165<br>124 | — | — | — | — | — |
| $CH_2CH_2NH_2$ | $NH_2$ | $C_{15}N_{14}N_4S \cdot 2HCl$ | >300 | $1.1 \times 10^{-7}$ | $3.2 \times 10^{-7}$ | 12.50<br>6.25<br>3.12 | 160<br>131<br>125 | 17 (0.1) | 19 (0.5) | 18 (1) | 15 (1) | 0 (3) |
| $CH_2CH_2N(C_2H_5)_2$ | $NHCH_2CH_2NH_2$ | $C_{21}H_{27}N_5S \cdot 3HBr$ | 261 Dec | $4.6 \times 10^{-8}$ | $9.7 \times 10^{8}$ | 25.00<br>12.50<br>6.25<br>3.12 | 148 Cures<br>297 Cures<br>215<br>178 | 19 (0.5) | 0 (3) | 20 (0.5) | 15 (1) | 0 (3) |
| $CH_2CH_2NH_2$ | $NHCH_2NHCH_2CH_2OH$ | $C_{19}H_{23}N_5OS \cdot 3HCl$ | 264 dec | $2.4 \times 10^{-8}$ | $8.8 \times 10^{-8}$ | — | — | 15 (0.5) | 0 (3) | 15 (0.5) | 0 (3) | 0 (3) |
| $CH_2CH_2N(C_2H_5)_2$ | $NHCH_2CH_2CH_2NH_2$ | $C_{22}H_{29}N_5S$<br>$\cdot 3HCl \cdot 2.1H_2O$ | 222 dec | $2.9 \times 10^{-7}$ | $8.8 \times 10^{-7}$ | 25.00<br>12.50<br>6.25<br>3.12 | 169<br>154<br>142<br>132 | — | — | — | — | — |

TABLE 1 (cont.)

| $R_2$ | $R_5$ | Formula | mp, °C | L1210 in vitro $ID_{50}$ (M) | HCA—3 in vitro $ID_{50}$ (M) | P388 in vivo Dose (mg/kg) | T/C × 100 | ABF Zone Diameter (cont. in mg/ml) A. Aero-genes | E. Coli | B. Sub-tilis | S. Fae-calis | P. Avellaneum |
|---|---|---|---|---|---|---|---|---|---|---|---|---|
| $CH_2CH_2NHC_2H_5$ | $NO_2$ | $C_{17}H_{16}N_4O_2S \cdot HCl$ | | $2.4 \times 10^{-8}$ | $1.4 \times 10^{-7}$ | 12.50 | 187 | — | — | — | — | — |
| $CH_2CH_2NHC_2H_5$ | $NO_2$ | $C_{17}H_{18}N_4S \cdot 2HCl$ | | $7.1 \times 10^{-8}$ | $3.0 \times 10^{-7}$ | 12.50 | 188 | — | — | — | — | — |
| $(CH_2)_3N(CH_3)_2$ | $NHCH_2CH_2NH_2$ | $C_{20}H_{25}N_5S \cdot 3HCl$ | | $2.9 \times 10^{-8}$ | $1.3 \times 10^{-7}$ | 12.50<br>6.25 | 298<br>185 | — | — | — | — | — |

TABLE 2

Chemical, Antitumor, Antibacterial, and Antifungal Data on 2,5-(Disubstituted)-3,7,8,9,10-(Substituted)-2*H*[1]benzothiopyrano-[4,3,2-*cd*]indazoles

| $R_2$ | $R_5$ | R7—R10 | Formula | mp, °C | L1210 in vitro $IC_{50}$ (M) | HCA—3 in vitro $IC_{50}$ (M) | P388 in vivo Dose (mg/kg) | P388 in vivo T/C × 100 | ABF Zone Diameter (cont. in mg/ml) A. Aerogenes | E. Coli | B. Subtilis | S. Faecalis | P. Avellaneum |
|---|---|---|---|---|---|---|---|---|---|---|---|---|---|
| $CH_2CH_2N(C_2H_5)_2$ | $NO_2$ | 7-$OCH_3$, 10-OH | $C_{20}H_{22}N_4O_4S \cdot HCl$ | 267—270 | $1.0 \times 10^{-6}$ | $4.2 \times 10^{-7}$ | | | | | | | |
| $CH_2CH_2N(C_2H_5)_2$ | $NO_2$ | 7,10-$(OCH_3)_2$ | $C_{21}H_{24}N_4O_4S \cdot 0.1C_3H_7NO$ | 188—190 | $1.3 \times 10^{-6}$ | $5.0 \times 10^{-7}$ | | | | | | | |
| $CH_2CH_2N(C_2H_5)_2$ | $NO_2$ | 7,10-$(OH)_2$ | $C_{19}H_{20}N_4O_4S \cdot HBr \cdot 0.2H_2O$ | 283 dec | $1.7 \times 10^{-6}$ | | | | | | | | |
| $CH_2CH_2N(C_2H_5)_2$ | $NH_2$ | 7,10-$(OH)_2$ | $C_{19}H_{22}N_4O_2S \cdot HBr$ | 229—223 | $>2.2 \times 10^{-6}$ | $>2.2 \times 10^{-6}$ | | | 0 (3) | 0 (3) | 0 (3) | 19 (3) | 0 (3) |
| $CH_2CH_2N(C_2H_5)_2$ | $NHCH_2CH_2NHCH_2CH_2OH$ | 7,10-$(OH)_2$ | $C_{23}H_{31}N_5O_3S \cdot 2.4HBr \cdot 0.4H_2O$ | 243—245 | $>1.5 \times 10^{-6}$ | $>1.5 \times 10^{-6}$ | 50.00<br>25.00<br>12.50<br>6.25 | 138<br>121<br>118<br>123 | 14 (3) | 0 (3) | 0 (3) | 15 (0.5) | 0 (3) |
| $CH_2CH_2NHCH_2CH_2OH$ | $NO_2$ | $C_{20}H_{22}N_4O_3S$ | 240—244 | 240—244 | $6.4 \times 10^{-7}$ | | 25.00<br>12.50 | 209<br>142 | | | | | |
| $CH_2CH_2NHCH_2CH_2OH$ | $NH_2$ | 9-$OCH_3$ | $C_{20}H_{24}N_4OS \cdot 2HCl$ | 275 dec | $1.6 \times 10^{-6}$ | | 50.00 | 154 | | | | | |

TABLE 2 TABLE 2 (cont.)

| $R_2$ | $R_5$ | R7—R10 | Formula | mp, °C | L1210 in vitro $IC_{50}$ (M) | HCA—3 in vitro $IC_{50}$ (M) | P388 in vivo Dose (mg/kg) | T/C × 100 | ABF Zone Diameter (cont. in mg/ml) A. Aerogenes | E. Coli | B. Subtilis | S. Faecalis | P. Avellaneum |
|---|---|---|---|---|---|---|---|---|---|---|---|---|---|
| $CH_2CH_2N(C_2H_5)_2$ | 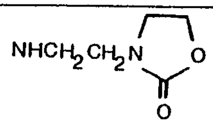 | 9-$OCH_3$ | $C_{25}H_{31}N_5O_3S \cdot 2HCl$ | | | $1.0 \times 10^{-6}$ | 100.00 | 220 | — | — | — | — | — |
| $CH_2CH_2N(C_2H_5)_2$ | $NH_2$ | 9-OH | $C_{19}H_{22}N_4OS \cdot 2HCl$ | | $1.2 \times 10^{-8}$ | $2.8 \times 10^{-8}$ | 6.25 | 238 | — | — | — | — | — |
| $CH_2CH_2N(C_2H_5)_2$ | $NHCH_2CH_2NHCH_2CH_2OH$ | 9-$OCH_3$ | $C_{24}H_{33}N_5O_2S5HCl$ | | $2.0 \times 10^{-7}$ | $2.3 \times 10^{-7}$ | 25.00 | 177 | — | — | — | — | — |
| $CH_2CH_2N(C_2H_5)_2$ | $NHCH_2CH_2NH_2$ | 9-$OCH_3$ | $C_{22}H_{29}N_5OS \cdot 3HCl$ | | $2.1 \times 10^{-7}$ | $2.9 \times 10^{7}$ | 12.50 | 177 | — | — | — | — | — |
| $CH_2CH_2N(C_2H_5)_2$ | 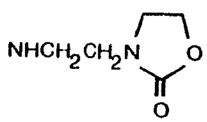 | 9-OH | $C_{24}H_{29}N_5O_3S \cdot 2HCl$ | | $5.5 \times 10^{-9}$ | $2.4 \times 10^{-8}$ | 12.50 | 175 | — | — | — | — | — |

PREPARATION OF PHARMACEUTICAL COMPOSITIONS

When being utilized as antibiotic and antifungal agents, the compounds of the invention can be. prepared and administered in a wide variety of topical, oral, and parenteral dosage forms. It will be clear to those skilled in the art that the following dosage forms may comprise as the active component, one or more compounds of formula *1*, a corresponding pharmaceutically acceptable salt of any of said compounds, or a mixture of such compounds and/or salts.

For preparing pharmaceutical compositions from the compounds described by this invention, inert, pharmaceutically acceptable carriers can be either solid or liquid. Solid form preparations include powders, tablets, dispersible granules, capsules, sachets, and suppositories. A solid carrier can be one or more substances which may also act as diluents, flavoring agents, solubilizers, lubricants, suspending agents, binders, or tablets disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspensions suitable for oral use can be made by dispersing the finely divided active component in water with viscous material, i.e., natural or synthetic gums, resins, methyl cellulose, sodium carboxymethyl cellulose, and other well-known suspending agents.

Topical preparations included dusting powders, creams, lotions, gels, and sprays. These various topical preparations may be formulated by well-known procedures. See for example Remington's Pharmaceutical Sciences, Chapter 43, 14th Ed., 1970, Mack Publishing Co., Easton, Pennsylvania 18042, USA.

Preferably, the pharmaceutical preparation is in unit dosage form. In such form, the preparation is subdivided into unit doses containing appropriate quantities of the active component. The unit dosage form can be packaged preparation, the package containing discrete quantities of preparation, for example, packeted tablets, capsules and powders in vials or ampoules. The unit dosage form can also be a capsule, cachet, or tablet itself or it can be the appropriate number of any of these packaged forms.

The quantity of active compound in a unit dose of preparation may be varied or adjusted from 50 mg to 500 mg according to the particular application and the potency of the active ingredient.

In therapeutic use as antibiotic and antifungal agents, the compounds utilized in the pharmaceutical method of this invention are adminstered at the initial dosage of about 0.1 mg to about 50 mg per kilogram. A dose range of about 0.5 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like, N,N-dimethylacetamide, suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged

absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by sterilization accomplished by filtering. Generally, dispersions are prepared by incorporating the various sterilized active ingredients into a sterile vehicle which contains the basic dispersion medium and the required other ingredients from those enumerated above. In the case of the sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "Pharmaceutically acceptable carrier" includes any and all solvents, dispersion media, coatings, antibacterial and antifungal agents, isotonic and absorption delaying agents and the like. The use of such media and agents for pharmaceutically active substances is well-known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in unit dosage form for ease of administration and uniformity of dosage. Unit dosage form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel unit dosage forms of the invention are dictated by and directly dependent on (a) the unique characteristics of the active material and the particular therapeutic effect to be achieved, and (b) the limitation inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in unit dosage form as hereinbefore disclosed. A unit dosage form can, for example, contain the principal active compound in amounts ranging from about 0.1 to about 500 mg, with from about 0.5 to about 250 mg being preferred. Expressed in proportions, the active compound is generally present in from about 0.1 to about 500 mg/ml of carrier. In the case of compositions containing supplementary active ingredients, the dosages are determined by reference to the usual dose and the manner of administration of the said ingredients. The daily parenteral doses for mammalian subjects to be treated ranges from 0.1 mg/kg to 100 mg/kg. The preferred daily dosage range is 0.3 mg/kg to 10 mg/kg.

The invention and the best mode of practising the same are illustrated by the following examples of preferred embodiments of selected compounds and their preparation.

Example 1

*N,N*-Diethyl-5-nitro-2*H*[1]benzothiopyrano[4,3,2-*cd*]-indazole-2-ethanamine

A mixture of 70.0 g (0.24 mol) of 1-chloro-4-nitro-9*H*-thioxanthen-9-one [*J. Am. Chem. Soc.*, *74*; 4296 (1952)], 39.4 g (0.30 mol) of 2-diethylaminoethyl)hydrazine [*J. Med. Chem.*, *7*; 493 (1964)], 41.5 g (0.30 mol) of $K_2CO_3$, and 1000 ml of xylene was heated under reflux for 1.5 hours, filtered to remove inorganic material, and allowed to cool to ambient temperature. The precipitate that accumulated was filtered and dried in vacuo to provide 65.7 g of product, mp 150—152°C.

Example 2

5-Nitro-2*H*[1]benzothiopyrano[4,3,2-*cd*]indazole-2-ethanamine

A solution of 26.1 g (0.35 mol) of 2-(aminoethyl)hydrazine [British Patent 880,332] in 1.4 l of DMF at 25°C was treated portionwise with 81.7 g (0.28 mol) of 1-chloro-4-nitro-9*H*-thioxanthen-9-one over a 4.5 hour span. The precipitate that accumulated was collected, washed with MeOH, and taken up in 2.5 l of boiling $H_2O$. The insoluble material was removed by filtration and the filtrate was allowed to cool to room temperature and treated with 100 ml of 2*N* $NH_4OH$ to product 39.3 g of orange solid, mp 199—203°C.

Example 3

2-[[2-(5-Nitro-2*H*[1]benzothiopyrano[4,3,2-*cd*]indazol-2-yl)ethyl]amino]ethano, hydrochloride salt

A suspension of 42.3 g (0.4 mol) of 1-chloro-4-nitro-9*H*-thioxanthen-9-one in 350 ml of pyridine was treated dropwise with 20.7 g (0.17 mol) of 2-[(hydrazinoethyl)amino]ethanol over a five minute span, keeping the temperature less than 30°C. The mixture was stirred overnight at 25°C and the precipitate was collected. The filtrate was treated with an excess of i-PrOH saturated with gaseous HCL to precipitate a second crop of product. The two crops were combined and recrystallized from DMSO to give 31.1 g of product, mp 293—295°C dec.

Other 5-nitro-2-(substituted)-2*H*[1]benzothiopyrano[3,4,2-*cd*]indazoles, prepared in the manner of Examples 1—3, are as follows:

*N,N*-dimethyl-5-nitro-2*H*[1]benzothiopyrano[4,3,2-*cd*]indazole-2-ethanamine, mp 184—189°C, methanesulfonic acid salt, mp 266°C dec.

*N,N*-dimethyl-5-nitro-2*H*[1]benzothiopyrano[4,3,2-*cd*]indazole-2-propanamine, mp 132—135°C, hydrochloride salt, mp 309°C dec.

2-[(Hydrazinoethyl)amino]ethanol may be prepared by reaction of hydrazine with *N*-(2-hydroxyethyl)aziridine in an aqueous medium at reflux temperature. It is isolated by standard procedures as a clear liquid which has bp 120°C at 0.035 mmHg.

Example 4

5-Amino-*N,N*-diethyl-2*H*[1]benzothiopyrano[4,3,2-*cd*]indazole-2-ethanamine

A mixture of 54.0 g (0.15 mol) of *N,N*-diethyl-5-nitro-2*H*[1]benzothiopyrano[4,3,2-*cd*]indazole-2-ethanamine, 1.0 g of 20% Pd/C, and 600 ml of HOAc was hydrogenated in a Parr shaker at 25°C and an initial pressure of 50.0 psi. the mixture was filtered and the filtrate was concentrated in vacuo to a small volume, treated with 1 l of $H_2O$, made basic (pH 6.5) with 50% aqueous NaOH, and extracted three times with $CHCl_3$. The extracts were combined, dried ($MgSO_4$), and concentrated to dryness in vacuo. Crystallization of the residue from $CH_3CH$ gave 40.0 g of the product, mp 100—102°C.

Other 5-amino-2-(substituted-2*H*[1]benzothiopyrano[4,3,2]-*cd*]indazoles, prepared in like manner, are as follows:

2-[[2-(5-amino-2*H*[1]benzothiopyrano[4,3,2]-*cd*]indazol-2-yl)ethyl]amino]ethanol, hydrochloride salt, mp greater than 285°C dec.

5-amino-*N,N*-dimethyl-2*H*[1]benzothiopyrano[4,3,2]-*cd*]indazole-2-ethanamine, mp 131—134°C, hydrochloride salt, mp 273°C dec.

5-amino-*N,N*-dimethyl-2*H*[1]benzothiopyrano[4,3,2]-*cd*]indazole-2-propanamine, hydrochloride salt, mp 264°C dec.

5-amino-2*H*[1]benzothiopyrano[4,3,2]-*cd*]indazole-2-ethanamine, mp 131—140°C, hydrochloride salt, mp greater than 300°C.

*N*-[2-(5-amino-2*H*[1]benzothiopyrano[4,3,2]-*cd*]indazol-2-yl)ethyl]acetamide, mp 213—216°C, hydrochloride salt, mp 287°C dec.

Example 5

5-[[2-(Diethylamino)ethyl]amino]-*N,N*-diethyl-2*H*[1]-benzothiopyrano[4,3,2-*cd*]indazole-2-ethanamine, hydrochloride salt

A mixture of 3.0 g (0.0089 mol) of 5-amino]-*N,N*-diethyl-2*H*[1]-benzothiopyrano[4,3,2-*cd*]indazole-2-ethan amine, 3.5 g (0.013 mol) of 2-(diethylamino)ethylbromide, hydrobromide, and 4.6 g (0.034 mol) of $K_2CO_3$ in 120 ml of toluene was heated under reflux for eight hours, allowed to cool to room temperature overnight, and filtered. The solid was triturated in boiling $CH_3CN$, filtered to remove insoluble inorganics, and concentrated to dryness in vacuo. The residue was dissolved in acetone and treated with an excess of iPrOH saturated with gaseous HCl. The precipitate that accumulated was collected and recrystallized from $CH_3OH$/EtOH mixture to give 2.4 g of product, mp 234—236°C.

Example 6

*N*-[2-[2-(Diethylamino)ethyl]-2*H*[1]benzothiopyrano[4,3,2-*cd*]indazol-5-yl]-1,2-ethanediamine, hydrobromide salt

A solution of 5.0 g (0.015 mol) of 5-amino-*N,N*-diethyl-2*H*[1]benzothiopyrano[4,3,2-*cd*)indazole-2-ethanamine and 9.2 g (0.045 mol) of 2-bromoethylamine, hydrochloride in 50 ml of EtOH was heated under reflux for four days and allowed to cool to room temperature. The solid was collected and recrystallized from MeOH to give 2.6 g of product, mp 263°C dec.

Example 7

*N*-[2-[2-(Diethylamino)ethyl]-2*H*-[1]benzothiopyrano[4,3,2-*cd*]indazol-5-yl]acetamide, hydrochloride salt

A solution of 3.0 g (0.0089 mol) of 5-amino-*N,N*-diethyl-2*H*-[1]benzothiopyrano[4,3,2-*cd*]indazol-2-ethanamine in 30 ml of pyridine was treated dropwise with 0.9 g (0.011 mol) of acetyl chloride, stirred at 25°C for 30 minutes, and filtered. The solid was recrystallized from EtOH to provide 2.6 g of product, mp 208—211°C.

The free base, mp 151—154°C, may be prepared by dissolving the above product in a minimum amount of hot EtOH followed by treatment with an excess of 1*N* NaOH.

Example 8

2-Chloro-*N*-[2-[2-(diethylamino)ethyl]-2*H*[1]benzothiopyrano[4,3,2-*cd*]indazol-5-yl]acetamide

A mixture of 10.2 g (0.030 mol) of 5-amino-*N,N*-diethyl-2*H*[1]benzothiopyrano[4,3,2-*cd*]indazole-2-ethanamine and 8.3 ml (0.060 mol) of $Et_3N$ in 90 ml of $CHCl_3$ was treated dropwise with a solution of 3.6 ml (0.045 mol) of chloroacetyl chloride in 20 ml of $CHCl_3$ over ten minutes. The reaction mixture was stirred at room temperature for three hours, treated with an additional 1.2 ml (0.015 mol) of chloroacetyl chloride in 50 ml of $CHCl_3$, and concentrated to dryness in vacuo. The residue was triturated three times with cold MeOH to give 8.3 g of product, mp 204—205°C dec.

Example 9

*N'*-[2-[2-(Diethylamino)ethyl]-2*H*[1]benzothiopyrano[4,3,2-*cd*]indazol-5-yl]-*N*,*N*-dimethylmethanimidamide

A solution of 3.0 g (0.089 mol) of 5-amino-*N*,*N*-diethyl-2*H*[1]benzothiopyrano[4,3,2-*cd*]indazole-2-ethanamine in 100 ml of DMF was treated with 1.3 g (0.011 mol) of *N*,*N*-dimethylformamide, dimethyl acetal. The reaction mixture was heated at 90°C for 18 hours, treated with an additional 0.6 g (0.0052 mol) of *N*,*N*-dimethylformamide, dimethyl acetal, stirred at 90°C for an additional 18 hours, and poured into 1 l of $H_2O$. The solid that accumulated was collected and recrstallized from cyclohexane to give 2.8 g of product, mp 108—109°C.

Example 10

3-[2-[[2-[2-(Diethylamino)ethyl]-2H[1]benzothiopyrano[4,3,2-*cd*]indazol-5-yl]amino]ethyl]-2-oxazolidinone

A mixture of 5.8 g 0.017 mol) of 5-amino-*N*,*N*-diethyl-2*H*[1]benzothiopyrano[4,3,2-*cd*]indazole-2-ethanamine and 5.1 g (0.034 mol) of 3-(2-chloroethyl)-2-oxazolidinone was stirred at 150°C for two hours, dissolved in 100 ml of $CHCl_3$ and washed twice with 2*H* NaOH. The solution was dried ($MgSO_4$) and concentrated in vacuo to dryness. The residue was flash chromatographed over silica gel, eluting with a $CHCl_3$/MeOH (4/1) mixture. The appropriate fractions were combined and concentrated to dryness in vacuo. The residue was treated with 20 ml of acetone and the resulting suspension was treated with 150 ml of $Et_2O$. The resulting precipitate was collected and dried to give 4.9 g of product, mp 90—94°C.

Example 11

*N*,*N*-Diethyl-5-[[2-(dimethylamino)ethyl]amino]2*H*[1]benzothiopyrano[4,3,2-*cd*]indazol-2-ethanamine

A mixture of 5.0 g (0.015 mol) of 5-amino-*N*,*N*-diethyl-2*H*[1]benzothiopyrano[4,3,2-*cd*]indazole-2-ethanamine, 2.4 g (0.015 mol) of 2,2-diethoxy-*N*,*N*-dimethyl ethanamine, and 0.001 g of p-toluenesulfonic acid in 100 ml of 2-propanol was heated under reflux for four hours, allowed to cool to room temperature, and treated portionwise with 1.0 g (0.026 mol) of $NaBH_4$ over a two hour period. The mixture was stirred at room temperature for 16 hours and poured into 1 l of $H_2O$. The precipitate that formed was collected, washed with $H_2O$, dried, and recrystallized to give the product.

An example of another 5-(monoalkylated or acylated)-2-(substituted)benzothiopyrano[4,3,2-*cd*]indazole, prepared in the manner of Examples 5—11, is as follows:

*N*-[2-[2-(diethylamino)ethyl-2*H*[1]benzothiopyrano[4,3,2-*cd*]indazol-5-yl]-1,3-propanediamine, hydrochloride salt, mp 222°C dec.

Example 12

*N*-[2-(Acetyloxy)ethyl]-*N*-[2-(5-nitro-2*H*[1]benzothiopyrano[4,3,2-*cd*]indazole-2-yl)ethyl]acetamide

A mixture of 1.0 g (0.0025 mol) of 2-[[2-(5-nitro-2*H*[1]benzothiopyrano[4,3,2-*cd*]indazol-2-yl)ethyl]amino]ethanol and 0.5 g (0.0053 mol) of sodium acetate in 50 ml of $Ac_2O$ was stirred at 25°C for 24 hours and poured into 500 ml of $H_2O$. The precipitate that accumulated was collected and dried to give 1.0 g of product, mp 113—116°C.

Example 13

5-Nitro-N-[2-(phenylmethoxy)ethyl]-N-(phenylmethyl)2H[1]benzothiopyrano[4,3,2-*cd*]indazole-2-ethanamine, hydrochloride

*Method A*

A mixture of 5.0 g (0.017 mol) of 1-chloro-4-nitro-9*H*-thioxanthen-9-one, 6.0 g (0.020 mol) of 2-[*N*-[2-(phenylmethoxy)ethyl]-*N*-(phenylmethyl)aminoethyl]hydrazine, 5 ml of triethylamine, and 100 ml of DMF was stirred at 25°C for one hour, then at 80°C for 15 minutes. The mixture was poured into water and the solution was extracted with dichloromethane. The dried dichloromethane layer was chromatographed on silica gel, eluting first with dichloromethane then 9:1 dichloromethane-methanol to give the product as an oil. The oil was dissolved in 20 ml of dichloromethane and treated with 2-propanol:ether saturated with gaseous HCl. The solid was collected and dried in vacuo to give 6.6 g of product, mp 210—214°C.

*N*-(2-hydrazinoethyl)-*N*-[2-(phenylmethoxy)ethyl]benzenementhanamine is prepared as follows: A solution of 10.0 g (0.033 g) of N-(2-chloroethyl)-N-[2-(phenylmethoxy)ethyl]benzenemethanamine [Nador, Kovatsits, and Gyermek, *Acta Chim. Acad. Sci. Hung. 2,* 153 (1952)], 52.7 g (1.65 mol) of anhydrous hydrazine, and 210 ml of absolute ethanol was stirred at 25°C for 18 hours. The mixture was poured into cold water and the solution was extracted with ether (3 × 250 ml). The combined ether layers were dried ($Na_2SO_4$) and concentrated at less than 35°C to give 7.2 g of the product as an unstable oil.

*Method B*

A mixture of 1.0 g (0.0025 mol) of 2-[(2-5-nitro-2*H*[1]benzothiopyrano[4,3,2-*cd*]indazol-2-yl)ethyl]amino]ethanol in 50 ml of DMF at 25°C was treated with a suspension of 0.12 g (0.005 mol) of NaH in 20 ml of DMF, stirred at 25°C for 15 minutes, and treated with 0.9 g (0.0053 mol) of benzyl bromide. The mixture was stirred at 60°C for two hours and poured into 300 ml of $H_2O$. Workup as in Method A gave the product.

Example 14

3-[2-(5-Nitro-2*H*[1]benzothiopyrano[4,3,2-*cd*]indazol-2-yl)ethyl]-2-oxazolidinone

A mixture of 1.0 g (0.0025 mol) of 2-[[2-(5-nitro-2*H*[1]benzothiopyrano[4,3,2-*cd*]indazol-2-yl)ethyl]amino]ethanol, 10.0 g (0.045 mol) of diphenyl carbonate, 0.3 g (0.0036 mol) of NaOAc and 10.0 g of phenol was heated at 110°C for 16 hours, allowed to cool to room temperature, and triturated twice in a small amount of acetone. The insoluble material was recrystallized to give the product.

An example of another 5-nitro-2-(substituted)-2*H*[1]benzothiopyrano[4,3,2-*cd*]indazole where the 2-substituent contains one or more reactive groups such as NH, $NH_2$, or OH, prepared as described in Examples 12 through 14 to protect these functionalities, is as follows:

*N*-[2-(5-nitro-2*H*[1]benzothiopyrano[4,3,2-*cd*]indazol-2-yl)ethyl]acetamide, mp 259°C dec.

These derivatized compounds are in turn reduced to the 5-amino compounds as described in Example 4 above, except when a benzyl group is used as the protecting group. In this instance, the reductions are carried out in MeOH, THF, or MeOH/THF mixtures using Raney nickel as the catalyst.

Example 15

2-[[2-[5-[[2-(Diethylamino)ethyl]amino]-2*H*[1]benzothiopyrano[4,3,2-*cd*]indazol-2-yl]ethyl]amino]ethanol, hydrochloride salt

A mixture of 3.3 g (0.008 mol) of *N*-[2-(acetyloxy)ethyl]-*N*-[2-(5-amino-2*H*[1]benzothiopyrano[4,3,2-*cd*]indazol-2-yl)ethyl]acetamide, 3.2 g (0.012 mol) of 2-(diethylamino)ethyl bromide, hydrobromide, and 2.5 g (0.018 mol) of $K_2CO_3$ in 250 ml of toluene was heated under reflux for four hours, allowed to cool to room temperature overnight, and filtered. The filtrate was concentrated in vacuo to dryness and the residue was flash chromatographed over 200 g of silica gel, eluting with a $CH_2Cl_2$/MeOH (6/1) mixture. The appropriate fractions were combined and concentrated to dryness in vacuo to give *N*-[2-(acetyloxy)ethyl]-*N*-[2-[5-[(2-diethylamino)ethyl]-2*H*[1]benzothiopyrano[4,3,2-*cd*]indazol-2-yl]ethyl]acetamide as an oil. This material was treated with 150 ml of 2*N* HCl, heated under reflux for one hour, and allowed to cool to room temperature overnight. The reaction mxiture was made basic with 50% aq NaOH and the precipitate that formed was collected, dried in vacuo, and dissolved in 200 ml of acetone. The solution was treated dropwise with i-PrOH saturated with gaseous HCl until precipitation was complete. The solid was collected and dried in vacuo to give 0.75 g of product, mp 208—212°C.

Example 16

2-[[2-[[2-[2-(Diethylamino)ethyl]-2*H*[1]benzothiopyrano[4,3,2-*cd*]indazol-5-yl]amino]ethyl]amino]ethanol, hydrochloride salt

A mixture of 2.2 g (0.0049 mol) of 3-[2-[[2-[2-(diethylamino)ethyl]-2*H*[1]benzothiopyrano[4,3,2-*cd*]indazol-5-yl]amino]ethyl]-2-oxazolidinone, 20 ml of 2*N* KOH in MeOH, 10 ml of $H_2O$, and 10 ml of THF was heated under reflux under a $N_2$ atmosphere for 14 hours and poured into 250 ml of $H_2O$. The mixture was extracted three times with $CH_2Cl_2$ and the $CH_2Cl_2$ extracts were combined, dried ($MgSO_4$), and concentrated to dryness in vacuo. The residue was dissolved in 125 ml of i-PrOH and treated dropwise with i-PrOH saturated with gaseous HCl until precipitation was complete. The solid was collected and triturated in a boiling EtOH/i-PrOH (1/1) mixture to give 2.1 g of product, mp 202—210°C.

Example 17

2-[[2-[[5-[[2-(Diethylamino)ethyl]amino]-2*H*[1]benzothiopyrano[4,3,2-*cd*]indazol-2-yl]ethyl]amino]ethanol, hydrochloride salt

A mixture of 5.0 g (0.0082 mol) of 5 - [[2 - diethylamino]ethyl]amino] - *N* - [2 - (phenylmethoxy)ethyl - *N* - (phenylmethyl) - 2*H*[1]benzothiopyrano[4,3,2 - *cd*]indazole - 2 - ethanamine, 0.2 g of 20% Pd/C, and 100 ml of HOAc was hydrogenated in a Parr shaker at 25°C and an initial pressure of 50 psi. The mixture was filtered and the filtrate was concentrated in vacuo to a small volume, treated with 300 ml of $H_2O$, made basic with 50% aqueous NaOH, and extracted three times with $CHCl_3$. The extracts were combined, dried ($MgSO_4$), and concentrated in vacuo to dryness. The residue was dissolved in a minimum amount of acetone and treated with i-PrOH saturated with gaseous HCl until preciptation was complete. The solid was collected and recrystallized to give the product, mp 208—212°C.

Example 18

2-[[2-[5-[[2-[(2-Hydroxyethyl)amino]ethyl]amino]-2*H*[1]benzothiopyrano[4,3,2-*cd*]indazol-2-yl]ethyl]amino]ethanol, hydrochloride salt

A mixture of 5.1 g (0.012 mol of *N*-[2-(acetyloxy)ethyl]-*N*-[2-(5-amino-2*H*[1]benzothiopyrano[4,3,2-*cd*]indazol-2-yl)ethyl]acetamide, hydrochloride salt and 11.6 g (0.077 mol) of 3-(β-chloroethyl)-2-oxazolidinone was stirred at 150°C for 2 hours, dissolved in 20 ml of a $CH_2Cl_2$/MeOH (20/1) mixture, and flash chromatographed over silica gel, eluting with a $CH_2Cl_2$/MeOH (20/1) mixture. The appropriate fractions were combined and concentrated to dryness in vacuo. The residue was treated with 20 ml of 2*N* KOH in MeOH, 10 ml of $H_2O$, and 10 ml of THF, and the mixture was heated under reflux under a $N_2$ atmosphere for 16 hours. The mixture was treated with 100 ml of $H_2O$ and 100 ml of $CH_2Cl_2$ and the layers were separated. The aqueous phase was extracted twice with $CH_2Cl_2$ and the $CH_2Cl_2$ extracts were combined, dried ($MgSO_4$), and concentrated to dryness in vacuo. The residue was dissolved in 50 ml of

EtOH and treated with an excess of i-PrOH saturated with gaseous HCl. The precipitate was collected and recrystallized from MeOH to give 0.6 g of product, mp 222—225°C.

Other 5-(substituted amino)-2-(substituted)-2*H*[1]benzothiopyrano[4,3,2-*cd*]indazoles where the 2-substituent contains reactive groups such as NH, $NH_2$, or OH which have been protected, prepared in the manner of Examples 15—18, are as follows:

3-[2-[[2-[2-[(2-hydroxyethyl)amino]ethyl-2*H*[1]benzothiopyrano[4,3,2-*cd*]indazol-5-yl]amino]ethyl-2-oxazolidinone, hydrochloride salt, mp 180—182°C.

*N*-[2-[5-[[2-(2-oxo-3-oxazolidinyl]ethyl]amino]-2*H*[1]benzothiopyrano[4,3,2-*cd*]indazol-2-yl]ethyl]acetamide, mp 145—147°C.

2-[[2-[[2-(2-aminoethyl)-2*H*[1]benzothiopyrano[4,3,2-*cd*]indazol-5-yl]amino]ethyl]amino]ethanol, hydrochloride salt, mp 264°C dec.

Example 19

*N*-[2-(Diethylamino)ethyl]-*N*-[2-[2-(diethylaminoethyl]-2*H*[1]benzothiopyrano[4,3,2-*cd*]indazol-5-yl]acetamide

A solution of 5.0 g (0.011 mol) of 5-[[(2-diethylamino)ethyl]amino]-*N*,*N*-diethyl-2*H*[1]benzothiopyrano[4,3,2-*cd*]indazol-2-ethanamine in 100 ml of pyridine was treated with 1.2 g (0.015 mol) of acetyl chloride, heated under reflux for two hours, and poured into 1 l of $H_2O$. The precipitate that formed was collected, dried, and recrystallized to give the product.

Other 5-(monosubstituted amino)-2-(substituted)-2*H*[1]benzothiopyrano[4,3,2-*cd*]indazoles are acylated at $N_5$ in like manner.

Example 20

5-[[*N*'-[2-(Diethylamino)ethyl]-*N*-ethyl]amino]*N*,*N*-diethyl-2*H*[1]benzothiopyrano[4,3,2-*cd*]indazol-2-ethanamine

Twenty-five milliliters of a 1*M* solution of $LiAlH_4$ (0.025 mol) in THF at 25°C was treated dropwise with a solution of 5.0 g (0.01 mol) of *N*-[2-diethylamino)ethyl-*N*-[2-[2(diethylamino)ethyl]-2*H*[1]benzothiopyrano[4,3,2-*cd*]indazol-5-yl]acetamide in 25 ml of THF over a 30 minute period. The mixture was heated under reflux for two hours, allowed to cool to room temperature, and treated dropwise with $H_2O$ until the excess $LiAlH_4$ was decomposed. The mixture was treated with 10 g of $MgSO_4$, stirred for 30 minutes, and filtered. The insoluble material was washed with THF three times and the filtrates were combined and concentrated in vacuo to dryness. The residue was crystallized and dried to give the product.

Other 5-(dialkylamino)-2-(substituted)-2*H*[1]benzothiopyrano[4,3,2-*cd*]indazoles are prepared from the corresponding acylated intermediates in like manner.

Example 21

*N*,*N*-Diethyl-7,10-dimethoxy-5-nitro-2*H*[1]benzothiopyrano[4,3,2-*cd*]indazol-2-ethanamine

A mixture of 16.2 g (0.046 mol) of 1-chloro-5,8-dimethoxy-4-nitro-9*H*-thioxanthen-9-one and 9.1 g (0.069 mol) of 2-(diethylaminoethyl)hydrazine in 400 ml of DMF was stirred at 25°C for six hours. The precipitate that accumulated was collected and dried to give 14.8 g of product, mp 188—190°C.

1-Chloro-5,8-dimethoxy-4-nitro-9*H*-thioxanthen-9-one is prepared as follows: A solution of 9.5 g (0.048 mol) of 2-amino-3,6-dimethoxybenzoic acid [P. K. Bannerjee and D. N. Chaudhury, *J. Indian Chem. Soc.*, 86 (4); 257 (1959)], 4.6 ml (0.11 mol) of 50% aqueous NaOH, 60 ml of $H_2O$, and 3.3 g (0.048 mol) of $NaNO_2$ was added slowly to a mixture of 15 ml of concentrated HCl and 20 g of ice chips which had been previously cooled in a salt-ice bath to −5°C. Good stirring was maintained throughout the addition and the temperature was kept below 5°C. After the addition was complete, the mixture was stirred to 0°C for one hour, neutralized (pH 5.5) with potassium acetate, and added while cold in a thin stream to an 80°C solution of 22.2 g (0.15 mol) of potassium ethyl xanthate in 75 ml of $H_2O$ under $N_2$. Copious $N_2$ evolution (foaming) occurred during the addition, and heat was applied as needed to maintain the temperature at 75—80°C. The reaction mixture, under $N_2$, was cooled to 20°C and acidified (pH 3) with concentrated HCl. The oily material which separated was extracted into $CH_2Cl_2$ (2×), keeping contact with air to a minimum. The extracts were combined, dried under $N_2$ ($MgSO_4$), and concentrated on a steam bath to a brown oil using a stream of $N_2$. The crude 2,5-dimethoxy-6-thiobenzoic acid was immediately dissolved in 40 ml of hot anhydrous EtOH and added to a pre-mixed, 25°C mixture of 9.2 g (0.048 mol) of 2,4-dichloronitrobenzene in sodium ethoxide [2.2 g (0.096 g atom) of sodium spheres dissolved in 90 ml of anhydrous EtOH]. The resulting suspension was heated under reflux for 16 hours, concentrated to dryness in vacuo and taken up in 250 ml of ether and 250 ml of $H_2O$. The layers were separated and the aqueous layer was extracted twice with ether to remove organic soluble inpurities, and made acidic (pH 1) with concentrated HCl. The solid which formed was collected, dried, and recrystallized from EtOH to give a first crop of product. The mother liquor was concentrated to dryness in vacuo and the residue was crystallized from $CH_3CN$ to give a second crop of product. The mother liquor was concentrated to dryness in vacuo and the residue was flash chromatographed over 500 g of silica gel, eluting with $CH_2Cl_2$/MeOH (15/1). Combination of the appropriate fractions, concentration to dryness in vacuo, and crystallization of the residue from acetonitrile provided a third crop of product. All crops were combined to give 7.8 g of 2-[(5-chloro-2-nitrophenyl)thio]-3,6-dimethoxybenzoic acid, mp 218—220°C.

A mixture of 19.7 g (0.053 mol) of the above benzoic acid, 600 ml of trifluoroacetic acid, and 300 ml of trifluoroacetic anhydride was stirred at 50°C for four hours, treated with an additional 70 ml of trifluoroacetic anhydride, and stirred at 50°C for 20 hours. The reaction mixture was poured into 9 l of $H_2O$ and the precipitate that accumulated was collected and dried to give 16.4 g of 1-chloro-5,8-dimethoxy-4-nitro-9*H*-thioxanthen-9-one, mp 222—228°C.

Other 10-hydroxy-7-methoxy-, 10-methoxy-7-hydroxy-, and 7,10-dimethoxy-5-nitro-2-(substituted)-2*H*[1]benzothiopyrano[4,3,2-*cd*]indazoles can be prepared in the manner of Examples 1 through 3 and 21. One such compound is 2-[2-(diethylamino)-ethyl]-7-methoxy-5-nitro-2*H*[1]benzothiopyrano[4,3,2-*cd*]indazol-10-ol, hydrochloride salt, mp 267—270°C. The latter compound is prepared from 1-chloro-5-methoxy-8-hydroxy-4-nitro-9*H*-thioxanthen-9-one which in turn is prepared as follows: A suspension of 5.8 g (0.016 mol) of 2-[(5-chloro-2-nitrophenyl)thio]-3,6-dimethoxybenzoic acid, 50 ml of toluene, and 3.8 g (0.032 mol) of thionyl chloride was heated under reflux for two hours, concentrated to dryness in vacuo, and dissolved in 50 ml of nitrobenzene. The solution was treated portionwise with 3.6 g (0.026 mol) of $AlCl_3$, keeping the temperature below 35°C during the addition. The mixture was stirred for two hours at 70°C, chilled in a refrigerator overnight, and poured into 400 ml of ice-cold $H_2O$. The mixture was extracted four times with $CH_2Cl_2$ and the extracts were combined, dried ($MgSO_4$), and concentrated in vacuo to dryness. The residue was triturated successively in petroleum ether and hot MeOH, and recrystallized from DMF to give 1.2 g of product, 272—273°C.

## Example 22

5-Amino-*N,N*-diethyl-7,10-dimethoxy-2*H*[1]benzothiopyrano[4,3,2-*cd*]indazole-2-ethanamine

3.1 g of *N,N*-diethyl-7,10-dimethoxy-5-nitro-2*H*[1]benzothiopyrano[4,3,2-*cd*]indazole-2-ethanamine was hydrogenated and the product was isolated as described in Example 4 to give 2.3 g, mp 134—139°C.

Other 5-amino-7,10-(dimethoxy, hydroxymethoxy, and dihydroxy)-2-(substituted)-2*H*[1]benzothiopyrano[4,3,2-*cd*]indazoles can be prepared from the appropriate 7 and/or 10-methoxy-2-(substituted)-5-nitro-2*H*[1]benzothiopyrano[4,3,2-*cd*]indazoles in the manner of Example 4. One such compound is 5-amino-2-[2-(diethylamino)ethyl]-2*H*[1]-benzothiopyrano[4,3,2-*cd*]indazole-7,10-diol, hydrochloride salt, mp 229—233°C.

## Example 23

3-[2-[[2-[2-(Diethylamino)ethyl]-7,10-dimethoxy-2*H*[1]benzothiopyrano[4,3,2-*cd*]indazol-5-yl]amino]ethyl]-2-oxazolidinone

A mixture of 2.2 g (0.0055 mol) of 5-amino-*N,N*-diethyl-7,10-dimethoxy-2*H*[1]benzothiopyrano[4,3,2-*cd*]indazole-2-ethanamine and 1.6 g (0.011 mol) of 3-(β-chloroethyl)-2-oxoazolidinone was treated in the manner described in Example 10 to furnish 1.6 g of the title compound, mp greater than 250°C dec.

Other 7,10-dimethoxy-5-(alkylamino or acylamino)-2-(substituted)-2*H*[1]benzothiopyrano[4,3,2-*cd*]indazoles are prepared in the manner of Examples 5 through 11, with the exception of those compounds which contain side chains with reactive groups such as NH, $NH_2$, or OH. In such cases, the reactive groups must first be protected as described in Examples 12 through 14.

These derivatized compounds are in turn reduced to the 5-amino compounds as described in Example 4, except when benzyl groups are used as the protecting groups. In this instance, the reductions are carried out in MeOH, THF, or MeOH/THF mixtures using Raney nickel as the catalyst. The 5-amino compounds are then derivatized in the manner of Examples 5, 7, 8, 9, 10, and 15 to provide 7,10-dimethoxy-5-(monoalkylated or acylated)-2-(substituted)-2*H*[1]benzothiopyrano[4,3,2-*cd*]indazoles.

7,10-Dimethoxy-5-(monoalkylamino or acylamino-2-(substituted))-2*H*[1]benzothiopyrano[4,3,2-*cd*]indazoles may be acylated at $N_5$ in the manner of Example 18 and the acyl derivatives are reduced using $LiAlH_4$ in the manner of Example 20 to provide 7,10-dimethoxy-5-(disubstituted amino)-2-(substituted)-2*H*[1]benzothiopyrano[4,3,2-*cd*]indazoles.

## Example 24

2-[[2-[[2-[2-(Diethylamino)ethyl]-7,10-dimethoxy-2*H*[1]benzothiopyrano[4,3,2-*cd*]indazol-5-yl]amino]ethyl]amino]ethanol

A mixture of 5.9 g (0.012 mol) of 3-[2-[[2-[2-diethylamino)ethyl]-7,10-dimethoxy-2*H*[1]benzothiopyrano[4,3,2-*cd*]indazol-5-yl]amino]ethyl]-2-oxazolidinone, 70 ml of 2*N* KOH in MeOH, 50 ml of THF, and 35 ml of $H_2O$ was treated and the product isolated as described in Example 16 with the following exception: after concentrating the $CHCl_3$ extracts in vacuo to dryness, the residue was crystallized from $CH_3CN$ to give 1.9 g of product, mp 119—123°C.

Other 7,10-dimethoxy-2,5-(disubstituted)-2*H*[1]benzothiopyrano[4,3,2-*cd*]indazoles are prepared in the manner of Examples 15—18.

## Example 25

2-[2-(Diethylamino)ethyl]-5-[[2-[(2-hydroxyethyl)amino]ethyl]amino]-2*H*[1]benzothiopyrano[4,3,2-*cd*]indazole-7,10-diol, hydrobromide salt

A suspension of 1.9 g (0.0039 mol) of 2 - [[2 - [[2 - [2 - (diethylamino)ethyl] - 7,10 - dimethoxy - 2*H*[1]benzothiopyrano[4,3,2 - *cd*]indazol - 5 - yl]amino]ethyl]amino]ethanol in 100 ml of ethylene

dichloride was treated via syringe with 4.5 ml (12 g, 0.047 mol) of boron tribromide, stirred at 25°C for two days, treated with an additional 2.0 ml (5.2 g, 0.021 mol) of boron tribromide, and heated at 50°C for two hours. The reaction mixture was cooled to 5°C, treated dropwise with 100 ml of MeOH, and evaporated to 50 ml using a stream of air. The precipitate was collected and recrystallized from MeOH to give, in two crops, 2.0 g of product, mp 243—245°C.

Other 2,5-(disubstituted)-2*H*[1]benzothiopyrano[4,3,2-*cd*]indazole-7,10-diols, are prepared from the corresponding 7,10-dimethoxy precursors in the manner of Example 25. One such compound is 2-[2-(diethylamino)ethyl]-5-nitro-2*H*[1]benzothiopyrano[4,3,2-*cd*]indazole-7,10-diol, hydrobromide salt, mp 283°C dec.

Example 26

*N,N*-Diethyl-9-methoxy-5-nitro-2*H*[1]benzothiopyrano[4,3,2-*cd*]indazole-2-ethanamine

A mixture of 1.2 g (0.0091 mol) of 2-(diethylaminoethyl)hydrazine, 2.0 g (0.0069 mol) of 1-chloro-7-methoxy-4-nitro-9*H*-thioxanthen-9-one, and 0.97 g (0.0069 mol) of powdered $K_2CO_3$ in 30 ml of xylene was heated at 70°C for 1.5 hours, chilled in an ice bath, and filtered. The solid was triturated in $H_2O$ and dried to give 1.4 g of product, mp 154—156°C. The product was taken up in 30 ml of boiling EtOH, treated with 4.0 ml (0.004 mol) of 1*N* methanesulfonic acid in EtOH, and chilled. The resulting precipitate was collected and dried to give 1.8 g of product as the methanesulfonic acid salt, mp 240—244°C.

Dissolution of 1.0 g of the product in EtOH followed by treatment with an excess of i-PrOH saturated with gaseous HCl provided 0.8 g of the product as the hydrochloride salt, mp 275°C dec.

1-Chloro-7-methoxy-4-nitro-9*H*-thioxanthen-9-one is prepared as follows:

*Route A*

A solution of 27.6 g (0.16 mol) of 2-amino-5-methoxybenzoic acid [N. B. Chapman, G. M. Gibson, and F. G. Mann, *J. Chem. Soc.,* 890 (1947)], 16.0 ml (0.38 mol) of 50% aqueous NaOH, 220 ml of $H_2O$, and 11.4 g (0.16 mol) of $NaNO_2$ was added slowly to a mixture of 50 ml of concentrated HCl and 65 g of ice chips which had been previously cooled in a salt-ice bath to −5°C. Good stirring was maintained throughout the addition and the temperature was kept below 5°C. After the addition was complete, the mixture was stirred at 0°C for one hour, neutralized (pH 5.1) with potassium acetate, and added while cold in a thin stream to an 80°C solution of 76.9 g (0.48 mol) of potassium ethyl xanthate in 275 ml of $H_2O$ under $N_2$. Copious $N_2$ evolution (foaming) occurred during the addition, and heat was applied as needed to maintain the temperature at 75—80°C. The reaction mixture, under $N_2$, was cooled to 20°C and acidified (pH 3) with concentrated HCl. The mixture was treated with 200 ml of $CH_2Cl_2$, shaken, and filtered to remove an insoluble solid. The layers were separated and the aqueous phase was extracted with a second 200 ml portion of $CH_2Cl_2$, keeping contact with air to a minimum. The extracts were combined, dried under $N_2$ ($MgSO_4$), and concentrated in vacuo to dryness.

The crude 5-methoxy-2-thiobenzoic acid was immediately dissolved in 140 ml of hot anhydrous EtOH and added to a premixed, 25°C mixture of 31.7 g (0.16 mol) of 2,4-dichloronitrobenzene in sodium ethoxide [7.6 g (0.33 g-atom) of sodium spheres dissolved in 330 ml of anhydrous EtOH]. The resulting suspension was heated under reflux for one hour, concentrated to dryness in vacuo and taken up in 400 ml of ether and 1 l of $H_2O$. The layers were separated and the aqueous layer was extracted twice with ether to remove organic soluble impurities and made acidic (pH 1) with concentrated HCl. The solid which formed was collected, dried, and recrystallized from EtOH to give, in two crops, 19.8 g of 2-[(5-chloro-2-nitrophenyl)-thio]-5-methoxybenzoic acid, mp 184—186°C.

A mixture of 17.6 g (0.058 mol) of the above benzoic acid, 90 ml of toluene, and 4.6 ml (0.064 mol) of thionyl chloride was heated under reflux for two hours, concentrated to dryness in vacuo, and dissolved in 140 ml of nitrobenzene. The solution was treated portionwise with 7.7 g (0.058 mol) of $AlCl_3$, keeping the temperature below 35°C during the addition. The mixture was stirred at room temperature for 20 hours and poured into 800 ml of ice-cold $H_2O$. The mixture was stirred for one hour and the $H_2O$ was decanted from the tarry residue. The mass was washed with $H_2O$ and triturated in boiling MeOH to give 8.2 g of product, mp 235—238°C. Other 1-chloro-4-nitro-9*H*-thioxanthen-9-ones containing an alkoxy or benzyloxy substituent at positions 5, 6, 7, or 8 may be prepared in an analogous manner starting from appropriately substituted benzoic acids.

*Route B*

An ice-cooled suspension of 3.0 g (0.125 mol) of oil-free sodium hydride in 100 ml of tetrahydrofuran was treated portionwise during ten minutes with 12.2 g (0.052 mol) of 2,6-dichloro-3-nitrobenzoic acid [Lehmstedt and Schrader, *Ber.* 70B; 1526 (1937)]. After stirring for ten minutes, the suspension was treated dropwise with 7.0 g (0.05 mol) of 4-methoxybenzenethiol [C. M. Suter and H. L. Hansen, *J. Am. Chem. Soc.,* 54; 4100 (1934)] in 50 ml of tetrahydrofuran. After stirring for 30 minutes at 0°C, the cooling bath was removed and the mixture was maintained at 25°C for 12 hours. The mixture was acidified with 150 ml of 10% aqueous HCl, then trated with 200 ml of ethyl acetate. The organic layer was separated and the aqueous phase was extracted with 100 ml of ethyl acetate. The combined organic phases were dried ($MgSO_4$), and concentrated to a yellow solid which was purified by flash chromatography on silica gel, utilizing dichloromethane:methanol (8:1) as eluting solvent, to give 11.9 g of 6-chloro-2-[(4-methoxyphenyl)thio]-3-nitrobenzoic acid, mp 154—157°C, following crystallization from toluene.

A mixture of 10.2 g (0.03 mol) of the above benzoic acid, 360 ml of trifluoroacetic acid, and 180 ml of trifluoroacetic anhydride was stirred at room temperature for 12 hours. The solution was concentrated and the residual solid was triturated from methanol to give 9.2 g of product, mp 234—237°C. Other 1-chloro-4-nitro-9*H*-thioxanthen-9-ones containing an alkoxy or benzyloxy substituent at positions 5, 6, 7, or 8 may be prepared in an analogous manner starting from appropriately substituted benzenethiols.

Example 27

*N,N*-Diethyl-5-nitro-9-phenylmethoxy-2*H*[1]benzothiopyrano[4,3,2-*cd*]indazole-2-ethanamine

A mixture of 1.2 g (0.0091 mol) of 2-(diethylaminoethyl)hydrazine, 2.7 g (0.0069 mol) of 1-chloro-4-nitro-7-phenylmethoxy-9*H*-thioxanthen-9-one, and 0.97 g (0.0069 mol) of powdered $K_2CO_3$ in 30 ml of xylene was reacted and 2.0 g of the product was isolated as described in Example 26.

Other 9-(methoxy, phenylmethoxy, or *p*-halo or *p*-methoxy-substituted phenylmethoxy)-5-nitro-2-(substituted)-2*H*[1]benzothiopyrano[4,3,2-*cd*]indazoles are prepared in the manner of Examples 1 through 3, 21, 26, and 27.

Example 28

5-Amino-*N,N*-diethyl-9-methoxy-2*H*[1]benzothiopyrano[4,3,2-*cd*]indazole-2-ethanamine

3.0 g of *N,N*-diethyl-9-methoxy-5-nitro-2*H*[1]benzothiopyrano[4,3,2-*cd*]indazole-2-ethanamine was hydrogenated and the product was isolated as described in Example 4 to give 1.8 g of product, mp 152—153°C.

Other 5-amino-9-methoxy-2-(substituted)-2*H*[1]benzothiopyrano[4,3,2-*cd*]indazoles are prepared from the appropriate 9-methoxy-2-(substituted)-5-nitro-2*H*[1]benzothiopyrano[4,3,2-*cd*]indazoles in the manner of Example 4.

Example 29

5-Amino-*N,N*-diethyl-9-phenylmethoxy-2*H*[1]benzothiopyrano[4,3,2-*cd*]indazole-2-ethanamine

A mixture of 0.47 g (0.001 mol) of *N,N*-diethyl-5-nitro-9-phenylmethoxy-2*H*[1]benzothiopyrano[4,3,2-*cd*]indazole-2-ethanamine, 0.5 g of Raney nickel, and 100 ml of MeOH was hydrogenated in a Parr shaker at 25°C and an initial pressure of 50.0 psi. The mixture was filtered and the filtrate was concentrated to dryness. Crystallization of the residue from the appropriate solvent furnished the product.

Other 5-amino-9-(phenyl or substituted phenyl)-methoxy-2-(substituted)-2*H*[1]benzothiopyrano[4,3,2-*cd*]indazoles are prepared from the appropriate 5-nitro-9-phenylmethoxy-2-(substituted)-2*H*[1]benzothiopyrano[4,3,2-*cd*]indazoles in the manner of Example 29.

Example 30

3-[2-[[2-[2-(Diethylamino)ethyl]-9-methoxy-2*H*[1]benzothiopyrano[4,3,2-*cd*]indazol-5-yl]amino]ethyl]-2-oxazolidinone

A mixture of 16.0 g (0.043 mol) 5-amino-*N,N*-diethyl-9-methoxy-2*H*[1]benzothiopyrano[4,3,2-*cd*]indazole-2-ethanamine and 32.0 g (0.21 mol) of 3-(β-chloroethyl)-2-oxazolidinone was reacted and the product was isolated to furnish 15.2 g, mp 135—138°C. The reaction was as described in Example 10, with the following exception: The reaction was run at 100°C for 13 hours instead of 150°C for 2 hours.

The product was dissolved in a minimum amount of hot EtOH and treated with i-PrOH saturated with gaseous HCl until precipitation was complete. The solid was collected and recrystallized from EtOH to give 1.1 g of the product as the hydrochloride salt, mp 212°C dec.

Other 7-, 8-, 9-, 10-(methoxy, phenylmethoxy, *p*-halo- or *p*-methoxy substituted phenylmethoxy)-5-(monoalkylated or acylated)-2-(substituted)-2*H*[1]benzothiopyrano[4,3,2-*cd*]indazoles are prepared in the manner of Examples 5 through 11, with the exception of those compounds which contain side chains with reactive groups such as NH, $NH_2$ or OH. In such cases, the reactive groups must first be protected as described in Examples 12 through 14.

These derivatized compounds are in turn reduced to the 5-amino compounds as described in Example 4, except when benzyl group(s) are used as the protecting group(s). In this instance, the reductions are carried out in MeOH, THF, or MeOH/THF mixtures using Raney nickel as the catalyst. The 5-amino compounds are then derivatized in the manner of Examples 5, 7, 8, 9, 10, and 15 to provide 7-, 8-, 9-, 10-(methoxy, phenylmethoxy, or *p*-halo- or *p*-methoxy-substituted phenylmethoxy)-5-(monoalkylated or acylated)-2-(substituted)-2*H*[1]benzothiopyrano[4,3,2-*cd*]indazoles.

7, 8-, 9-, 10-(Methoxy, phenylmethoxy, or *p*-halo- or *p*-methoxy-substituted phenylmethoxy)-5-(monoalkylated or acylated-2-(substituted)-2*H*[1]benzothiopyrano[4,3,2-*cd*]indazoles may be acylated at $N_5$ in the manner of Example 19 and the acyl derivatives reduced using $LiAlH_4$ in the manner of Example 20 to provide 7-, 8-, 9-, 10-(methoxy, phenylmethoxy, or *p*-halo- or *p*-methoxy-substituted phenylmethoxy)-5-(disubstituted amino)-2-(substituted)-2*H*[1]benzothiopyrano[4,3,2-*cd*]indazoles.

Example 31

2-[[2-[[2-[2-(Diethylamino)ethyl]-9-methoxy-2*H*[1]benzothiopyrano[4,3,2-*cd*]indazol-5-yl]amino]ethyl]amino]ethanol

A mixture of 8.0 g (0.017 mol) of 3-[2-[[2-[2-(diethylamino)ethyl]-9-methoxy-2*H*[1]benzothiopyrano[4,3,2-*cd*]indazol-5-yl]amino]ethyl]-2-oxazolidinone, 70 ml of 2*N* KOH in MeOH, 35 ml of THF, and 35 ml of $H_2O$ was treated and the product isolated as described in Example 24 to furnish 6.4 g of the product, mp 111—115°C.

## Example 32

5-Amino-2-[2-(diethylamino)ethyl]-2*H*[1]benzothiopyrano[4,3,2-*cd*]indazol-9-ol

A mixture of 2.0 g (0.0054 mol) of 5-amino-*N,N*-diethyl-9-methoxy-2*H*[1]benzothiopyrano[4,3,2-*cd*]indazole-2-ethanamine and 40 ml of 48% HBr was heated at 130°C under inert atmosphere ($N_2$) for three hours, allowed to cool to room temperature, and poured into 200 ml of $H_2O$. The mixture was made strongly basic with 50% aqueous NaOH. The pH was then adjusted to 8.5 with 2*N* HCl and the gum that formed was triturated successively with $CH_2Cl_2$, $Et_2O$, and $H_2O$, and recrystallized from EtOH to give 1.2 g of the product, mp 224—232°C.

The product (1.2 g) was dissolved in a minimum amount of hot EtOH and treated with i-PrOH saturated with gaseous HCl until precipitation was complete. The solid was collected and recrystallized from EtOH to give 1.2 g of the product as the hydrochloride salt, mp 279°C dec.

Other 2,5-(disubstituted)-2*H*[1]benzothiopyrano[4,3,2-*cd*]indazole-9-ols are prepared from the corresponding 9-methoxy precursors in the manner of Example 32.

## Example 33

2-[2-(Diethylamino)ethyl]-5-[[2-[(2-hydroxyethyl)amino]ethyl]amino]-2*H*[1]benzothiopyrano[4,3,2-*cd*]indazol-9-ol, hydrochloride salt

A mixture of 5.0 g (0.0094 mol) of 2-[[2-[[2-[2-(diethylamino)ethyl]-9-phenylmethoxy-2*H*[1]benzothiopyrano[4,3,2-*cd*]indazol-5-yl]amino]ethyl]amino]ethanol, 0.2 g of 20% Pd/C, and 100 ml of HOAc was hydrogenated in a Parr shaker at 25°C and an initial pressure of 50 psi. The mixture was filtered and the filtrate was concentrated in vacuo to a small volume, treated with 300 ml of $H_2O$, and the pH was adjusted to 6.5 with 50% aqueous NaOH. The mixture was extracted three times with $CHCl_3$ and the extracts were combined, dried ($MgSO_4$), and concentrated in vacuo to dryness. The residue was dissolved in a minimum amount of EtOH and treated with i-PrOH until precipitation was complete. The solid was collected and recrystallized to give the product.

Other 2,5-(disubstituted)-2*H*[1]benzothiopyrano[4,3,2-*cd*]indazol-7,8,9 or 10-ols are prepared from the corresponding 7,8,9 or 10-(phenyl and substituted phenyl)-methoxy precursors in the manner of Example 33.

## Example 34

3-[2-[[2-[2-(diethylamino)ethyl]-9-hydroxy-2*H*[1]benzothiopyrano[4,3,2-*cd*]indazol-5-yl]amino]ethyl]-2-oxazolidinone

A mixture of 3.5 g (0.0075 mol) of 3-[2-[[2-[2-(diethylamino)ethyl]-9-methoxy-2*H*[1]benzothiopyrano[4,3,2-*cd*]indazol-5-yl]amino]ethyl]-2-oxazolidinone and 15 ml of a one molar solution of boron tribromide in dichloromethane was heated at reflux for 15 minutes. The suspension was cooled and poured into saturated aqueous sodium bicarbonate. The aqueous mixture was extracted with a mixture of dichloromethane:methanol (4:1). The organic phase was washed with brine, dried ($Na_2SO_4$), and evaporated to a residue that was triturated with 2-propanol to give 2.6 g of product.

0.5 grams of the product was dissolved in a minimum amount of hot EtOH until precipitation was complete. The solid was collected and dried at 65°C to give 0.7 g of the product as the hydrochloride salt, mp 223—226°C (decomposition).

Other 2,5-(disubstituted)2*H*[1]benzothiopyrano[4,3,2-*cd*]indazole-9-ols are prepared from the corresponding 9-methoxy precursors in the manner of Example 34.

## Example 25

2-[2-(Diethylamino)ethyl]-5-[[2-[(2-hydroxyethyl]aminoethyl]amino-2*H*[1]benzothiopyrano[4,3,2-*cd*]indazol-9-ol

A solution of 2.0 g (0.0043 mol) of 3-[2-[[2-[2-(diethylamino)ethyl]-9-hydroxy-2*H*[1]benzothiopyrano[4,3,2-*cd*]indazol-5-yl]amino]ethyl]-2-oxazolidinone and 100 ml of 2*N* KOH in MeOH was heated at reflux for 18 hours and isolated as described in Example 16 to give the crude product. Chromatography over silica gel utilizing 3:1 ethyl acetate:MeOH as eluant gave 0.9 g of pure product. Salt formation as described n Example 34 gave 0.9 g of the product as the hydrochloride salt, mp 224—234°C (decomposition).

## Example 36

*N,N*-Diethyl-7-methoxy-5-nitro-2*H*[1]benzothiopyrano[4,3,2-*cd*]indazole-2-ethanamine

Reaction of a mixture of 1.8 g (0.0137 mol) of 2-(diethylaminoethyl)hydrazine, 3.0 g (0.0093 mol) of 1-chloro-5-methoxy-4-nitro-9*H*-thioxanthen-9-one in 80 ml of DMF as described in Example 21 gave 2.7 g of product.

1-Chloro-5-methoxy-4-nitro-9*H*-thioxanthen-9-one is prepared from 2-methoxybenzenethiol [L. Gottermann, *Ber. 32;* 1136 (1899)] in the manner of Example 26.

Other 7-(methoxy, phenylmethoxy, or *p*-halo- or *p*-methoxy-substituted phenylmethoxy)-5-nitro-2-(substituted-2*H*[1]benzothiopyrano[4,3,2-*cd*]indazoles are prepared in the manner of Example 1 to 3, 21, 26, and 36.

Example 37

5-Amino-*N,N*-diethyl-7-methoxy-2*H*[1]benzothiopyrano[4,3,2-*cd*]indazole-2-ethanamine

Hydrogenation of *N,N*-diethyl-7-methoxy-5-nitro-2*H*[1]benzothiopyrano[4,3,2-*cd*]indazole-2-ethanamine as described in Example 4 gave the product.

Other 5-amino-7-methoxy-2-(substituted)-2*H*[1]benzothiopyrano[4,3,2-*cd*]indazoles are prepared from the appropriate 7-methoxy-2-(substituted)-5-nitro-2*H*[1]benzothiopyrano[4,3,2-*cd*]indazoles in the manner of Example 4.

Example 38

*N*-[2-[2-(Diethylamino)ethyl]-7-methoxy-2*H*[1]benzothiopyrano[4,3,2-*cd*]indazol-5-yl]-1,2-ethanediamine, hydrobromide salt

Reaction of a solution of 3.5 g (0.0095 mol) of 5-amino-*N,N*-diethyl-7-methoxy-2*H*[1]benzothiopyrano[4,3,2-*cd*]indazole-2-ethanamine and 5.8 g (0.0283 mol) of 2-bromoethylamine, hydrobromide, in 30 ml of EtOH as described in Example 6 gave 1.6 g of product.

Example 39

5-[2-(Aminoethyl)amino]-2-[2-(diethylamino)ethyl]-2*H*[1]benzothiopyrano[4,3,2-*cd*]indazol-7-ol, hydrobromide salt

Reaction of a suspension of 2.3 g (0.0047 mol) of *N*-[2-[2-(diethylamino)ethyl-7-methoxy-2*H*[1]benzothiopyrano[4,3,2-*cd*]indazol-5-yl]-1,2-ethanediamine, hydrobromide salt, and excess boron tribromide in ethylene dichloride as described in Example 25 gave 2.4 g of product.

Other 2,5-(disubstituted)-2*H*[1]benzothiopyrano[4,3,2-*cd*]indazole-7-ols are prepared from the corresponding 7-methoxy precursors in the manner of Example 25.

Example 40

5-Amino-2-[2-(diethylamino)ethyl]-2*H*[1]benzothiopyrano[4,3,2-*cd*]indazol-7-ol

Reaction of 5-amino-*N,N*-diethyl-7-methoxy-2*H*[1]benzothiopyrano[4,3,2-*cd*]indazole-2-ethanamine with 48% HBr as described in Example 32 gave the product.

Other 5-amino-2-(substituted)-2*H*[1]benzothiopyrano[4,3,2-*cd*]indazol-7-ols are prepared from the appropriate 5-amino-7-methoxy-2-(substituted)-2*H*[1]benzothiopyrano[4,3,2-*cd*]indazoles in the manner of Example 32.

Example 41

*N,N*-Diethyl-8-methoxy-5-nitro-2*H*[1]benzothiopyrano[4,3,2-*cd*]indazole-2-ethanamine

Reaction of a mixture of 2.2 g (0.0169 mol) of 2-(diethylaminoethyl)hydrazine, 3.7 g (0.0115 mol) of 1-chloro-6-methoxy-4-nitro-9*H*-thioxanthen-9-one in 100 ml of DMF as described in Example 21 gave 3.4 g of product.

1-chloro-6-methoxy-4-nitro-9*H*-thioxanthen-9-one is prepared from 3-methoxybenzenethiol [L. Szathmary, *Ber. 43*; 2485 1910)] in the manner of Example 26.

Other 8-(methoxy, phenylmethoxy, or *p*-halo- or *p*-methoxy-substituted phenylmethoxy)-5-nitro-2-(substituted)-2*H*[1]benzothiopyrano[4,3,2-*cd*]indazoles are prepared in the manner of Examples 1 to 3, 21, 26 and 41.

Example 42

5-Amino-*N,N*-diethyl-8-methoxy-2*H*[1]benzothiopyrano[4,3,2-*cd*]indazole-2-ethanamine

Hydrogenation of *N,N*-diethyl-8-methoxy-5-nitro-2*H*[1]benzothiopyrano[4,3,2-*cd*]indazole-2-ethanamine as described in Example 4 gave the product.

Other 5-amino-8-methoxy-2-(substituted)-2*H*[1]benzothiopyrano[4,3,2-*cd*]indazoles are prepared from the appropriate 8-methoxy-2-(substituted)-5-nitro-2*H*[1]benzothiopyrano[4,3,2-*cd*]indazoles in the manner of Example 4.

Example 43

*N*-[2-[2-(Diethylamino)ethyl]-8-methoxy-2*H*[1]benzothiopyrano[4,3,2-*cd*]indazol-5-yl]-1,2-ethanediamine, hydrobromide salt

Reaction of a solution of 4.5 g (0.0122 mol) of 5-amino-*N,N*-diethyl-8-methoxy-2*H*[1]benzothiopyrano[4,3,2-*cd*]indazole-2-ethanamine and 7.5 g (0.0366 mol) of 2-bromoethylamine, hydrobromide, in 40 ml of EtOH as described in Example 6 gave 2.4 g of product.

Example 44

5-[2-(Aminoethyl)amino]-2-[2-(diethylamino)ethyl]-2*H*[1]benzothiopyrano[4,3,2-*cd*]indazol-8-ol, hydrobromide salt

Reaction of a suspensionn of 5.0 g (0.0102 mol) of *N*-[2-[2-(diethylamino)ethyl-7-methoxy-2*H*[1]benzothiopyrano[4,3,2-*cd*]indazol-5-yl]-1,2-ethanediamine, hydrobromide salt, and excess boron tribromide in ethylene dichloride as described in Example 25 gave 4.8 g of product.

Other 2,5-(disubstituted)-2*H*[1]benzothiopyrano[4,3,2-*cd*]indazole-8-ols are prepared from the corresponding 8-methoxy precursors in the manner of Example 25.

Example 45

5-Amino-2-[2-(diethylamino)ethyl]-2*H*[1]benzothiopyrano[4,3,2-*cd*]indazol-8-ol

Reaction of 5-amino-*N,N*-diethyl-8-methoxy-2*H*[1]benzothiopyrano[4,3,2-*cd*]indazole-2-ethanamine with 48% HBr as described in Example 32 gave the product.

Other 5-amino-2-(substituted)-2*H*[1]benzothiopyrano[4,3,2-*cd*]indazol-8-ols are prepared from the appropriate 5-amino-8-methoxy-2-(substituted)-2*H*[1]benzothiopyrano[4,3,2-*cd*]indazoles in the manner of Example 32.

Example 46

*N,N*-Diethyl-10-methoxy-5-nitro-2*H*[1]benzothiopyrano[4,3,2-*cd*]indazole-2-ethanamine

Reaction of a mixture of 2.5 g (0.0192 mol) of 2-(diethylaminoethyl)hydrazine, 4.2 g (0.0131 mol) of 1-chloro-8-methoxy-4-nitro-9*H*-thioxanthen-9-one in 110 ml of DMF as described in Example 21 gave 2.7 g of product.

1-Chloro-8-methoxy-4-nitro-9*H*-thioxanthen-9-one is prepared in the manner of Example 26.

Other 10-(methoxy, phenylmethoxy, or *p*-halo- or *p*-methoxy-substituted phenylmethoxy)-5-nitro-2-(substituted)-2*H*[1]benzothiopyrano[4,3,2-*cd*]indazoles are prepared in the manner of Examples 1 to 3, 21, 26, and 46.

Example 47

5-Amino-*N,N*-diethyl-10-methoxy-2*H*[1]benzothiopyrano[4,3,2-*cd*]indazole-2-ethanamine

Hydrogenation of *N,N*-diethyl-10-methoxy-5-nitro-2*H*[1]benzothiopyrano[4,3,2-*cd*]indazole-2-ethanamine as described in Example 4 gave the product.

Other 5-amino-10-methoxy-2-(substituted)-2*H*[1]benzothiopyrano[4,3,2-*cd*]indazoles are prepared from the appropriate 10-methoxy-2-(substituted)-5-nitro-2*H*[1]benzothiopyrano[4,3,2-*cd*]indazoles in the manner of Example 4.

Example 48

*N*-[2-(Diethylamino)ethyl]-10-methoxy-2*H*[1]benzothiopyrano[4,3,2-*cd*]indazol-5-yl]-1,2-ethanediamine, hydrobromide salt

Reaction of a solution of 3.2 g (0.0087 mol) of 5-amino-*N,N*-diethyl-10-methoxy-2*H*[1]benzothiopyrano[4,3,2-*cd*]indazole-2-ethanamine and 5.3 g (0.0259 mol) of 2-bromoethylamine, hydrobromide, in 27 ml of EtOH as described in Example 6 gave 1.3 g of product.

Example 49

5-[2-(Aminoethyl)amino]-2-[2-(diethylamino)ethyl]-2*H*[1]benzothiopyrano[4,3,2-*cd*]indazol-10-ol, hydrobromide salt

Reaction of a suspension of 1.7 g (0.0035 mol) of *N*-[2-[2-(diethylamino)ethyl)-10-methoxy-2*H*[1]benzothiopyrano[4,3,2-*cd*]indazol-5-yl]-1,2-ethanediamine, hydrobromide salt, and excess boron tribromide in ethylene dichloride as described in Example 25 gave 1.9 g of product.

Other 2,5-(disubstituted)-2*H*[1]benzothiopyrano[4,3,2-*cd*]indazole-10-ols are prepared from the corresponding 10-methoxy precursors in the manner of Example 25.

Example 50

5-Amino-2-[2-(diethylamino)ethyl]-2*H*[1]benzothiopyrano[4,3,2-*cd*]indazol-10-ol

Reaction of 5-amino-*N,N*-diethyl-10-methoxy-2*H*[1]benzothiopyrano[4,3,2-*cd*]indazole-2-ethanamine with 48% HBr as described in Example 32 gave the product.

Other 5-amino-2-(substituted)-2*H*[1]benzothiopyrano[4,3,2-*cd*]indazol-10-ols are prepared from the appropriate 5-amino-10-methoxy-2-(substituted)-2*H*[1]benzothiopyrano[4,3,2-*cd*]indazoles in the manner of Example 32.

Example 51

*N,N*-Diethyl-3-methoxy-5-nitro-2*H*[1]benzothiopyrano[4,3,2-*cd*]indazole-2-ethanamine

Reaction of a mixture of 4.5 g (0.0343 mol) of 2-(diethylaminoethyl)hydrazine, 7.5 g (0.0233 mol) of 1-chloro-2-methoxy-4-nitro-9*H*-thioxanthen-9-one in 200 ml of DMF as described in Example 21 gave 7.6 g of product. 1-Chloro-2-methoxy-4-nitro-9*H*-thioxanthen-9-one is prepared from 2-thiobenzoic acid and 2,4-dichloro-5-nitroanisole [C. Bloomfield, A. K. Manglik, R. B. Mootie, K. Schofield, and G. D. Tokin, *J. Chem. Soc. Perkin Trans. II*, 75 (1983)] in the manner of Example 26.

Other 3-(methoxy, phenylmethoxy, or *p*-halo- or *p*-methoxy-substituted phenylmethoxy)-5-nitro-2-(substituted)-2*H*[1]benzothiopyrano[4,3,2-*cd*]indazoles are prepared in the manner of Examples 1 to 3, 21, 26 and 51.

Example 52

5-Amino-*N,N*-diethyl-3-methoxy-2*H*[1]benzothiopyrano[4,3,2-*cd*]indazole-2-ethanamine

Hydrogenation of *N,N*-diethyl-3-methoxy-5-nitro-2*H*[1]benzothiopyrano[4,3,2-*cd*]benzothiopyrano[4,3,2-*cd*]indazole-2-ethanamine as described in Example 4 gave the product.

Other 5-amino-3-methoxy-2-(substituted)-2*H*[1]benzothiopyrano[4,3,2-*cd*]indazoles are prepared from the appropriate 3-methoxy-2-(substituted)-5-nitro-2*H*[1]benzothiopyrano[4,3,2-*cd*]indazoles in the manner of Example 4.

Example 53

*N*-[2-[2-(Diethylamino)ethyl]-3-methoxy-2*H*[1]benzothiopyrano[4,3,2-*cd*]indazol-5-yl]-1,2-ethanediamine, hydrobromide salt

Reaction of a solution of 7.0 g (0.019 mol) of 5-amino-*N,N*-diethyl-3-methoxy-2*H*[1]benzothiopyrano[4,3,2-*cd*]indazole-2-ethanamine and 11.6 g (0.0566 mol) of 2-bromoethylamine, hydrobromide, in 60 ml of EtOH as described in Example 6, gave 3.1 g of product.

Example 54

5-[2-(Aminoethyl)amino]-2-[2-(diethylamino)ethyl]-2*H*[1]benzothiopyrano[4,3,2-*cd*]indazol-3-ol, hydrobromide salt

Reaction of a suspension of 4.2 g (0.0085 mol) of *N*-[2-[2-(diethylamino)ethyl-3-methoxy-2*H*[1]benzothiopyrano[4,3,2-*cd*]indazol-5-yl]-1,2-ethanediamine, hydrobromide salt, and excess boron tribromide in ethylene dichloride as described in Example 25 gave 3.9 g of product.

Other 2,5-(disubstituted)-2*H*[1]benzothiopyrano[4,3,2-*cd*]indazole-3-ols are prepared from the corresponding 3-methoxy precursors in the manner of Example 25.

Example 55

5-Amino-2-[2-(diethylamino)ethyl]2*H*[1]benzothiopyrano[4,3,2-*cd*]indazol-3-ol

Reaction of 5-amino-*N,N*-diethyl-3-methoxy-2*H*[1]benzothiopyrano[4,3,2-*cd*]indazole-2-ethanamine with 48% HBr as described in Example 32 gave the product.

Other 5-amino-2-(substituted)-2*H*[1]benzothiopyrano[4,3,2-*cd*]indazol-3-ols are prepared from the appropriate 5-amino-3-methoxy-2-(substituted)-2*H*[1]benzothiopyrano[4,3,2-*cd*]indazoles in the manner of Example 32.

Example 56

N,N-Diethyl-3,9-dimethoxy-5-nitro-2*H*[1]benzothiopyrano[4,3,2-*cd*]indazole-2-ethanamine

Reaction of a mixture of 1.8 g (0.0137 mol) of 2-(diethylaminoethyl)hydrazine, 3.1 g (0.0093 mol) of 1-chloro-2,7-dimethoxy-4-nitro-9H-thioxanthen-9-one in 80 ml of DMF as described in Example 21 gave 2.4 g of product.

1-Chloro-2,7-dimethoxy-4-nitro-9H-thioxanthen-9-one is prepared from 5-methoxy-2-thiobenzoic acid and 2,4-dichloro-5-nitroanisole in the manner of Example 26.

Other 3,9-di-(methoxy, phenylmethoxy, or *p*-halo- or *p*-methoxy-substituted phenylmethoxy)-5-nitro-2-(substituted)-2*H*[1]benzothiopyrano[4,3,2-*cd*]indazoles are prepared in the manner of Examples 1 to 3, 21, 26 and 56.

Example 57

5-Amino-N,N-diethyl-3,9-dimethoxy-2*H*[1]benzothiopyrano[4,3,2-*cd*]indazole-2-ethanamine

Hydrogenation of N,N-diethyl-3,9-dimethoxy-5-nitro-2*H*[1]benzothiopyrano[4,3,2-*cd*]indazole-2-ethanamine as described in Example 4 gave the product.

Other 5-amino-3,9-dimethoxy-2-(substituted)-2*H*[1]benzothiopyrano[4,3,2-*cd*]indazoles are prepared from the appropriate -3,9-dimethoxy-2-(substituted)-5-nitro-2*H*[1]benzothiopyrano[4,3,2-*cd*]indazoles in the manner of Example 4.

Example 58

*N*-[2-[2-(Diethylamino)ethyl]-3,9-dimethoxy-2*H*[1]benzothiopyrano[4,3,2-*cd*]indazolyl-5-yl]-1,2-ethanediamine, hydrobromide salt

Reaction of a solution of 3.6 g (0.0095 mol) of 5-amino-*N,N*-diethyl-3,9-dimethoxy-2*H*[1]benzothiopyrano[4,3,2-*cd*]indazole-2-ethanamine and 5.8 g (0.0283 mol) of 2-bromoethylamine, hydrobromide, in 30 ml of EtOH as described in Example 6 gave 1.8 g of product.

Example 59

5-[2-(Aminoethyl)amino]-2-[2-(diethylamino)ethyl]-2*H*[1]benzothiopyrano[4,3,2-*cd*]indazol-3,9-diol, hydrobromide salt

Reaction of a suspension of 2.4 g (0.0047 mol) of *N*-[2-[2-(diethylamino)ethyl-3,9-dimethoxy-2*H*[1]benzothiopyrano[4,3,2-*cd*]indazol-5-yl]-1,2-ethanediamine, hydrobromide salt, and excess boron tribromide in ethylene dichloride as described in Example 25 gave 2.2 g of product.

Other 2,5-(disubstituted)-2*H*[1]benzothiopyrano[4,3,2-*cd*]indazole-3,9-diols are prepared from the corresponding 3,9-dimethoxy precursors in the manner of Example 25.

Example 60

5-Amino-2-[2-(diethylamino)ethyl]-2*H*[1]benzothiopyrano[4,3,2-*cd*]indazol-3,9-diol

Reaction of 5-amino-*N,N*-diethyl-3,9-dimethoxy-2*H*[1]benzothiopyrano[4,3,2-*cd*]indazole-2-ethanamine with 48% HBr as described in Example 32 gave the product.

Other 5-amino-2-(substituted)-2*H*[1]benzothiopyrano[4,3,2-*cd*]indazol-3,9-diols are prepared from the appropriate 5-amino-3,9-dimethoxy-2-(substituted)-2*H*[1]benzothiopyrano[4,3,2-*cd*]indazoles in the manner of Example 32.

Example 61

Preparation of Intravenous Formulations

A solution of 14.7 g of 2-[[2-[5-[[2-[(2-hydroxyethyl)amino]-2*H*[1]benzothiopyrano[4,3,2-*cd*]indazole-2-yl]ethyl]amino]ethanol (from Example 18) as the dihydrochloride salt is prepared in 1 l of water for injection at room temperature with stirring. The solution is sterile filtered into 500 5 ml vials, each of which contains 2 ml of solution containing 25 mg of drug as the base, and sealed under nitrogen.

Alternatively, after sterile filtration into vials, the water may be removed by lyophilization, and the vials then sealed aseptically, to provide a powder which is redissolved prior to injection.

**Claims for the Contracting States: BE CE DE FR GB IT LI LU NL SE**

1. Benzothiopyrano[4,3,2-*cd*]indazole compounds having in free base form the structural formula 1:

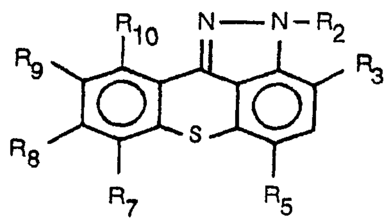

1

and the pharmaceutically acceptable salts thereof; wherein the substituents $R_3$, $R_7$, $R_8$, $R_9$ and $R_{10}$ represent hydrogen, hydroxy or alkoxy of from 1 to 4 carbon atoms; wherein $R_2$ is ANR'R'' wherein A is a straight or branched alkylene chain of from 2 to 5 carbon atoms, optionally substituted with hydroxyl; wherein R' and R'' are hydrogen or straight or branched alkyl of from 1 to 4 carbon atoms, optionally substituted with hydroxyl, R' and R'' taken together also representing

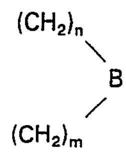

wherein n and m are each 2 or 3 and B is a direct bond or O, S, or NR'''; wherein R''' is hydrogen or straight or branched alkyl of from 1 to 4 carbon atoms, optionally substituted with hydroxyl; and wherein $R_5$ is nitro, $NH_2$,

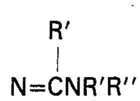

NHR', or NR'$R_2$ or NR'R'''' wherein R'''' is from 1 to 4 carbon acyl, chloracetyl, or

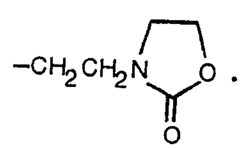

2. Benzothiopyrano[4,3,2-*cd*]indazole compounds according to Claim 1 having in free base form the structural formula 2:

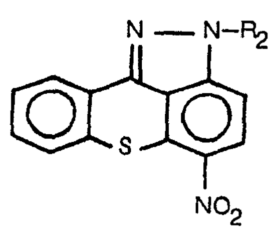

2

and the pharmaceutically acceptable salts thereof, wherein $R_2$ is ANR'R'' wherein A is a straight or branched alkylene chain of from 2 to 5 carbon atoms, optionally substituted with hydroxyl; wherein R' and

R″ are hydrogen or straight or branched alkyl of from 1 to 4 carbon atoms, optionally substituted with hydroxyl, R′ and R″ taken together also representing

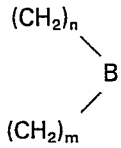

wherein n and m are each 2 or 3 and B is a direct bond or O, S, or NR‴ wherein R‴ is hydrogen or straight or branched alkyl of from 1 to 4 carbon atoms, optionally substituted with hydroxyl.

3. Benzothiopyrano[4,3,2-*cd*]indazole compounds according to Claim 1 having in free base form the structural formula 4a:

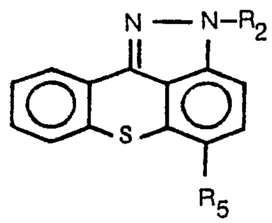

4a

and the pharmaceutically acceptable salts thereof, wherein $R_2$ is ANR′R″ wherein A is a straight or branched alkylene chain of from 2 to 5 carbon atoms, optionally substituted with hydroxyl; wherein R′ and R″ are hydrogen or straight or branched alkyl of from 1 to 4 carbon atoms, optionally substituted with hydroxyl, R′ and R″ taken together also representing

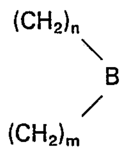

wherein n and m are each 2 or 3 and B is a direct bond or O, S, or NR‴ wherein R‴ is hydrogen or straight or branched alkyl of from 1 to 4 carbon atoms, optionally substituted with hydroxyl, and wherein $R_5$ is nitro, $NH_2$,

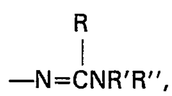

NHR′, NR′$R_2$ or NR′R⁗ wherein R⁗ is from 1 to 4 carbon acyl, chloroacetyl, or

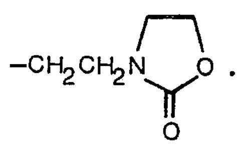

4. A compound according to Claim 3 wherein $R_2$ is diethyl aminoethyl and $R_5$ is $NHCH_2CH_2NHCH_2CH_2OH$.

5. A compound according to Claim 3 wherein $R_2$ is $CH_2CH_2NHCH_2CH_2OH$ and $R_5$ is $NHCH_2CH_2NHCH_2CH_2OH$.

6. A compound according to Claim 3 wherein $R_2$ is diethylaminoethyl and $R_5$ is aminoethylamino.

7. Benzothiopyrano[4,3,2-*cd*]indazole compounds according to Claim 1 having in free base form the structural formula 6:

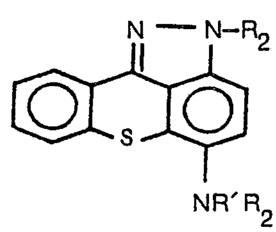

6

and the pharmaceutically acceptable salts thereof, wherein $R_2$ is ANR'R'' wherein A is a straight or branched alkylene chain of from 2 to 5 carbon atoms, optionally substituted with hydroxyl; wherein R' and R'' are hydrogen or straight or branched alkyl of from 1 to 4 carbon atoms, optionally substituted with hydroxyl, R' and R'' taken together also representing

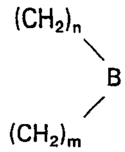

wherein n and m are each 2 or 3 and B is a direct bond or O, S, or NR''' wherein R''' is hydrogen or straight or branched alkyl of from 1 to 4 carbon atoms, optionally substituted with hydroxyl.

8. Compounds according to Claim 1 which are:

2-[[2-[[2-[2-(diethylamino)ethyl]-2*H*[1]benzothiopyrano[4,3,2-*cd*]indazol-5-yl]amino]ethyl]amino]ethanol;

2-[[2-[5-[[2-[(2-hydroxyethyl)amino]ethyl]amino]-2*H*[1]benzothiopyrano[4,3,2-*cd*]indazol-2-yl]-ethyl]amino]ethanol;

N-[2-[2-(diethylamino)ethyl]-2*H*[1]benzothiopyrano[4,3,2-*cd*]indazol-5-yl]-1,2-ethanediamine;

*N,N*-diethyl-5-nitro-2*H*[1]benzothiopyrano[4,3,2-*cd*]indazole-2-ethanamine;

5-nitro-2*H*[1]benzothiopyrano[4,3,2-*cd*]indazole-2-ethanamine;

2-[[2-(5-nitro-2*H*[1]benzothiopyrano[4,3,2-*cd*]indazol-2-yl)ethyl]amino]ethanol;

5-amino-*N,N*-diethyl-2*H*[1]benzothiopyrano[4,3,2-*cd*]indazole-2-ethanamine;

*N,N*-diethyl-9-methoxy-5-nitro-2*H*[1]benzothiopyrano[4,3,2-*cd*]indazole-2-ethanamine;

*N*-[2-[2-(diethylamino)ethyl]-2*H*[1]benzothiopyrano[4,3,2-*cd*]indazol-5-yl]-1,3-propanediamine;

5-amino-*N,N*-dimethyl-2*H*[1]benzothiopyrano[4,3,2-*cd*]indazole-2-propanamine;

*N*-[2-[2-(diethylamino)ethyl]-2*H*[1]benzothiopyrano[4,3,2-*cd*]indazol-5-yl]-*N,N*-dimethylmethanimidamide;

3-[2-[[2-[2-(diethylamino)ethyl]-2*H*[1]benzothiopyrano[4,3,2-*cd*]indazol-5-yl]amino]ethyl]-2-oxazolidinone;

*N,N*-dimethyl-5-nitro-2*H*[1]benzothiopyrano[4,3,2-*cd*]indazole-2-propanamine;

2-[[2-(5-amino-2*H*[1]benzothiopyrano[4,3,2-*cd*]indazol-2-yl)ethyl]amino]ethanol;

2-[[2-[[2-(2-aminoethyl)-2*H*[1]benzothiopyrano[4,3,2-*cd*]indazol-5-yl]amino]ethyl]amino]ethanol;

5-amino-2-[2-(diethylamino)ethyl]-2*H*[1]benzothiopyrano[4,3,2-*cd*]indazol-9-ol;

2-[2-(diethylamino)ethyl]-5-[[2-[(2-hydroxyethyl)amino]ethyl]amino]-2*H*[1]benzothiopyrano[4,3,2-*cd*]indazol-9-ol;

*N*-[2-[3-(dimethylamino)propyl]-2*H*[1]benzothiopyrano[4,3,2-*cd*]indazol-5-yl]-1,2-ethanediamine;

3-[2-[[2-[2-(diethylamino)ethyl]-9-hydroxy-2*H*[1]benzothiopyrano[4,3,2-*cd*]indazol-5-yl]);

2-[2-(diethylamino)ethyl]-5-[[2-[(2-hydroxyethyl]aminoethyl]amino-2*H*[1]benzothiopyrano[4,3,2-*cd*]indazol-9-ol;

*N,N*-diethyl-7-methoxy-5-nitro-2*H*[1]benzothiopyrano[4,3,2-*cd*]indazole-2-ethanamine;

5-amino-*N,N*-diethyl-7-methoxy-2*H*[1]benzothiopyrano[4,3,2-*cd*]indazole-2-ethanamine;

*N*-[2-[2-(diethylamino)ethyl]-7-methoxy-2*H*[1]benzothiopyrano[4,3,2-*cd*]indazol-5-yl]-1,2-ethanediamine, hydrobromide salt;

5-[2-(aminoethyl)amino]-2-[2-diethylamino)ethyl]-2*H*[1]benzothiopyrano[4,3,2-*cd*]indazol-7-ol, hydrobromide salt;

5-amino-2-[2-(diethylamino)ethyl]-2*H*[1]benzothiopyrano[4,3,2-*cd*]indazol-7-ol;

*N,N*-diethyl-8-methoxy-5-nitro-2*H*[1]benzothiopyrano[4,3,2-*cd*]indazole-2-ethanamine;

5-amino-*N,N*-diethyl-8-methoxy-2*H*[1]benzothiopyrano[4,3,2-*cd*]indazole-2-ethanamine;

*N*-[2-[2-(diethylamino)ethyl]-8-methoxy-2*H*[1]benzothiopyrano[4,3,2-*cd*]indazol-5-yl]-1,2-ethanediamine, hydrobromide salt;

5-[2-(aminoethyl)amino]-2-[2-diethylamino)ethyl]-2*H*[1]benzothiopyrano[4,3,2-*cd*]indazol-8-ol, hydrobromide salt;

5-amino-2-[2-(diethylamino)ethyl]-2*H*[1]benzothiopyrano[4,3,2-*cd*]indazol-8-ol;

*N,N*-diethyl-10-methoxy-5-nitro-2*H*[1]benzothiopyrano[4,3,2-*cd*]indazole-2-ethanamine;

5-amino-*N,N*-diethyl-10-methoxy-2*H*[1]benzothiopyrano[4,3,2-*cd*]indazole-2-ethanamine;

*N*-[2-[2-(diethylamino)ethyl]-10-methoxy-2*H*[1]benzothiopyrano[4,3,2-*cd*]indazol-5-yl]-1,2-ethanediamine, hydrobromide salt;

5-[2-(aminoethyl)amino]-2-[2-diethylamino)ethyl]-2*H*[1]benzothiopyrano[4,3,2-*cd*]indazol-10-ol, hydrobromide salt;

5-amino-2-[2-(diethylamino)ethyl]-2*H*[1]benzothiopyrano[4,3,2-*cd*]indazol-10-ol;

*N,N*-diethyl-3-methoxy-5-nitro-2*H*[1]benzothiopyrano[4,3,2-*cd*]indazole-2-ethanamine;

5-amino-*N,N*-diethyl-3-methoxy-2*H*[1]benzothiopyrano[4,3,2-*cd*]indazole-2-ethanamine;

*N*-[2-[2-(diethylamino)ethyl]-3-methoxy-2*H*[1]benzothiopyrano[4,3,2-*cd*]indazol-5-yl]-1,2-ethanediamine, hydrobromide salt;

5-[2-(aminoethyl)amino]-2-[2-diethylamino)ethyl]-2*H*[1]benzothiopyrano[4,3,2-*cd*]indazol-3-ol, hydrobromide salt;

5-amino-2-[2-(diethylamino)ethyl]-2*H*[1]benzothiopyrano[4,3,2-*cd*]indazol-3-ol;

*N,N*-diethyl-3,9-dimethoxy-5-nitro-2*H*[1]benzothiopyrano[4,3,2-*cd*]indazole-2-ethanamine;

5-amino-*N,N*-diethyl-3,9-dimethoxy-2*H*[1]benzothiopyrano[4,3,2-*cd*]indazole-2-ethanamine;

*N*-[2-[2-(diethylamino)ethyl]-3,9-dimethoxy-2*H*[1]benzothiopyrano[4,3,2-*cd*]indazol-5-yl]-1,2-ethanediamine, hydrobromide salt;

5-[2-(aminoethyl)amino]-2-[2-diethylamino)ethyl]-2*H*[1]benzothiopyrano[4,3,2-*cd*]indazol-3,9-diol, hydrobromide salt;

5-amino-2-[2-(diethylamino)ethyl]-2*H*[1]benzothiopyrano[4,3,2-*cd*]indazol-3,9-diol;

2-[[2-[5-[[2-(diethylamino)ethyl]amino]-2*H*[1]benzothiopyrano[4,3,2-*cd*]indazol-2-yl]ethyl]amino]ethanol; and the pharmaceutically acceptable salts of said compounds.

9. Benzothiopyrano[4,3,2-*cd*]indazole compounds according to Claim 1 having in free base form the structural formula 16;

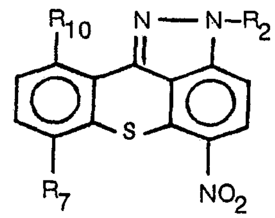

16

and the pharmaceutically acceptable salts thereof; wherein $R_2$ has the meaning according to Claim 1 and $R_7$ and $R_{10}$ are hydrogen, hydroxy or lower, $C_{1-4}$ alkoxy, at least one of $R_7$ and $R_{10}$ being hydroxy or lower, $C_{1-4}$, alkoxy.

10. Benzothiopyrano[4,3,2-*cd*] indazole compounds according to Claim 1 having in free base form the structural formula 17:

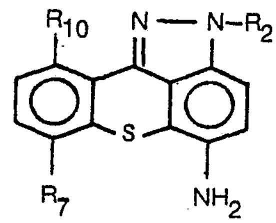

17

and the pharmaceutically acceptable salts thereof; wherein $R_2$ has the meaning according to Claim 1, and $R_7$ and $R_{10}$ are hydrogen, hydroxy or lower, $C_{1-4}$, alkoxy, at least one of $R_7$ and $R_{10}$ being hydroxy or lower, $C_{1-4}$, alkoxy.

11. Benzothiopyrano[4,3,2-*cd*]indazole compounds according to Claim 1 having in free base form the structural formula 18:

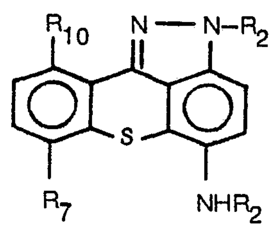

18

and the pharmaceutically acceptable salts thereof; wherein $R_2$ has the meaning according to Claim 1, and $R_7$ and $R_{10}$ are hydrogen, hydroxy or lower, $C_{1-4}$, alkoxy, at least one of $R_7$ and $R_{10}$ being hydroxy or lower $C_{1-4}$, alkoxy.

12. Benzothiopyrano[4,3,2-*cd*]indazole compounds according to Claim 1, having in free base form the structural formula 24:

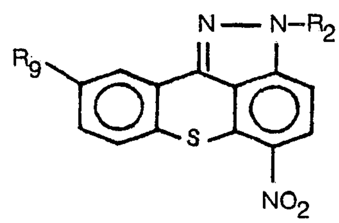

24

and the pharmaceutically acceptable salts thereof; wherein $R_9$ is hydroxy or alkoxy of from 1 to 4 carbon atoms and $R_2$ has the meaning according to Claim 1.

13. Benzothiopyrano[4,3,2-*cd*]indazole compounds according to Claim 1 having in free base form the structural formula 25:

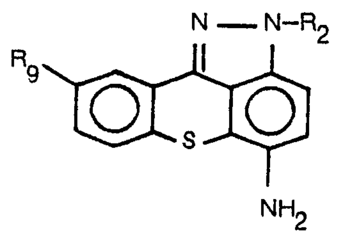

25

and the pharmaceutically acceptable salts thereof; wherein $R_9$ is hydroxy or alkoxy of from 1 to 4 carbon atoms and $R_2$ has the meaning according to Claim 1.

14. Benzothiopyrano[4,3,2-*cd*]indazole compounds according to Claim 1, having in free base form the structural formula 26:

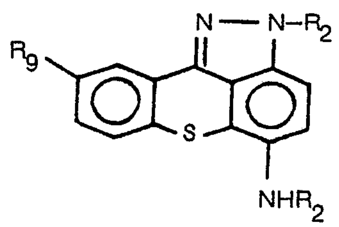

26

and the pharmaceutically acceptable salts thereof; wherein $R_9$ is hydroxy or alkoxy of from 1 to 4 carbon atoms and $R_2$ has the meaning according to Claim 1.

15. A process for preparing benzothiopyrano[4,3,2-*cd*]indazole compounds having the structural formula 1:

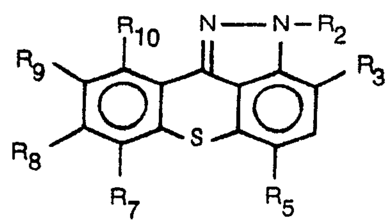

1

according to Claim 1, which comprises: reacting a 1-chloro-4-nitro-9*H*-thioxanthen-9-one and an $R_2$-substituted hydrazine to form the compounds as claimed in Claim 1 wherein $R_5$ is nitro; and, if desired, converting said compounds by reduction to compounds as claimed in Claim 1 wherein $R_5$ is $NH_2$; and if further desired, converting the resulting compounds wherein $R_5$ is $NH_2$ to compounds according to Claim 1 wherein $R_5$ is $NHR_2$ by alkylation with a haloalkylamine having the formula $XR_2$, optionally after covering sensitive groups with protecting groups; or by reaction with an aldehyde or acetal, ketone or ketal and reducing the resulting Schiff base, or by monoacylating with a reactive derivative of an acylating agent of formula $R^aCOOH$ and reducing the acylated product, optionally after covering sensitive groups with protecting groups; and if further desired, converting the compounds wherein $R_5$ is $NHR_2$ to compounds wherein $R_5$ is $NR'R_2$ by further acylation and reduction; and if desired removing the protecting groups by hydrolysis or reduction; and isolating the product in free base or acid addition salt form; wherein X is chloro or bromo and $R_2$, $R_3$, R′, $R_5$, $R_7$, $R_8$, $R_9$, and $R_{10}$ have the meaning according to Claim 1, and $R^a$ is a straight or branched alkyl having from 1 to 3 carbon atoms.

A process for preparing benzothiopyrano[4,3,2-*cd*]indazole compounds according to Claim 1, having in free base form the structural formula:

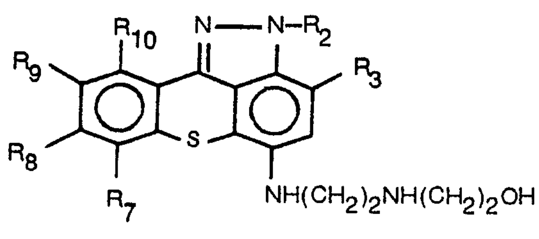

by reacting a compound having the structural formula:

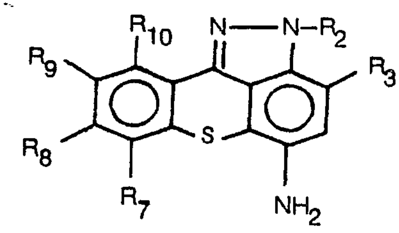

wherein $R_3$, $R_7$, $R_8$, $R_9$, and $R_{10}$ are hydrogen, hydroxyl, lower $C_{1-4}$ alkoxy, benzyloxy, or *p*-halo- or *p*-methoxy substituted benzyloxy, with 3-(β-haloethyl)-2-oxazolidinone to obtain an oxazolidinone having the structural formula:

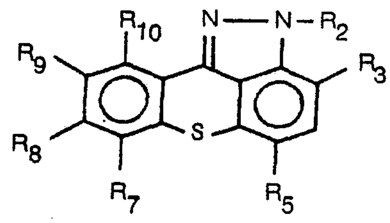

wherein $R_5$ is an N-[2-(2-oxo-3-oxazolidinyl)ethyl]amino group and subjecting the latter compound to alkaline hydrolysis to obtain a compound having the structural formula:

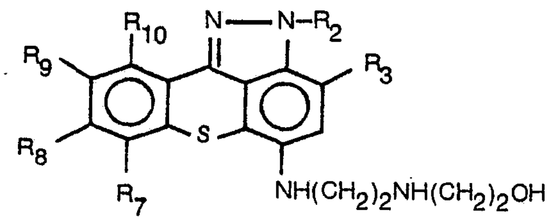

if necessary, removing the benzyloxy groups by hydrogenolysis; and isolating the product in free base or acid addition salt form.

17. A process according to Claim 16 in which one or more of $R_3$, $R_7$, $R_8$, $R_9$ and $R_{10}$ are benzyloxy or alkoxy wherein the benzyloxy and alkoxy groups are removed by hydrolysis or treatment with boron tribromide.

18. A pharmaceutical composition comprising a compound having the structural formula 1 according to Claim 1 in combination with a pharmaceutically acceptable carrier.

19. A pharmaceutical composition comprising a compound having the structural formula 2 according to Claim 2, in combination with a pharmaceutically acceptable carrier.

20. A pharmaceutical composition comprising a compound having the structural formula 4a according to Claim 3 in combination with a pharmaceutically acceptable carrier.

21. A pharmaceutical composition comprising a compound having the structural formula 6 according to Claim 7, in combination with a pharmaceutically acceptable carrier.

**Claims for the Contracting State: AT**

1. A process for preparing benzothiopyrano [4,3,2-*cd*]indazole compounds having the structural formula 1:

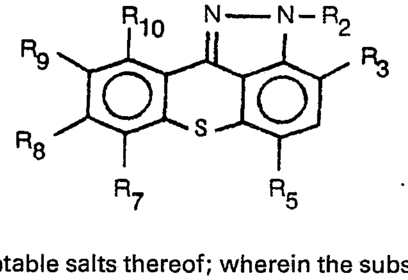

1

and the pharmaceutically acceptable salts thereof; wherein the substituents $R_3$, $R_7$, $R_8$, $R_9$ and $R_{10}$ represent hydrogen, hydroxy or alkoxy of from 1 to 4 carbon atoms; wherein $R_2$ is ANR'R'' wherein A is a straight or branched alkylene chain of from 2 to 5 carbon atoms, optionally substituted with hydroxyl; wherein R' and R'' are hydrogen or straight or branched alkyl of from 1 to 4 carbon atoms, optionally substituted with hydroxyl, R' and R'' taken together also representing

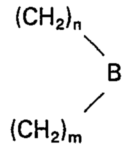

wherein n and m are each 2 or 3 and B is a direct bond or O, S, or NR'''; wherein R''' is hydrogen or straight or branched alkyl of from 1 to 4 carbon atoms, optionally substituted with hydroxyl; and wherein $R_5$ is nitro, $NH_2$,

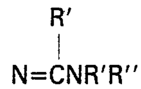

NHR', or $NR'R_2$ or NR'R'''' wherein R'''' is from 1 to 4 carbon acyl, chloracetyl, or

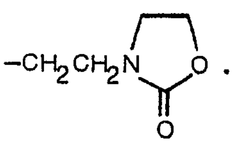

which process comprises:

reacting a 1-chloro-4-nitro-9*H*-thioxanthen-9-one and an $R_2$-substituted hydrazine, to form the compounds wherein $R_5$ is nitro; and, if desired,

converting said compounds by reduction to the compounds wherein $R_5$ is $NH_2$; and, if further desired,

converting the resulting compounds wherein $R_5$ is $NH_2$ to compounds wherein $R_5$ is $NHR_2$ by alkylation with a haloalkylamine having the formula $XR_2$, optionally after covering sensitive groups with protecting groups; or by reaction with an aldehyde, acetal, ketone or ketal and reducing the resulting Schiff base; or by monoacylating with a reactive derivative of an acylating agent of formula RaCOOH and reducing the acylated product, optionally after covering sensitive groups with protecting groups; and, if further desired,

converting the compounds wherein $R_5$ is $NHR_2$ to compounds wherein $R_5$ is $NR'R_2$ by further acylation and reduction; and, if desired,

removing the protecting groups by hydrolysis or reduction; and

isolating the product in free base or acid addition salt form;

wherein X is chloro or bromo and $R^a$ is a straight or branched alkyl of from 1 to 3 carbon atoms.

2. A process for preparing benzothiopyrano [4,3,2-*cd*]indazole compounds having in free base form the structural formula:

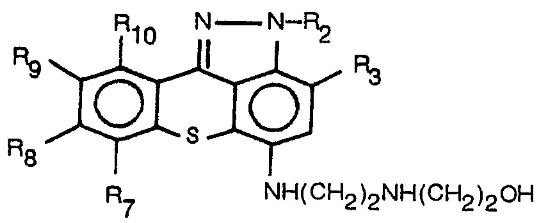

and the pharmaceutically acceptable salts thereof, wherein $R_2$, $R_3$, $R_7$, $R_8$, $R_9$ and $R_{10}$ are as defined in Claim 1, which process comprises:

reacting a compound having the structural formula:

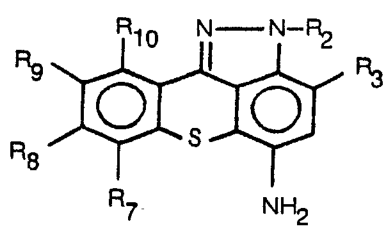

3

wherein $R_3$, $R_7$, $R_8$, $R_9$ and $R_{10}$ are hydrogen, hydroxyl, lower alkoxy, benzyloxy, or *p*-halo- or *p*-methoxy substituted benzyloxy, with 3-(β-haloethyl)-2-oxazolidinone to obtain an oxazolidinone having the structural formula:

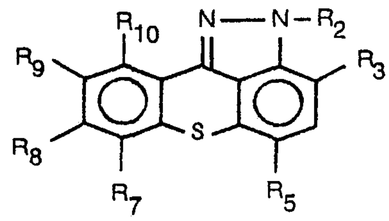

1

wherein $R_5$ is an N-[2-(2-oxo-3-oxazolidinyl)ethyl]amino group; and

subjecting the latter compound to alkaline hydrolysis to obtain a compound having the structural formula:

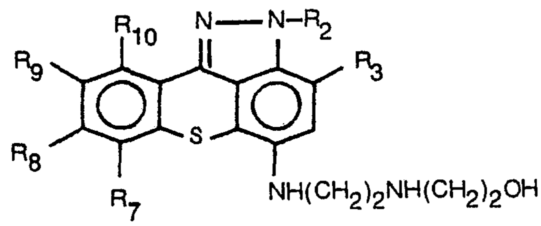

and, if necessary,

removing the benzyloxy groups by hydrogenolysis; and

isolating the product in free base or acid addition salt form.

3. A process according to Claim 2, in which one or more of $R_3$, $R_7$, $R_8$, $R_9$ and $R_{10}$ are benzyloxy or alkoxy wherein the benzyloxy and alkoxy groups are removed by hydrolysis or by treatment with boron tribromide.

4. A process according to Claim 1 for preparing benzothiopyrano[4,3,2-*cd*]indazole compounds, having in free base form the structural formula 2:

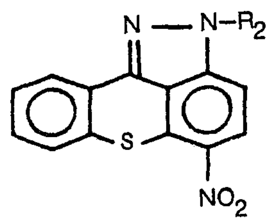

2

and the pharmaceutically acceptable salt thereof, wherein $R_2$ is ANR′R″ wherein A is a straight or branched alkylene chain of from 2 to 5 carbon atoms, optionally substituted with hydroxyl; wherein R′ and R″ are hydrogen or straight or branched alkyl of from 1 to 4 carbon atoms, optionally substituted with hydroxyl, R′ and R″ taken together also representing

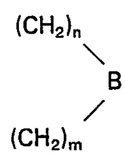

wherein n and m are each 2 or 3 and B is a direct bond or O, S, or NR‴; wherein R‴ is hydrogen or straight or branched alkyl of from 1 to 4 carbon atoms, optionally substituted with hydroxyl.

5. A process according to Claim 1, 2 or 3, for preparing benzothiopyrano[4,3,2-*cd*]indazole compounds having in free base form the structural formula 4a:

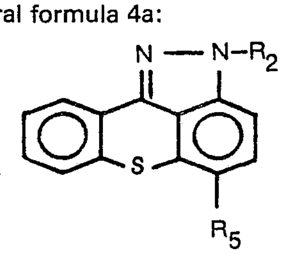

4a

and the pharmaceutically acceptable salts thereof, wherein the substituents $R_3$, $R_7$, $R_8$, $R_9$ and $R_{10}$ represent hydrogen, hydroxy or alkoxy of from 1 to 4 carbon atoms; wherein $R_2$ is ANR′R″ wherein A is a straight or branched alkylene chain of from 2 to 5 carbon atoms, optionally substituted with hydroxyl; wherein R′ and

R'' are hydrogen or straight or branched alkyl of from 1 to 4 carbon atoms, optionally substituted with hydroxyl, R' and R'' taken together also representing

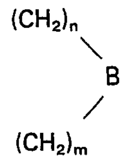

wherein n and m are each 2 or 3 and B is a direct bond or O, S, or NR'''; wherein R''' is hydrogen or straight or branched alkyl of from 1 to 4 carbon atoms, optionally substituted with hydroxyl, and wherein $R_5$ is nitro, $NH_2$,

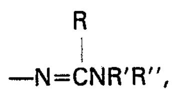

NHR', $NR'R_2$ or NR'R'''' wherein R'''' is from 1 to 4 carbon acyl, chloroacetyl, or

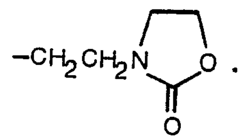

6. A process according to Claim 1, 2, 3 or 5, wherein $R_2$ is diethyl aminoethyl and $R_5$ is $NHCH_2CH_2NHCH_2CH_2OH$.

7. A process according to Claim 1, 2, 3 or 5, wherein $R_2$ is $CH_2CH_2NHCH_2CH_2OH$ and $R_5$ is $NHCH_2CH_2NHCH_2CH_2OH$.

8. A process according to Claim 1 or 5, wherein $R_2$ is diethylaminoethyl and $R_5$ is aminoethylamino.

9. A process according to Claim 1, 2, 3 or 5, for preparing benzothiopyrano[4,3,2-*cd*]indazole having in free base form the structural formula 6:

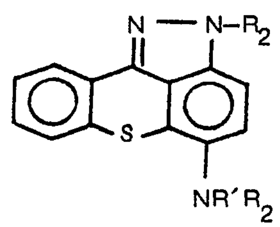

6

and the pharmaceutically acceptable salts thereof wherein $R_2$ is ANR'R'' wherein A is a straight or branched alkylene chain of from 2 to 5 carbon atoms, optionally substituted with hydroxyl; wherein R' and R'' are hydrogen or straight or branched alkyl of from 1 to 4 carbon atoms, optionally substituted with hydroxyl, R' and R'' taken together also representing

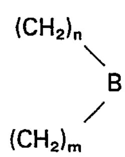

wherein n and m are each 2 or 3 and B is a direct bond or O, S, or NR'''; wherein R''' is hydrogen or straight or branched alkyl of from 1 to 4 carbon atoms, optionally substituted with hydroxyl.

10. A process according to Claim 1, 2, 3 or 5 for preparing:

2-[[2-[[2-[2-(diethylamino)ethyl]-2*H*[1]benzothiopyrano[4,3,2-*cd*]indazol-5-yl]amino]ethyl]amino]ethanol;

2-[[2-[5-[[2-[(2-hydroxyethyl)amino]ethyl]amino]-2*H*[1]benzothiopyrano[4,3,2-*cd*]indazol-2-yl]-ethyl]amino]ethanol;

N-[2-[2-(diethylamino)ethyl]-2*H*[1]benzothiopyrano[4,3,2-*cd*]indazol-5-yl]-1,2-ethanediamine;

*N,N*-diethyl-5-nitro-2*H*[1]benzothiopyrano[4,3,2-*cd*]indazole-2-ethanamine;

5-nitro-2*H*[1]benzothiopyrano[4,3,2-*cd*]indazole-2-ethanamine;

2-[[2-(5-nitro-2*H*[1]benzothiopyrano[4,3,2-*cd*]indazol-2-yl)ethyl]amino]ethanol;

5-amino-*N,N*-diethyl-2*H*[1]benzothiopyrano[4,3,2-*cd*]indazole-2-ethanamine;

*N,N*-diethyl-9-methoxy-5-nitro-2*H*[1]benzothiopyrano[4,3,2-*cd*]indazole-2-ethanamine;

*N*-[2-[2-(diethylamino)ethyl]-2*H*[1]benzothiopyrano[4,3,2-*cd*]indazol-5-yl]-1,3-propanediamine;
5-amino-*N,N*-dimethyl-2*H*[1]benzothiopyrano[4,3,2-*cd*]indazole-2-propanamine;
*N*-[2-[2-(diethylamino)ethyl]-2*H*[1]benzothiopyrano[4,3,2-*cd*]indazol-5-yl]-*N,N*-dimethylmethanimidamide;
3-[2-[[2-[2-(diethylamino)ethyl]-2*H*[1]benzothiopyrano[4,3,2-*cd*]indazol-5-yl]amino]ethyl]-2-oxazolidinone;
*N,N*-dimethyl-5-nitro-2*H*[1]benzothiopyrano[4,3,2-*cd*]indazole-2-propanamine;
2-[[2-(5-amino-2*H*[1]benzothiopyrano[4,3,2-*cd*]indazol-2-yl)ethyl]amino]ethanol;
2-[[2-[[2-(2-aminoethyl)-2*H*[1]benzothiopyrano[4,3,2-*cd*]indazol-5-yl]amino]ethyl]amino]ethanol;
5-amino-2-[2-(diethylamino)ethyl]-2*H*[1]benzothiopyrano[4,3,2-*cd*]indazol-9-ol;
2-[2-(diethylamino)ethyl]-5-[[2-[(2-hydroxyethyl)amino]ethyl]amino]-2*H*[1]benzothiopyrano[4,3,2-*cd*]indazol-9-ol;
*N*-[2-[3-(dimethylamino)propyl]-2*H*[1]benzothiopyrano[4,3,2-*cd*]indazol-5-yl]-1,2-ethanediamine;
3-[2-[[2-[2-(diethylamino)ethyl]-9-hydroxy-2*H*[1]benzothiopyrano[4,3,2-*cd*]indazol-5-yl);
2-[2-(diethylamino)ethyl]-5-[[2-[(2-hydroxyethyl]aminoethyl]amino-2*H*[1]benzothiopyrano[4,3,2-*cd*]indazol-9-ol;
*N,N*-diethyl-7-methoxy-5-nitro-2*H*[1]benzothiopyrano[4,3,2-*cd*]indazole-2-ethanamine;
5-amino-*N,N*-diethyl-7-methoxy-2*H*[1]benzothiopyrano[4,3,2-*cd*]indazole-2-ethanamine;
*N*-[2-[2-(diethylamino)ethyl]-7-methoxy-2*H*[1]benzothiopyrano[4,3,2-*cd*]indazol-5-yl]-1,2-ethanediamine, hydrobromide salt;
5-[2-(aminoethyl)amino]-2-[2-diethylamino)ethyl]-2*H*[1]benzothiopyrano[4,3,2-*cd*]indazol-7-ol, hydrobromide salt;
5-amino-2-[2-(diethylamino)ethyl]-2*H*[1]benzothiopyrano[4,3,2-*cd*]indazol-7-ol;
*N,N*-diethyl-8-methoxy-5-nitro-2*H*[1]benzothiopyrano[4,3,2-*cd*]indazole-2-ethanamine;
5-amino-*N,N*-diethyl-8-methoxy-2*H*[1]benzothiopyrano[4,3,2-*cd*]indazole-2-ethanamine;
*N*-[2-[2-(diethylamino)ethyl]-8-methoxy-2*H*[1]benzothiopyrano[4,3,2-*cd*]indazol-5-yl]-1,2-ethanediamine, hydrobromide salt;
5-[2-(aminoethyl)amino]-2-[2-diethylamino)ethyl]-2*H*[1]benzothiopyrano[4,3,2-*cd*]indazol-8-ol, hydrobromide salt;
5-amino-2-[2-(diethylamino)ethyl]-2*H*[1]benzothiopyrano[4,3,2-*cd*]indazol-8-ol;
*N,N*-diethyl-10-methoxy-5-nitro-2*H*[1]benzothiopyrano[4,3,2-*cd*]indazole-2-ethanamine;
5-amino-*N,N*-diethyl-10-methoxy-2*H*[1]benzothiopyrano[4,3,2-*cd*]indazole-2-ethanamine;
*N*-[2-[2-(diethylamino)ethyl]-10-methoxy-2*H*[1]benzothiopyrano[4,3,2-*cd*]indazol-5-yl]-1,2-ethanediamine, hydrobromide salt;
5-[2-(aminoethyl)amino]-2-[2-diethylamino)ethyl]-2*H*[1]benzothiopyrano[4,3,2-*cd*]indazol-10-ol, hydrobromide salt;
5-amino-2-[2-(diethylamino)ethyl]-2*H*[1]benzothiopyrano[4,3,2-*cd*]indazol-10-ol;
*N,N*-diethyl-3-methoxy-5-nitro-2*H*[1]benzothiopyrano[4,3,2-*cd*]indazole-2-ethanamine;
5-amino-*N,N*-diethyl-3-methoxy-2*H*[1]benzothiopyrano[4,3,2-*cd*]indazole-2-ethanamine;
*N*-[2-[2-(diethylamino)ethyl]-3-methoxy-2*H*[1]benzothiopyrano[4,3,2-*cd*]indazol-5-yl]-1,2-ethanediamine, hydrobromide salt;
5-[2-(aminoethyl)amino]-2-[2-diethylamino)ethyl]-2*H*[1]benzothiopyrano[4,3,2-*cd*]indazol-3-ol, hydrobromide salt;
5-amino-2-[2-(diethylamino)ethyl]-2*H*[1]benzothiopyrano[4,3,2-*cd*]indazol-3-ol;
*N,N*-diethyl-3,9-dimethoxy-5-nitro-2*H*[1]benzothiopyrano[4,3,2-*cd*]indazole-2-ethanamine;
5-amino-*N,N*-diethyl-3,9-dimethoxy-2*H*[1]benzothiopyrano[4,3,2-*cd*]indazole-2-ethanamine;
*N*-[2-[2-(diethylamino)ethyl]-3,9-dimethoxy-2*H*[1]benzothiopyrano[4,3,2-*cd*]indazol-5-yl]-1,2-ethanediamine, hydrobromide salt;
5-[2-(aminoethyl)amino]-2-[2-diethylamino)ethyl]-2*H*[1]benzothiopyrano[4,3,2-*cd*]indazol-3,9-diol, hydrobromide salt;
5-amino-2-[2-(diethylamino)ethyl]-2*H*[1]benzthiopyrano[4,3,2-*cd*]indazol-3,9-diol;
2-[[2-[5-[[2-(diethylamino)ethyl]amino]-2*H*[1]benzothiopyrano[4,3,2-*cd*]indazol-2-yl]ethyl]amino]ethanol; and the pharmaceutically acceptable salts of said compounds.

11. A process according to Claim 1 for preparing benzothiopyrano[4,3,2-*cd*]indazole compounds having in free base form the structural formula 16:

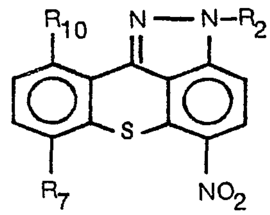

16

and the pharmaceutically acceptable salts thereof; wherein $R_2$ has the meaning according to Claim 1 and $R_7$ and $R_{10}$ are hydrogen, hydroxy or lower, $C_{1-4}$, alkoxy, at least one of $R_7$ and $R_{10}$ being hydroxy or lower, $C_{1-4}$, alkoxy.

12. A process according to Claim 1 for preparing benzothiopyrano[4,3,2-*cd*]indazole compounds having in free base form the structural formula 17:

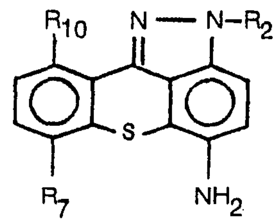

17

and the pharmaceutically acceptable salts thereof; wherein $R_2$ has the meaning according to Claim 1, and $R_7$ and $R_{10}$ are hydrogen, hydroxy or lower, $C_{1-4}$, alkoxy, at least one of $R_7$ and $R_{10}$ being hydroxy or lower, $C_{1-4}$, alkoxy.

13. A process according to Claim 1 or 2, for preparing benzothiopyrano[4,3,2-*cd*]indazole compounds having in free base form the structural formula 18:

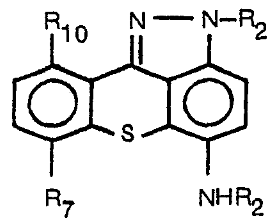

18

and the pharmaceutically acceptable salts thereof; wherein $R_2$ has the meaning according to Claim 1 and $R_7$ and $R_{10}$ are hydrogen, hydroxy or lower, $C_{1-4}$, alkoxy, at least one of $R_7$ and $R_{10}$ being hydroxy or lower, $C_{1-4}$, alkoxy.

14. A process according to Claim 1 for preparing benzothiopyrano[4,3,2-*cd*]indazole compounds having in free base form the structural formula 24:

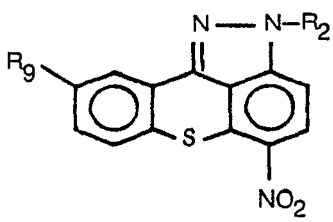

24

and the pharmaceutically acceptable salts thereof; wherein $R_9$ is hydroxy or alkoxy of from 1 to 4 carbon atoms and $R_2$ has the meaning according to Claim 1.

15. A process according to Claim 1 for preparing benzothiopyrano[4,3,2-*cd*]indazole compounds having in free base form the structural formula 25:

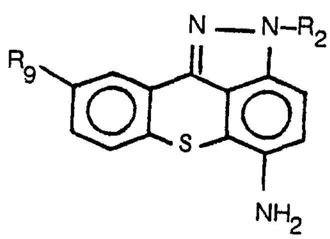

25

and the pharmaceutically acceptable salts thereof; wherein $R_9$ is hydroxy or alkoxy of from 1 to 4 carbon atoms and $R_2$ has the meaning according to Claim 1.

16. A process according to Claim 1 or 2, for preparing benzothiopyrano[4,3,2-*cd*]indazole compounds having in free base form the structural formula 26:

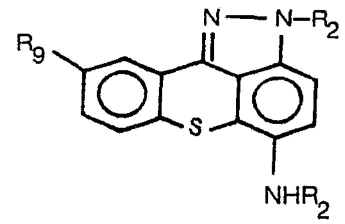

26

and the pharmaceutically acceptable salts thereof; wherein $R_9$ is hydroxy or alkoxy of from 1 to 4 carbon atoms and $R_2$ has the meaning according to Claim 1.

17. A process for preparing a pharmaceutical composition which process comprises combining a compound having the structural formula 1 according to Claim 1 with a pharmaceutically acceptable carrier.

18. A process for preparing a pharmaceutical composition which process comprises combining a compound having the structural formula 2 according to Claim 4, with a pharmaceutically acceptable carrier.

19. A process for preparing a pharmaceutical composition which process comprises combining a compound having the structural formula 4a according to Claim 5 with a pharmaceutically acceptable carrier.

20. A process for preparing a pharmaceutical composition which process comprises combining a compound having the structural formula 6 according to Claim 9, with a pharmaceutically acceptable carrier.

**Patentansprüche für die Vertragsstaaten: BE CH DE FR GB IT LI LU NL SE**

1. Benzothiopyrano[4,3,2-*cd*]indazolverbindungen, die in Form der freien Base die Sturkturformel 1:

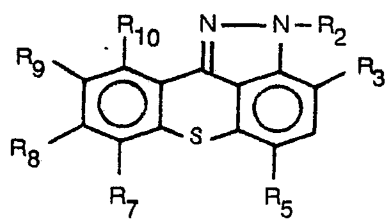

1

haben, und deren pharmazeutisch akzeptable Salze; worin die Substituenten $R_3$, $R_7$, $R_8$, $R_9$ und $R_{10}$ Wasserstoff, Hydroxy oder Alkoxy mit 1 bis 4 Kohlenstoffatomen bedeuten; worin $R_2$ für ANR'R'' steht, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

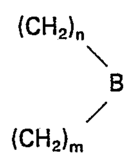

bedeuten, worin n und m jeweils 2 oder 3 bedeuten und B eine Direktbindung oder O, S oder NR''' bedeutet; worin R''' Wasserstoff oder gerades oder verzweigtes Alkyl mit 1 bis 4 Kohlenstoffatomen, gegebenenfalls durch Hydroxyl substituiert, bedeutet; und worin $R_5$ für Nitro, $NH_2$,

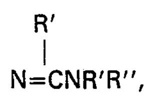

NHR' oder $NR'R_2$ oder NR'R'''' steht, worin R'''' für 1 bis 4 Kohlenstoff-Acyl, Chloracetyl oder

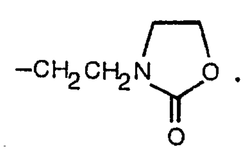

steht.

2. Benzothiopyrano[4,3,2-*cd*]indazolverbindungen nach Anspruch 1, die in Form der freien Base die Strukturformel 2

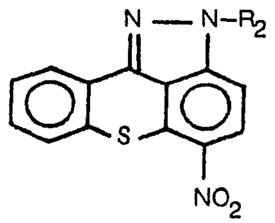

2

haben, und deren pharmazeutisch akzeptable Salze, worin $R_2$ ANR'R'' bedeutet, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

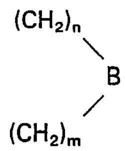

bedeuten, worin n und m jeweils 2 oder 3 bedeuten und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet.

3. Benzothiopyrano[4,3,2-*cd*]indazolverbindungen nach Anspruch 1, die in Form der freien Base die Strukturformel 4a

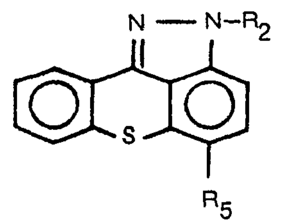

4a

haben, und deren pharmazeutisch akzeptable Salze, worin $R_2$ ANR'R'' bedeutet, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, R' und R'' zusammengenomen auch

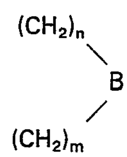

worin n und m jeweils 2 oder 3 bedeuten und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet, und worin $R_5$ Nitro, $NH_2$,

R
|
—N=CNR'R'',

NHR', $NR'R_2$oder NR'R'''' bedeutet, worin R'''' für 1 bis 4 Kohlenstoff-Acyl, Chloracetyl oder

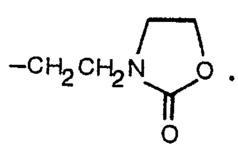

steht.

4. Verbindung nach Anspruch 3, worin $R_2$ Diäthylaminoäthyl bedeutet und $R_5$ für $NHCH_2CH_2NHCH_2CH_2OH$ steht.

5. Verbindung nach Anspruch 3, worin $R_2$ für $CH_2CH_2NHCH_2CH_2OH$ und $R_5$ für $NHCH_2CH_2NHCH_2CH_2OH$ stehen.

6. Verbindung nach Anspruch 3, worin $R_2$ Diäthylaminoäthyl bedeutet und $R_5$ für Aminoäthylamino steht.

7. Benzothiopyrano[4,3,2-*cd*]indazolverbindungen nach Anspruch 1, die in Form der freien Base die Strukturformel 6

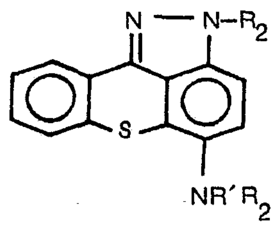

6

haben, und deren pharmazeutisch akzeptable Salze, worin $R_2$ ANR'R'' bedeutet, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

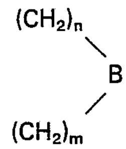

bedeuten, worin n und m jeweils 2 oder 3 bedeuten und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet.

8. Verbindungen nach Anspruch 1, die

2-[[2-[[2-[2-(Diäthylamino)-äthyl]-2*H*[1]benzothiopyrano[4,3,2-*cd*]indazol-5-yl]-amino]-äthyl]-amino]-äthanol;

2-[[2-[5-[[2-[(2-Hydroxyäthyl)-amino]-äthyl]-amino]-2*H*[1]benzothiopyrano[4,3,2-*cd*]indazol-2-yl]-äthyl]-amino]-äthanol;

N-[2-[2-(Diäthylamino)-äthyl]-2H[1]benzothiopyrano[4,3,2-*cd*]indazol-5-yl]-1,2-äthandiamin;

*N,N*-Diäthyl-5-nitro-2H[1]benzothiopyrano[4,3,2-*cd*]indazol-2-äthanamin;

5-Nitro-2H[1]benzothiopyrano[4,3,2-*cd*]indazol-2-äthanamin;

2-[[2-(5-Nitro-2H[1]benzothiopyrano[4,3,2-*cd*]indazol-2-yl)-äthyl]-amino]-äthanol;

5-Amino-*N,N*-diäthyl-2*H*[1]benzothiopyrano[4,3,2-*cd*]indazol-2-äthanamin;

*N,N*-Diäthyl-9-methoxy-5-nitro-2*H*[1]benzothiopyrano[4,3,2-*cd*]indazol-2-äthanamin;

*N*-[2-[2-(Diäthylamino)-äthyl]-2*H*[1]benzothiopyrano[4,3,2-*cd*]indazol-5-yl]-1,3,-propandiamin;

5-Amino-*N,N*-dimethyl-2*H*[1]benzothiopyrano[4,3,2-*cd*]indazol-2-propanamin;

*N*'-[2-[2-(Diäthylamino)-äthyl]-2*H*[1]benzothiopyrano[4,3,2-*cd*]indazol-5-yl]-*N,N*-dimethylmethanimidamid;

3-[2-[[2-[2-(Diäthylamino)-äthyl]-2*H*[1]benzothiopyrano[4,3,2-*cd*]indazol-5-yl]-amino]-äthyl]-2-oxazolidinon;

*N,N*-Dimethyl-5-nitro-2*H*[1]benzothiopyrano[4,3,2-*cd*]indazol-2-propanamin;

2-[[2-(5-Amino-2*H*[1]benzothiopyrano[4,3,2-*cd*]indazol-2-yl)-äthyl]-amino]-äthanol;

2-[[2-[[2-(2-Aminoäthyl)-2*H*[1]benzothiopyrano[4,3,2-*cd*]indazol-5-yl]-amino]-äthyl]-amino]-äthanol;

5-Amino-2-[2-(diäthylamino)-äthyl]-2*H*[1]benzothiopyrano[4,3,2-*cd*]indazol-9-ol;

2-[2-(Diäthylamino)-äthyl]-5-[[2-[(2-hydroxyäthyl)-amino]-äthyl]-amino]-2*H*[1]benzothiopyrano[4,3,2-*cd*]indazol-9-ol;

*N*-[2-[3-(Dimethylamino)-propyl]-2*H*[1]benzothiopyrano[4,3,2-*cd*]indazol-5-yl]-1,2-äthandiamin;

3-[2-[[2-[2-(Diäthylamino)-äthyl]-9-hydroxy-2*H*[1]benzothiopyrano[4,3,2-*cd*]indazol-5-yl];

2-[2-(Diäthylamino)-äthyl]-5-[[2-[(2-hydroxyäthyl)-aminoäthyl]-amino-2H[1]benzothiopyrano[4,3,2-*cd*]indazol-9-ol;

*N,N*-Diäthyl-7-methoxy-5-nitro-2H[1]benzothiopyrano[4,3,2-*cd*]indazol-2-äthanamin;

5-Amino-*N,N*-diäthyl-7-methoxy-2*H*[1]benzothiopyrano[4,3,2-*cd*]indazol-2-äthanamin;

*N*-[2-[2-(Diäthylamino)-äthyl]-7-methoxy-2*H*[1]benzothiopyrano[4,3,2-*cd*]indazol-5-yl]-1,2-äthandiamin-Hydrobromidsalz;

5-[2-(Aminoäthyl)-amino]-2-[2-(diäthylamino)-äthyl]-2*H*[1]benzothiopyrano[4,3,2-*cd*]indazol-7-ol-Hydrobromidsalz;

5-Amino-2-[2-(diäthylamino)-äthyl]-2*H*[1]benzothiopyrano[4,3,2-*cd*]indazol-7-ol;

*N,N*-Diäthyl-8-methoxy-5-nitro-2*H*[1]benzothiopyrano[4,3,2-*cd*]indazol-2-äthanamin;

5-Amino-*N,N*-diäthyl-8-methoxy-2*H*[1]benzothiopyrano[4,3,2-*cd*]indazol-2-äthanamin;

*N*-[2-[2-(Diäthylamino)-äthyl]-8-methoxy-2*H*[1]benzothiopyrano[4,3,2-*cd*]indazol-5-yl]-1,2-äthandiamin-Hydrobromidsalz;

5-[2-(Aminoäthyl)-amino]-2-[2-(diäthylamino)-äthyl]-2*H*[1]benzothiopyrano[4,3,2-*cd*]indazol-8-ol-Hyddrobromidsalz;

5-Amino-2-[2-(diäthylamino)-äthyl]-2*H*[1]benzothiopyrano[4,3,2-*cd*]indazol-8-ol;
*N,N*-Diäthyl-10-methoxy-5-nitro-2*H*[1]benzothiopyrano[4,3,2-*cd*]indazol-2-äthanamin;
5-Amino-*N,N*-diäthyl-10-methoxy-2*H*[1]benzothiopyrano[4,3,2-*cd*]indazol-2-äthanamin;
*N*-[2-[2-(Diäthylamino)-äthyl]-10-methoxy-2*H*[1]benzothiopyrano[4,3,2-*cd*]indazol-5-yl]-1,2-äthandiamin-Hydrobromidsalz;
5-[2-(Aminoäthyl)-amino]-2-[2-(diäthylamino)-äthyl]-2*H*[1]benzothiopyrano[4,3,2-*cd*]indazol-10-ol-Hydrobromidsalz;
5-Amino-2-[2-(diäthylamino)-äthyl]-2*H*[1]benzothiopyrano[4,3,2-*cd*]indazol-10-ol;
*N,N*-Diäthyl-3-methoxy-5-nitro-2*H*[1]benzothiopyrano[4,3,2-*cd*]indazol-2-äthanamin;
5-Amino-*N,N*-diäthyl-3-methoxy-2*H*[1]benzothiopyrano[4,3,2-*cd*]indazol-2-äthanamin;
N-[2-[2-(Diäthylamino)-äthyl]-3-methoxy-2*H*[1]benzothiopyrano[4,3,2-*cd*]indazol-5-yl]-1,2-äthandiamin-Hydrobromidsalz;
5-[2-(Aminoäthyl)-amino]-2-[2-(diäthylamino)-äthyl]-2*H*[1]benzothiopyrano[4,3,2-*cd*]indazol-3-ol-Hydrobromidsalz;
5-Amino-2-[2-(diäthylamino)-äthyl]-2*H*[1]benzothiopyrano[4,3,2-*cd*]indazol-3-ol;
N,N-Diäthyl-3,9-dimethoxy-5-nitro-2*H*[1]benzothiopyrano[4,3,2-*cd*]indazol-2-äthanamin;
5-Amino-*N,N*-diäthyl-3,9-dimethoxy-2*H*[1]benzothiopyrano[4,3,2-*cd*]indazol-2-äthanamin;
*N*-[2-[2-(Diäthylamino)-äthyl]-3,9-dimethoxy-2*H*[1]benzothiopyrano[4,3,2-*cd*]indazol-5-yl]-1,2-äthandiamin-Hydrobromidsalz;
5-[2-(Aminoäthyl)-amino]-2-[2-(diäthylamino)-äthyl]-2*H*[1]benzothiopyrano[4,3,2-*cd*]indazol-3,9-diol-Hydrobromidsalz;
5-Amino-2-[2-(diäthylamino)-äthyl]-2H[1]benzothiopyrano[4,3,2-*cd*]indazol-3,9-diol;
2-[[2-[5-[[2-(Diäthylamino)-äthyl]-amino-2*H*[1]benzothiopyrano[4,3,2-*cd*]indazol-2-yl]-äthyl]-amino]-äthanol sind, und die pharmazeutisch akzeptablen Salze dieser Verbindungen.

9. Benzothiopyrano[4,3,2-*cd*]indazolverbindungen nach Anspruch 1, die in Form der freien Base die Strukturformel 16

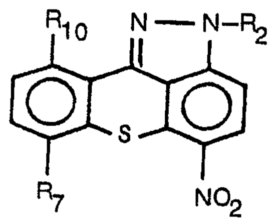

16

haben, und deren pharmazeutisch akzeptable Salze;
worin $R_2$ die Bedeutung gemäß Anspruch 1 hat und $R_7$ und $R_{10}$ Wasserstoff, Hydroxy oder Niedrig-$C_{1-4}$-Alkoxy bedeuten, wobei zumindest eines von $R_7$ und $R_{10}$ Hydroxy oder Niedrig-$C_{1-4}$-Alkoxy ist.

10. Benzothiopyrano[4,3,2-*cd*]indazolverbindungen nach Anspruch 1, die in Form der freien Base die Strukturformel 17

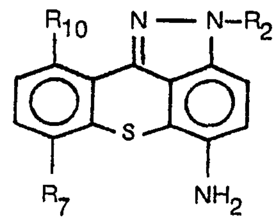

17

haben, und deren pharmazeutisch akzeptablen Salze;
worin $R_2$ die Bedeutung gemäß Anspruch 1 hat, und $R_7$ und $R_{10}$ Wasserstoff, Hydroxy oder Niedrig-$C_{1-4}$-Alkoxy bedeuten, wobei zumindest eines von $R_7$ und $R_{10}$ Hydroxy oder Niedrig-$C_{1-4}$-Alkoxy ist.

11. Benzothiopyrano[4,3,2-*cd*]indazolverbindungen nach Anspruch 1, die in Form der freien Base die Strukturformel 18

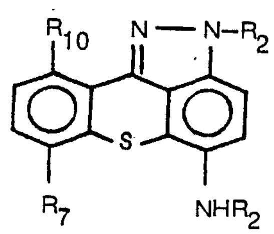

18

haben, und deren pharmazeutisch akzeptable Salze;
worin $R_2$ die Bedeutung gemäß Anspruch 1 hat, und $R_7$ und $R_{10}$ Wasserstoff, Hydroxy oder Niedrig-$C_{1-4}$-Alkoxy bedeuten, wobei zumindest eines von $R_7$ und $R_{10}$ Hydroxy oder Niedrig-$C_{1-4}$-Alkoxy ist.

12. Benzothiopyrano[4,3,2-*cd*]indazolverbindungen nach Anspruch 1, die in Form der freien Base die Strukturformel 24

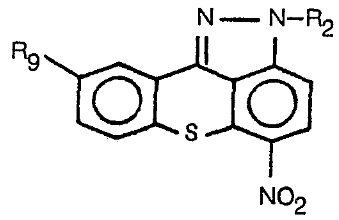

24

haben, und deren pharmazeutisch akzeptable Salze;
worin $R_9$ Hydroxy oder Alkoxy mit 1 bis 4 Kohlenstoffatomen bedeutet und $R_2$ die Bedeutung gemäß Anspruch 1 hat.

13. Benzothiopyrano[4,3,2-*cd*]indazolverbindungen nach Anspruch 1, die in Form der freien Base die Strukturformel 25

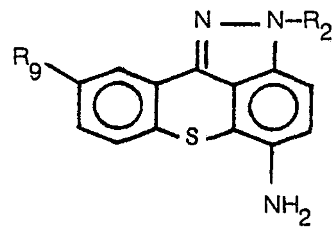

25

haben, und deren pharmazeutisch akzeptable Salze;
worin $R_9$ Hydroxy oder Alkoxy mit 1 bis 4 Kohlenstoffatomen bedeutet und $R_2$ die Bedeutung gemäß Anspruch 1 hat.

14. Benzothiopyrano[4,3,2-*cd*]indazolverbindungen nach Anspruch 1, die in Form der freien Base die Strukturformel 26

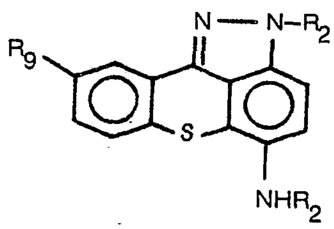

26

haben, und deren pharmazeutisch akzeptable Salze;
worin $R_9$ Hydroxy oder Alkoxy mit 1 bis 4 Kohlenstoffatomen bedeutet und $R_2$ die Bedeutung gemäß Anspruch 1 hat.

15. Verfahren zur Herstellung von Benzothiopyrano[4,3,2-*cd*]indazolverbindungen mit der Strukturformel 1

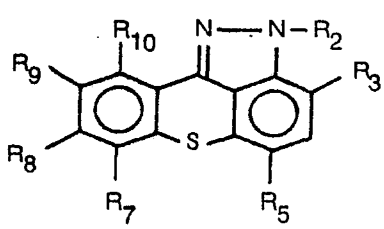

1

gemäß Anspruch 1, welches umfaßt: Umsetzung eines 1-Chlor-4-nitro-9*H*-thioxanthen-9-ons und eines $R_2$-substituierten Hydrazins zur Bildung der in Anspruch 1 beanspruchten Verbindungen, worin $R_5$ Nitro bedeutet; und gewünschtenfalls Überführung dieser Verbindungen durch Reduktion in Verbindungen wie in Anspruch 1 beansprucht, worin $R_5$ für $NH_2$ steht; und, falls weiters erwünscht, Überführung der resultierenden Verbindungen, worin $R_5$ für $NH_2$ steht, in Verbindungen gemäß Anspruch 1, worin $R_5$ für $NHR_2$ steht, durch Alkylierung mit einem Halogenalkylamin mit der Formel $XR_2$, gegebenenfalls nach Versehen empfindlicher Gruppen mit Schutzgruppen; oder durch Umsetzung mit einem Aldehyd oder Acetal, Keton oder Ketal und Reduktion der resultierenden Schiffschen Base oder durch Monoacylierung mit einem reaktiven Derivat eines Acylierungsmittels der Formel $R^aCOOH$ und Reduktion des acylierten Produkts, gegebenenfalls nach Versehen empfindlicher Gruppen mit Schutzgruppen; und falls weiters

erwünscht, Überführung der Verbindungen, worin $R_5$ für $NHR_2$ steht, in Verbindungen, worin $R_5$ für $NR'R_2$ steht, durch weitere Acylierung und Reduktion; und gewünschtenfalls Entfernung der Schutzgruppen durch Hydrolyse oder Reduktion; und Isolierung des Produkts in Form der freien Base oder des Säureadditionssalzes; worin X Chlor oder Brom bedeutet und $R_2$, $R_3$, R', $R_5$, $R_7$, $R_8$, $R_9$ und $R_{10}$ die Bedeutung gemäß Anspruch 1 haben, und $R^a$ ein gerades oder verzweigtes Alkyl mit 1 bis 3 Kohlenstoffatomen bedeutet.

16. Verfahren zur Herstellung von Benzothiopyrano[4,3,2-*cd*]indazolverbindungen nach Anspruch 1, die in Form der freien Base die Strukturformel

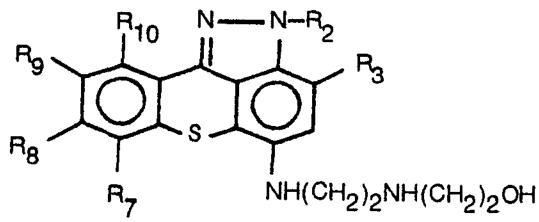

haben, durch Umsetzung einer Verbindung mit der Strukturformel

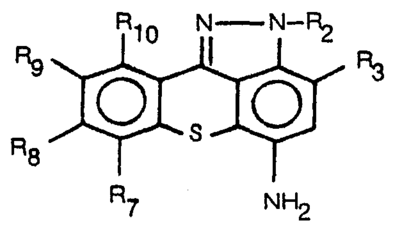

worin $R_3$, $R_7$, $R_8$, $R_9$ und $R_{10}$ Wasserstoff, Hydroxyl, Niedrig-$C_{1-4}$-Alkoxy, Benzyloxy oder p-halogen- oder p-methoxysubstituiertes Benzyloxy bedeuten, mit 3-(β-Halogenäthyl)-2-oxazolidinon zum Erhalt eines Oxazolidinons mit der Strukturformel

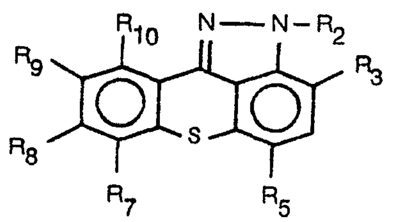

worin $R_5$ eine N-[2-(2-Oxo-3-oxazolidinyl)-äthyl]-aminogruppe bedeutet, und Unterwerden der letzteren Verbindung einer alkalischen Hydrolyse zum Erhalt einer Verbindung mit der Strukturformel

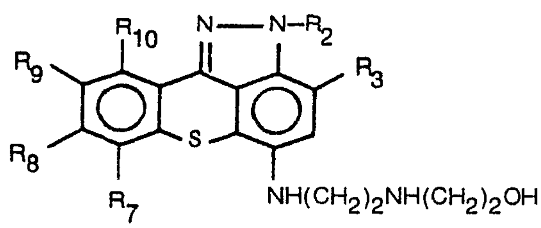

falls notwendig, Entfernung der Benzyloxygruppen durch Hydrogenolyse; und Isolierung des Produkts in der Form der freien Base oder eines Säureadditionssalzes.

17. Verfahren nach Anspruch 16, worin eines oder mehrere von $R_3$, $R_7$, $R_8$, $R_9$ und $R_{10}$ Benzyloxy oder Alkoxy bedeuten, worin die Benzyloxy-und Alkoxygruppen durch Hydrolyse oder Behandlung mit Bortribromid entfernt werden.

18. Pharmazeutische Zusammensetzung umfassend eine Verbindung mit der Strukturformel 1 nach Anspruch 1 in Kombination mit einem pharmazeutisch akzeptablen Träger.

19. Pharmazeutische Zusammensetzung umfassend eine Verbindung mit der Strukturformel 2 nach Anspruch 2 in Kombination mit einem pharmazeutisch akzeptablen Träger.

20. Pharmazeutische Zusammensetzung umfassend eine Verbindung mit der Strukturformel 4a nach Anspruch 3 in Kombination mit einem pharmazeutisch akzeptablen Träger.

21. Pharmazeutische Zusammensetzung umfassend eine Verbindung mit der Strukturformel 6 nach Anspruch 7 in Kombination mit einem pharmazeutisch akzeptablen Träger.

**Patentansprüche für den Vertragsstaat: At**

1. Verfahren zur Herstellung von Benzothiopyrano[4,3,2-*cd*]indazolverbindungen mit der Strukturformel 1

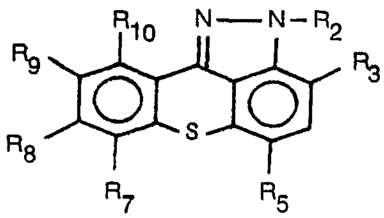

1

und deren pharmazeutisch akzeptablen Salzen; worin die Substituenten $R_3$, $R_7$, $R_8$, $R_9$ und $R_{10}$ Wasserstoff, Hydroxy oder Alkoxy mit 1 bis 4 Kohlenstoffatomen bedeuten; worin $R_2$ ANR'R'' bedeutet; worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

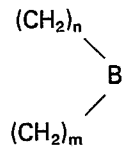

bedeuten, worin n und m jeweils 2 oder 3 sind und B eine Direktbindung oder O, S oder NR''' bedeutet; worin R''' Wasserstoff oder gerades oder verzweigtes Alkyl mit 1 bis 4 Kohlenstoffatomen, gegebenenfalls durch Hydroxyl substituiert, bedeutet; und worin $R_5$ für Nitro, $NH_2$,

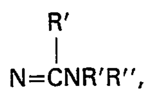

NHR' oder $NR'R_2$ oder NR'R'''' steht, worin R'''' 1 bis 4 Kohlenstoff-Acyl, Chloracetyl oder

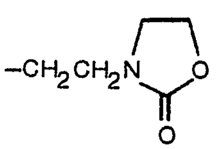

bedeutet, welches Verfahren umfaßt: Umsetzung eines 1-Chlor-4-nitro-9*H*-thioxanthen-9-ons und eines $R_2$-substituierten Hydrazins zur Bildung der Verbindungen, worin $R_5$ Nitro bedeutet; und gewünschtenfalls Überführung dieser Verbindungen durch Reduktion in die Verbindungen, worin $R_5$ für $NH_2$ steht, und, falls weiters erwünscht, Überführung der resultierenden Verbindungen, worin $R_5$ für $NH_2$ steht, in Verbindungen, worin $R_5$ für $NHR_2$ steht, durch Alkylierung mit einem Halogenalkylamin mit der Formel $XR_2$, gegebenenfalls nach Versehen empfindlicher Gruppen mit Schutzgruppen; oder durch Umsetzung mit einem Aldehyd, Acetal, Keton oder Ketal und Reduktion der resultierenden Schiffschen Base; oder durch Monoacylierung mit einem reaktiven Derivat eines Acylierungsmittels der Formel $R^aCOOH$ und Reduktion des acylierten Produkts, gegebenenfalls nach Versehen empfindlicher Gruppen mit Schutzgruppen; und, falls weiters erwünscht, Überführung der Verbindungen, worin $R_5$ für $NHR_2$ steht, in Verbindungen, worin $R_5$ $NR'R_2$ bedeutet, durch weitere Acylierung und Reduktion, und gewünschtenfalls Entfernen der Schutzgruppen durch Hydrolyse oder Reduktion; und Isolierung des Produkts in Form der freien Base oder des Säureadditionssalzes, worin X für Chlor oder Brom steht und $R^a$ ein gerades oder verzweigtes Alkyl mit 1 bis 3 Kohlenstoffatomen bedeutet.

2. Verfahren zur Herstellung von Benzothiopyrano[4,3,2-cd]indazolverbindungen, die in Form der freien Base die Strukturformel

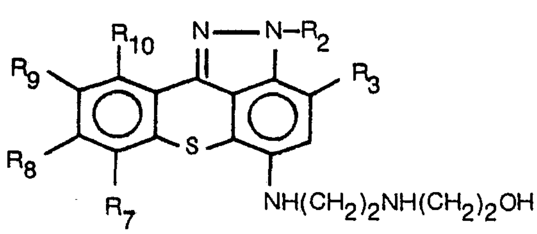

haben, und deren pharmazeutisch akzeptablen Salzen,
worin $R_2$, $R_3$, $R_7$, $R_8$, $R_9$ und $R_{10}$ der Definition des Anspruchs 1 entsprechen, welches Verfahren umfaßt: Umsetzung einer Verbindung mit der Strukturformel

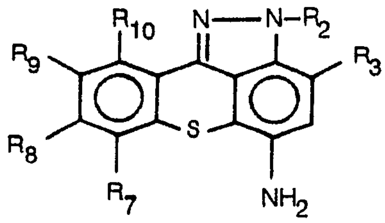

worin $R_3$, $R_7$, $R_8$, $R_9$ und $R_{10}$ Wasserstoff, Hydroxyl, Niedrigalkoxy, Benzyloxy oder p-halogen- oder p-methoxysubstituiertes Benzyloxy bedeuten, mit 3-(β-Halogenäthyl)-2-oxazolidinon zum Erhalt eines Oxazolidinons mit der Strukturformel

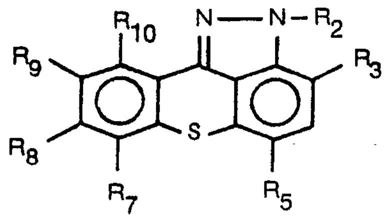

worin $R_5$ eine N-[2-(2-Oxo-3-oxazolidinyl)-äthyl]-aminogruppe bedeutet; und
Unterwerfen der letzteren Verbindung einer alkalischen Hydrolyse zum Erhalt einer Verbindung mit der Strukturformel

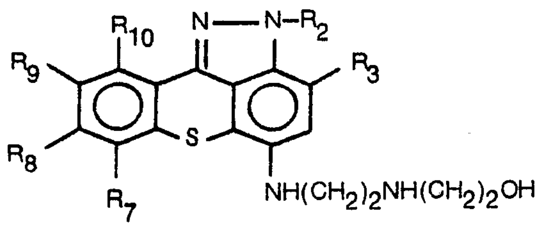

und, falls notwendig, Entfernung der Benzyloxygruppen durch Hydrogenolyse; und
Isolierung des Produkts in Form der freien Base oder des Säureadditionssalzes.

3. Verfahren nach Anspruch 2, worin eines oder mehrere von $R_3$, $R_7$, $R_8$, $R_9$ und $R_{10}$ Benzyloxy oder Alkoxy bedeuten, worin die Benzyloxy-und Alkoxygruppen durch Hydrolyse oder durch Behandlung mit Bortribromid entfernt werden.

4. Verfahren nach Anspruch 1 zur Herstellung von Benzothiopyrano[4,3,2-*cd*]indazolverbindungen, die in Form der freien Base die Strukturformel 2

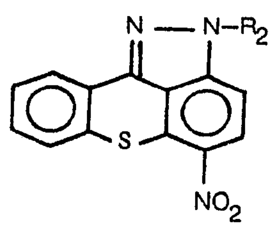

2

haben, und deren pharmazeutisch akzeptablen Salzen,
worin $R_2$ für ANR'R'' steht, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

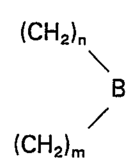

bedeuten, worin n und m jeweils 2 oder 3 sind und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet.

5. Verfahren nach Anspruch 1, 2 oder 3 zur Herstellung von Benzothiopyrano[4,3,2-*cd*]indazolverbindungen die in Form der freien Base die Strukturformel 4a

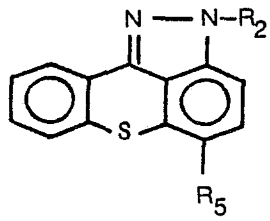

4a

haben, und deren pharmazeutisch akzeptablen Salzen, worin $R_2$ für ANR'R'' steht, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

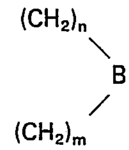

bedeuten, worin n und m jeweils 2 oder 3 sind und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet, und worin $R_5$ Nitro, $NH_2$,

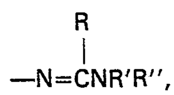,

NHR', $NR'R_2$oder NR'R'''' bedeutet, worin R'''' 1 bis 4 Kohlenstoff-Acyl, Chloracetyl oder

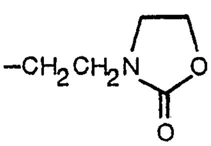.

bedeutet.

6. Verfahren nach Anspruch 1, 2, 3 oder 5, worin $R_2$ Diäthylaminoäthyl bedeutet und $R_5$ für $NHCH_2CH_2NHCH_2CH_2OH$ steht.

7. Verfahren nach Anspruch 1, 2, 3 oder 5, worin $R_2$ für $CH_2CH_2NHCH_2CH_2OH$ steht und $R_5$ $NHCH_2CH_2NHCH_2CH_2OH$ bedeutet.

8. Verfahren nach Anspruch 1 oder 5, worin $R_2$ Diäthylaminoäthyl bedeutet und $R_5$ für Aminoäthylamino steht.

9. Verfahren nach Anspruch 1, 2, 3 oder 5 zur Herstellung von Benzothiopyrano[4,3,2-*cd*]indazol, das in Form der freien Base die Strukturformel 6

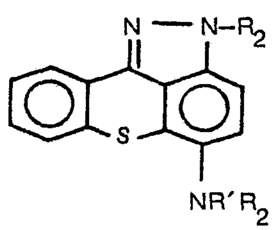

6

hat, und dessen pharmazeutisch akzeptablen Salzen, worin $R_2$ für ANR'R'' stet, worin A eine gerade oder verzweigte Alkylenkette mit 2 bis 5 Kohlenstoffatomen, gegebenenfalls durch Hydroxyl substituiert, bedeutet; worin R' und R'' Wasserstoff oder gerades oder verzweigtes Alkyl mit 1 bis 4 Kohlenstoffatomen, gegebenenfalls durch Hydroxyl substituiert, bedeuten, wobei R' und R'' zusammengenommen auch

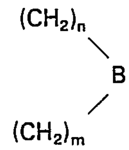

bedeuten, worin n und m jeweils 2 oder 3 sind und B eine Direktbindung oder O, S oder NR''' bedeutet, worin R''' Wasserstoff oder gerades oder verzweigtes Alkyl mit 1 bis 4 Kohlenstoffatomen, gegebenenfalls durch Hydroxyl substituiert, bedeutet.

10. Verfahren nach Anspruch 1, 2, 3 oder 5 zur Herstellung von

2-[[2-[[2-[2-(Diäthylamino)-äthyl]-2*H*[1]benzothiopyrano[4,3,2-*cd*]indazol-5-yl]-amino]-äthyl]-amino]-äthanol;

2-[[2-[5-[[2-[(2-Hydroxyäthyl)-amino]-äthyl]-amino]-2*H*[1]benzothiopyrano[4,3,2-*cd*]indazol-2-yl]-äthyl]-amino]-äthanol;

N-[2-[2-(Diäthylamino)-äthyl]-2H[1]benzothiopyrano[4,3,2-*cd*]indazol-5-yl]-1,2-äthandiamin;

*N*,*N*-Diäthyl-5-nitro-2H[1]benzothiopyrano[4,3,2-*cd*]indazol-2-äthanamin;

5-Nitro-2H[1]benzothiopyrano[4,3,2-*cd*]indazol-2-äthanamin;

2-[[2-(5-Nitro-2H[1]benzothiopyrano[4,3,2-*cd*]indazol-2-yl)-äthyl]-amino]-äthanol;

5-Amino-*N*,*N*-diäthyl-2*H*[1]benzothiopyrano[4,3,2-*cd*]indazol-2-äthanamin;

*N*,*N*-Diäthyl-9-methoxy-5-nitro-2*H*[1]benzothiopyrano[4,3,2-*cd*]indazol-2-äthanamin;

*N*-[2-[2-(Diäthylamino)-äthyl]-2*H*[1]benzothiopyrano[4,3,2-*cd*]indazol-5-yl]-1,3,-propandiamin;

5-Amino-*N*,*N*-dimethyl-2*H*[1]benzothiopyrano[4,3,2-*cd*]indazol-2-propanamin;

*N*'-[2-[2-(Diäthylamino)-äthyl]-2*H*[1]benzothiopyrano[4,3,2-*cd*]indazol-5-yl]-*N*,*N*-dimethylmethanimidamid;

3-[2-[[2-[2-(Diäthylamino)-äthyl]-2*H*[1]benzothiopyrano[4,3,2-*cd*]indazol-5-yl]-amino]-äthyl]-2-oxazolidinon;

*N*,*N*-Dimethyl-5-nitro-2*H*[1]benzothiopyrano[4,3,2-*cd*]indazol-2-propanamin;

2-[[2-(5-Amino-2*H*[1]benzothiopyrano[4,3,2-*cd*]indazol-2-yl)-äthyl]-amino]-äthanol;

2-[[2-[[2-(2-Aminoäthyl)-2*H*[1]benzothiopyrano[4,3,2-*cd*]indazol-5-yl]-amino]-äthyl]-amino]-äthanol;

5-Amino-2-[2-(diäthylamino)-äthyl]-2*H*[1]benzothiopyrano[4,3,2-*cd*]indazol-9-ol;

2-[2-(Diäthylamino)-äthyl]-5-[[2-[(2-hydroxyäthyl)-amino]-äthyl]-amino]-2*H*[1]benzothiopyrano[4,3,2-*cd*]indazol-9-ol;

*N*-[2-[3-(Dimethylamino)-propyl]-2*H*[1]benzothiopyrano[4,3,2-*cd*]indazol-5-yl]-1,2-äthandiamin;

3-[2-[[2-[2-(Diäthylamino)-äthyl]-9-hydroxy-2*H*[1]benzothiopyrano[4,3,2-*cd*]indazol-5-yl];

2-[2-(Diäthylamino)-äthyl]-5-[[2-[(2-hydroxyäthyl)-aminoäthyl]-amino-2H[1]benzothiopyrano[4,3,2-*cd*]indazol-9-ol;

*N*,*N*-Diäthyl-7-methoxy-5-nitro-2H[1]benzothiopyrano[4,3,2-*cd*]indazol-2-äthanamin;

5-Amino-*N*,*N*-diäthyl-7-methoxy-2*H*[1]benzothiopyrano[4,3,2-*cd*]indazol-2-äthanamin;

*N*-[2-[2-(Diäthylamino)-äthyl]-7-methoxy-2*H*[1]benzothiopyrano[4,3,2-*cd*]indazol-5-yl]-1,2-äthandiamin-Hydrobromidsalz;

5-[2-(Aminoäthyl)-amino]-2-[2-(diäthylamino)-äthyl]-2*H*[1]benzothiopyrano[4,3,2-*cd*]indazol-7-ol-Hydrobromidsalz;

5-Amino-2-[2-(diäthylamino)-äthyl]-2*H*[1]benzothiopyrano[4,3,2-*cd*]indazol-7-ol;

*N*,*N*-Diäthyl-8-methoxy-5-nitro-2*H*[1]benzothiopyrano[4,3,2-*cd*]indazol-2-äthanamin;

5-Amino-*N*,*N*-diäthyl-8-methoxy-2*H*[1]benzothiopyrano[4,3,2-*cd*]indazol-2-äthanamin;

*N*-[2-[2-(Diäthylamino)-äthyl]-8-methoxy-2*H*[1]benzothiopyrano[4,3,2-*cd*]indazol-5-yl]-1,2-äthandiamin-Hydrobromidsalz;

5-[2-(Aminoäthyl)-amino]-2-[2-(diäthylamino)-äthyl]-2*H*[1]benzothiopyrano[4,3,2-*cd*]indazol-8-ol-Hydrobromidsalz;

5-Amino-2-[2-(diäthylamino)-äthyl]-2*H*[1]benzothiopyrano[4,3,2-*cd*]indazol-8-ol;

*N*,*N*-Diäthyl-10-methoxy-5-nitro-2*H*[1]benzothiopyrano[4,3,2-*cd*]indazol-2-äthanamin;

5-Amino-*N*,*N*-diäthyl-10-methoxy-2*H*[1]benzothiopyrano[4,3,2-*cd*]indazol-2-äthanamin;

*N*-[2-[2-(Diäthylamino)-äthyl]-10-methoxy-2*H*[1]benzothiopyrano[4,3,2-*cd*]indazol-5-yl]-1,2-äthanediamin-Hydrobromidsalz;

5-[2-(Aminoäthyl)-amino]-2-[2-(diäthylamino)-äthyl]-2*H*[1]benzothiopyrano[4,3,2-*cd*]indazol-10-ol-Hydrobromidsalz;

5-Amino-2-[2-(diäthylamino)-äthyl]-2*H*[1]benzothiopyrano[4,3,2-*cd*]indazol-10-ol;

*N*,*N*-Diäthyl-3-methoxy-5-nitro-2*H*[1]benzothiopyrano[4,3,2-*cd*]indazol-2-äthanamin;

5-Amino-*N*,*N*-diäthyl-3-methoxy-2*H*[1]benzothiopyrano[4,3,2-*cd*]indazol-2-äthanamin;

*N*-[2-[2-(Diäthylamino)-äthyl]-3-methoxy-2*H*[1]benzothiopyrano[4,3,2-*cd*]indazol-5-yl]-1,2-äthandiamin-Hydrobromidsalz;

5-[2-(Aminoäthyl)-amino]-2-[2-(diäthylamino)-äthyl]-2*H*[1]benzothiopyrano[4,3,2-*cd*]indazol-3-ol-Hydrobromidsalz;

5-Amino-2-[2-(diäthylamino)-äthyl]-2*H*[1]benzothiopyrano[4,3,2-*cd*]indazol-3-ol;

*N*,*N*-Diäthyl-3,9-dimethoxy-5-nitro-2*H*[1]benzothiopyrano[4,3,2-*cd*]indazol-2-äthanamin;

5-Amino-*N,N*-diäthyl-3,9-dimethoxy-2*H*[1]benzothiopyrano[4,3,2-*cd*]indazol-2-äthanamin;
*N*-[2-[2-(Diäthylamino)-äthyl]-3,9-dimethoxy-2*H*[1]benzothiopyrano[4,3,2-*cd*]indazol-5-yl]-1,2-äthandiamin-Hydrobromidsalz;
5-[2-(Aminoäthyl)-amino]-2-[2-(diäthylamino)-äthyl]-2*H*[1]benzothiopyrano[4,3,2-*cd*]indazol-3,9-diol-Hydrobromidsalz;
5-Amino-2-[2-(diäthylamino)-äthyl]-2H[1]benzothiopyrano[4,3,2-*cd*]indazol-3,9-diol;
2-[[2-[5-[[2-(Diäthylamino)-äthyl]-amino-2*H*[1]benzothiopyrano[4,3,2-*cd*]indazol-2-yl]-äthyl]-amino]-äthanol
und der pharmazeutisch akzeptablen Salze dieser Verbindungen.

11. Verfahren nach Anspruch 1 zur Herstellung von Benzothiopyrano[4,3,2-*cd*]indazolverbindungen, die in Form der freien Base die Strukturformel 16

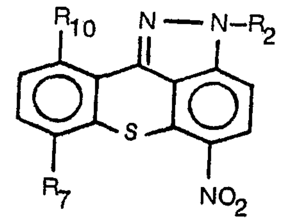

16

haben, und der pharmazeutisch akzeptablen Salze derselben; worin $R_2$ die Bedeutung gemäß Anspruch 1 hat und $R_7$ und $R_{10}$ Wasserstoff, Hydroxy oder Niedrig-$C_{1-4}$-Alkoxy bedeuten, wobei zumindest eines von $R_7$ und $R_{10}$ Hydroxy oder Niedrig-$C_{1-4}$-Alkoxy bedeutet.

12. Verfahren nach Anspruch 1 zur Herstellung von Benzothiopyrano[4,3,2-*cd*]indazolverbindungen, die in Form der freien Base die Strukturformel 17

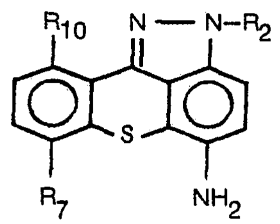

17

haben, und deren pharmazeutisch akzeptablen Salzen;
worin $R_2$ die Bedeutung gemäß Anspruch 1 hat und $R_7$ und $R_{10}$ für Wasserstoff, Hydroxy oder Niedrig-$C_{1-4}$-Alkoxy stehen, wobei zumindest eines von $R_7$ und $R_{10}$ Hydroxy oder Niedrig-$C_{1-4}$-Alkoxy bedeutet.

13. Verfahren nach Anspruch 1 oder 2 zur Herstellung von Benzothiopyrano[4,3,2-*cd*]indazolverbindungen, die in Form der freien Base die Strukturformel 18

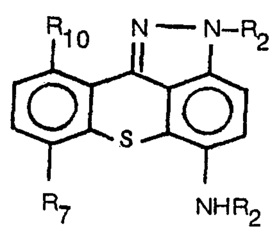

18

haben, und deren pharmazeutisch akzeptablen Salzen;
worin $R_2$ die Bedeutung gemäß Anspruch 1 hat und $R_7$ und $R_{10}$ Wasserstoff, Hydroxy oder Niedrig-$C_{1-4}$-Alkoxy bedeuten, wobei zumindest eines von $R_7$ und $R_{10}$ Hydroxy oder Niedrig-$C_{1-4}$-Alkoxy bedeutet.

14. Verfahren nach Anspruch 1 zur Herstellung von Benzothiopyrano[4,3,2-*cd*]indazolverbindungen, die in Form der freien Base die Strukturformel 24

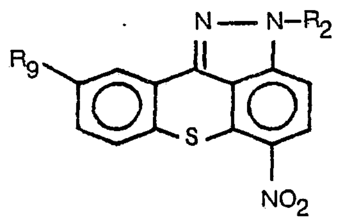

24

haben, und deren pharmazeutisch akzeptablen Salzen;

worin $R_9$ Hydroxy oder Alkoxy mit 1 bis 4 Kohlenstoffatomen bedeutet und $R_2$ die Bedeutung gemäß Anspruch 1 hat.

15. Verfahren nach Anspruch 1 zur Herstellung von Benzothiopyrano[4,3,2-*cd*]indazolverbindungen, die in Form der freien Base die Strukturformel 25

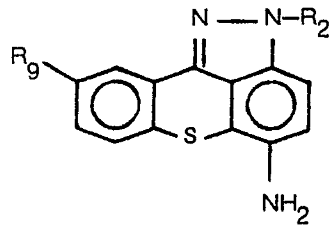

25

haben, und deren pharmazeutisch akzeptablen Salzen;
worin $R_9$ Hydroxy oder Alkoxy mit 1 bis 4 Kohlenstoffatomen bedeutet und $R_2$ die Bedeutung gemäß Anspruch 1 hat.

16. Verfahren nach Anspruch 1 oder 2 zur Herstellung von Benzothiopyrano[4,3,2-*cd*]indazolverbindungen, die in Form der freien Base die Strukturformel 26

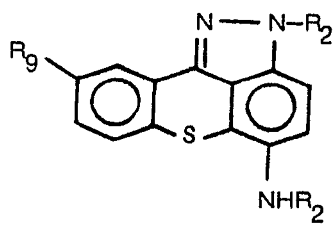

26

haben, und deren pharmazeutisch akzeptablen Salzen;
worin $R_9$ Hydroxy oder Alkoxy mit 1 bis 4 Kohlenstoffatomen bedeutet und $R_2$ die Bedeutung gemäß Anspruch 1 hat.

17. Verfahren zur Herstellung einer pharmazeutischen Zusammensetzung, welches Verfahren die Kombination einer Verbindung mit der Strukturformel 1 nach Anspruch 1 mit einem pharmazeutisch akzeptablen Träger umfaßt.

18. Verfahren zur Herstellung einer pharmazeutischen Zusammensetzung, welches Verfahren die Kombination einer Verbindung mit der Strukturformel 2 nach Anspruch 4 mit einem pharmazeutisch akzeptablen Träger umfaßt.

19. Verfahren zur Herstellung einer pharmazeutischen Zusammensetzung, welches Verfahren die Kombination einer Verbindung mit der Strukturformel 4a nach Anspruch 5 mit einem pharmazeutisch akzeptablen Träger umfaßt.

20. Verfahren zur Herstellung einer pharmazeutischen Zusammensetzung, welches Verfahren die Kombination einer Verbindung mit der Strukturformel 6 nach Anspruch 9 mit einem pharmazeutisch akzeptablen Träger umfaßt.

**Revendications pour les Etats contractants: BE CH DE FR GB IT LI LU NL SE**

1. Dérivés du benzothiopyrano[4,3,2-cd]indazole ayant, sous leur forme de base libre, la formule structurelle 1:

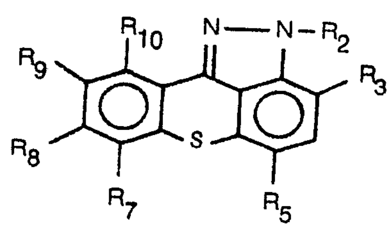

1

et leurs sels pharmaceutiquement acceptables;
dans laquelle: les substituants $R_3$, $R_7$, $R_8$, $R_9$ et $R_{10}$ représentent un atome d'hydrogène, un groupe hydroxy ou alkoxy renfermant de 1 à 4 atomes de carbone;

$R_2$ représente ANR'R'' dans lequel: A représente une chaîne alkylène droite ou ramifiée renfermant de 2 à 5 atomes de carbone, éventuellement substituée par un hydroxyle; R' et R'' représentent un atome d'hydrogène ou un radical alkyle à chaîne droite ou ramifiée renfermant de 1 à 4 atomes de carbone,

éventuellement substitué par un hydroxyle, R′ et R″ pris ensemble représentant également:

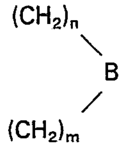

dans lequel: n et m sont chacun égaux à 2 ou 3; et B représente une liaison directe ou O, S, ou NR‴, dans lequel R‴ représente un atome d'hydrogène ou un radical alkyle à chaîne droite ou ramifiée renfermant de 1 à 4 atomes de carbone, éventuellement substitué par un hydroxyle; et $R_5$ représente un groupe nitro, $NH_2$,

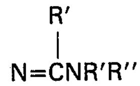

NHR′, ou $NR'R_2$ ou NR′R⁗ où R⁗ représente un radical acyle renfermant de 1 à 4 atomes de carbone, un radical chloroacétyle, ou

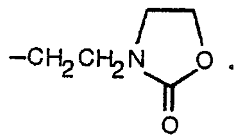

2. Dérivés du benzothiopyrano[4,3,2-cd]indazole selon la revendication 1, ayant sous leur forme de base libre la formule structurelle 2:

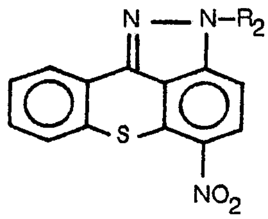

2

et leurs sels pharmaceutiquement acceptables, dans laquelle:

$R_2$ représente ANR′R″, A représentant une chaîne alkylène droite ou ramifiée renfermant de 2 à 5 atomes de carbone, éventuellement substituée par un hydroxyle; R′ et R″ représentant un atome d'hydrogène ou un radical alkyle à chaîne droite ou ramifiée renfermant de 1 à 4 atomes de carbone, éventuellement substitué par un hydroxyle, R′ et R″ pris ensemble représentant également:

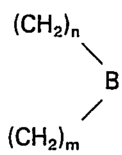

dans lequel: n et m sont chacun égaux à 2 ou 3; et B représente une liaison directe ou O, S, ou NR‴, dans lequel R‴ représente un atome d'hydrogène ou un radical alkyle à chaîne droite ou ramifiée renfermant de 1 à 4 atomes de carbone, éventuellement substitué par un hydroxyle.

3. Dérivés du benzothiopyrano[4,3,2-cd]indazole selon la revendication 1, ayant sous leur forme de base libre la formule structurelle 4a:

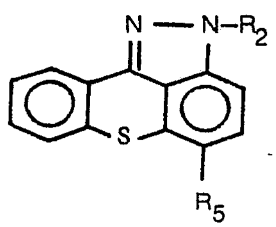

4a

et leurs sels pharmaceutiquement acceptables, dans laquelle:

$R_2$ représente ANR′R″, A représentant une chaîne alkylène droite ou ramifiée renfermant de 2 à 5

atomes de carbone, éventuellement substituée par un hydroxyle; R′ et R″ représentant un atome d'hydrogène ou un radical alkyle à chaîne droite ou ramifiée renfermant de 1 à 4 atomes de carbone, éventuellement substitué par un hydroxyle, R′ et R″ pris ensemble représentant également:

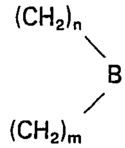

dans lequel: n et m sont chacun égaux à 2 ou 3; et B représente une liaison directe ou O, S, ou NR‴, R‴ représentant un atome d'hydrogène ou un radical alkyle à chaîne droite ou ramifiée renfermant de 1 à 4 atomes de carbone, éventuellement substitué par un hydroxyle; et $R_5$ représente un groupe nitro, $NH_2$,

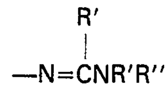

NHR′, ou $NR'R_2$ ou NR′R⁗ où R⁗ représente un radical acyle renfermant de 1 à 4 atomes de carbone, chloroacétyle, ou

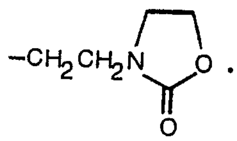

4. Un composé selon la revendication 3, dans lequel $R_2$ représente le groupe diéthylaminoéthyle et $R_5$ représente $NHCH_2CH_2NHCH_2CH_2OH$.

5. Un composé selon la revendication 3, dans lequel $R_2$ représente $CH_2CH_2NHCH_2CH_2OH$ et $R_5$ représente $NHCH_2CH_2NHCH_2CH_2OH$.

6. Un composé selon la revendication 3, dans lequel $R_2$ représente le groupe diéthylaminoéthyle et $R_5$ représente un radical aminoéthylamino.

7. Dérivés du benzothiopyrano[4,3,2-cd]indazole selon la revendication 1, ayant sous leur forme de base libre la formule structurelle 6:

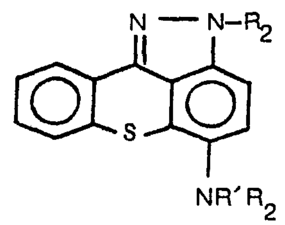

6

et leurs sels pharmaceutiquement acceptables, dans laquelle:

$R_2$ représente ANR′R″, A représentant une chaîne alkylène droite ou ramifiée renfermant de 2 à 5 atomes de carbone, éventuellement substituée par un hydroxyle; R′ et R″ représentant un atome d'hydrogène ou un radical alkyle à chaîne droite ou ramifiée renfermant de 1 à 4 atomes de carbone, éventuellement substitué par un hydroxyle, R′ et R″ pris ensemble représentant également:

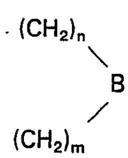

dans laquel: n et m sont chacun égaux à 2 ou 3; et B représente une liaison directe ou O, S, ou NR‴, dans lequel R‴ représente un atome d'hydrogène ou un radical alkyle à chaîne droite ou ramifiée renfermant de 1 à 4 atomes de carbone, éventuellement substitué par un hydroxyle.

8. Composés selon la revendication 1, qui sont:

. le 2-{[2-[[2-[2-(diéthylamino)éthyl]-2H(1)benzothiopyrano[4,3,2-cd]indazol-5-yl]amino]éthyl]amino}éthanol;

. le 2-{[2-[5-[[2-[(2-hydroxyéthyl)amino]éthyl]amino]-2H(1)benzothiopyrano[4,3,2-cd]indazol-2-yl]éthyl]amino}éthanol;

. la N-[2-[2-(diéthylamino)éthylamino)éthyl]-2H(1)benzothiopyrano[4,3,2-cd]indazol-5-yl]-1,2-éthanediamine;
. la N,N-diéthyl-5-nitro-2H(1)benzothiopyrano[4,3,2-cd]indazole-2-éthanamine;
. la 5-nitro-2H(1)benzothiopyrano[4,3,2-cd]indazole-2-éthanamine;
. le 2-{[2-(5-nitro-2H(1)benzothiopyrano[4,3,2-cd]indazol-2-yl)éthyl]amino}éthanol;
. la 5-amino-N,N-diéthyl-2H(1)benzothiopyrano[4,3,2-cd]indazole-2-éthanamine;
. la N,N-diéthyl-9-méthoxy-5-nitro-2H(1)benzothiopyrano[4,3,2-cd]indazole-2-éthanamine;
. la N-{2-[2-(diéthylamino)éthyl]-2H(1)benzothiopyrano[4,3,2-cd]indazol-5-yl}-1,3-propanediamine;
. la 5-amino-N,N-diméthyl-2H(1)benzothiopyrano[4,3,2-cd]indazole-2-propanamine;
. le N'-{2-[2-(diéthylamino)éthyl]-2H(1)benzothiopyrano[4,3,2-cd]indazol-5-yl}-N,N-diméthylméthanimidamide;
. la 3-{2-[[2-[2-(diéthylamino)éthyl]-2H(1)benzothiopyrano[4,3,2-cd]indazol-5-yl]amino]éthyl}-2-oxazolidinone;
. la N,N-diméthyl-5-nitro-2H(1)benzothiopyrano[4,3,2-cd]indazole-2-propanamine;
. le 2-{[2-(5-amino-2H(1)benzothiopyrano[4,3,2-cd]indazol-2-yl)éthyl]amino}éthanol;
. le 2-{[2-[[2-(2-aminoéthyl)-2H(1)benzothiopyrano[4,3,2-cd]indazol-5-yl]amino]éthyl]amino}éthanol;
. le 5-amino-2-{2-(diethylamino)éthyl}-2H(1)benzothiopyrano[4,3,2-cd]indazol-9-ol;
. le 2-{2-(diéthylamino)éthyl}-5-{[2-[(2-hydroxyéthyl)amino]éthyl]amino}-2H(1)benzothiopyrano[4,3,2-cd]indazol-9-ol;
. la N-{2-[3-(diméthylamino)propyl]-2H(1)benzothiopyrano[4,3,2-cd]indazol-5-yl}-1,2-éthanediamine;
. le 3-{2-[[2-[2-(diéthylamino)éthyl]-9-hydroxy-2H(1)benzothiopyrano[4,3,2-cd]indazol-5-yl];
. le 2-{2-(diéthylamino)éthyl}-5-{[2-[(2-hydroxyéthyl)amino]éthyl]amino}-2H(1)benzothiopyrano[4,3,2-cd]indazol-9-ol;
. la N,N-diéthyl-7-méthoxy-5-nitro-2H(1)benzothiopyrano[4,3,2-cd]indazole-2-éthanamine;
. la 5-amino-N,N-diéthyl-7-méthoxy-2H(1)benzothiopyrano[4,3,2-cd]indazole-2-éthanamine;
. le bromhydrate de N-{2-[2-(diéthylamino)éthyl]-7-méthoxy-2H(1)benzothiopyrano[4,3,2-cd]indazol-5-yl}-1,2-éthanediamine;
. le bromhydrate de 5-{2-(aminoéthyl)amino}-2-[2-(diéthylamino)éthyl]-2H(1)benzothiopyrano[4,3,2-cd]indazol-7-ol;
. le 5-amino-2-{2-(diéthylamino)éthyl}-2H(1)benzothiopyrano[4,3,2-cd]indazol-7-ol;
. la N,N-diéthyl-8-méthoxy-5-nitro-2H(1)benzothiopyrano[4,3,2-cd]indazol-2-éthanamine;
. la 5-amino-N,N-diéthyl-8-méthoxy-2H(1)benzothiopyrano[4,3,2-cd]indazol-2-éthanamine;
. le bromhydrate de N-{2-[2-(diéthylamino)éthyl]-8-méthoxy-2H(1)benzothiopyrano[4,3,2-cd]indazol-5-yl}-1,2-éthanediamine;
. le bromhydrate de 5-{2-(aminoéthyl)amino}-2-[2-(diéthylamino)éthyl]-2H(1)benzothiopyrano[4,3,2-cd]indazol-8-ol;
. le 5-amino-2-{2-(diéthylamino)éthyl}-2H(1)benzothiopyrano[4,3,2-cd]indazol-8-ol;
. la N,N-diéthyl-10-méthoxy-5-nitro-2H(1)benzothiopyrano[4,3,2-cd]indazole-2-éthanamine;
. la 5-amino-N,N-diéthyl-10-méthoxy-2H(1)benzothiopyrano[4,3,2-cd]indazole-2-éthanamine;
. le bromhydrate de N-{2-[2-(diéthylamino)éthyl]-10-méthoxy-2H(1)benzothiopyrano[4,3,2-cd]indazol-5-yl}-1,2-éthanediamine;
. le bromhydrate de 5-{2-(aminoéthyl)amino}-2-[2-(diéthylamino)éthyl]-2H(1)benzothiopyrano[4,3,2-cd]indazol-10-ol;
. le 5-amino-2-{2-(diéthylamino)éthyl}-2H(1)benzothiopyrano[4,3,2-cd]indazol-10-ol;
. la N,N-diéthyl-3-méthoxy-5-nitro-2H(1)benzothiopyrano[4,3,2-cd]indazole-2-éthanamine;
. la 5-amino-N,N-diéthyl-3-méthoxy-2H(1)benzothiopyrano[4,3,2-cd]indazole-2-éthanamine;
. le bromhydrate de N-{2-[2-(diéthylamino)éthyl]-3-méthoxy-2H(1)benzothiopyrano[4,3,2-cd]indazol-5-yl}-1,2-éthanediamine;
. le bromhydrate de 5-{2-(aminoéthyl)amino}-2-[2-(diéthylamino)éthyl]-2H(1)benzothiopyrano[4,3,2-cd]indazol-3-ol;
. le 5-amino-2-{2-(diéthylamino)éthyl}-2H(1)benzothiopyrano[4,3,2-cd]indazole-3-ol;
. la N,N-diéthyl-3,9-diméthoxy-5-nitro-2H(1)benzothiopyrano[4,3,2-cd]indazol-2-éthanamine;
. la 5-amino-N,N-diéthyl-3,9-diméthoxy-2H(1)benzothiopyrano[4,3,2-cd]indazole-2-éthanamine;
. le bromhydrate de N-{2-[2-(diéthylamino)éthyl]-3,9-diméthoxy-2H(1)benzothiopyrano[4,3,2-cd]indazol-5-yl}-1,2-éthanediamine;
. le bromhydrate de 5-{2-(aminoéthyl)amino}-2-[2-(diéthylamino)éthyl]-2H(1)benzothiopyrano[4,3,2-cd]indazol-3,9-diol;
. le 5-amino-2-{2-(diéthylamino)éthyl}-2H(1)benzothiopyrano[4,3,2-cd]indazol-3,9-diol;
. le 2-{[2-[5-[[2-(diethylamino)éthyl]amino]-2H(1)benzothiopyrano[4,3,2-cd]indazol-2-yl]éthyl]amino}éthanol;
et les sels pharmaceutiquement acceptables de ces composés.

9. Dérivés du benzothiopyrano[4,3,2-cd]indazole selon la revendication 1, ayant sous leur forme de base libre la formule structurelle 16:

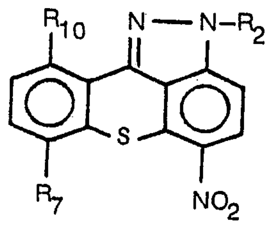

16

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

10. Dérivés du benzothiopyrano[4,2,3-cd]indazole selon la revendication 1, ayant sous leur forme de base libre la formule structurelle 17:

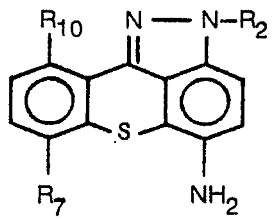

17

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

11. Dérivés du benzothiopyrano[4,3,2-cd]indazole selon la revendication 1, ayant sous leur forme de base libre la formule structurelle 18:

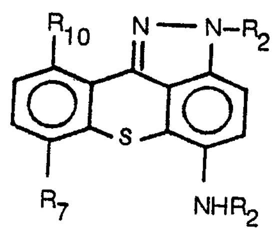

18

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

12. Dérivés du benzothiopyrano[4,3,2-cd]indazole selon la revendication 1, ayant sous leur forme de base libre la formule structurelle 24:

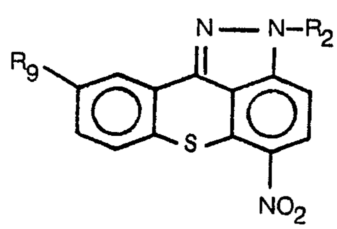

24

et leurs sels pharmaceutiquement acceptables,
dans laquelle: $R_9$ représente un groupe hydroxy ou alkoxy renfermant de 1 à 4 atomes de carbone; et $R_2$ est défini comme à la revendication 1.

13. Dérivés du benzothiopyrano[4,3,2-cd]indazole selon la revendication 1, ayant sous leur forme de base libre la formule structurelle 25:

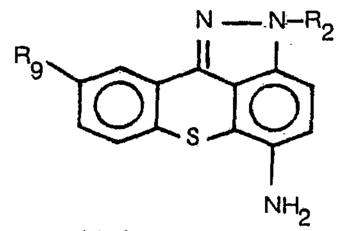

25

et leurs sels pharmaceutiquement acceptables,
dans laquelle: $R_9$ représente un groupe hydroxy ou alkoxy renfermant de 1 à 4 atomes de carbone; et $R_2$ est défini comme à la revendication 1.

14. Dérivés du benzothiopyrano[4,3,2-cd]indazole selon la revendication 1, ayant sous leur forme de base libre la formule structurelle 26:

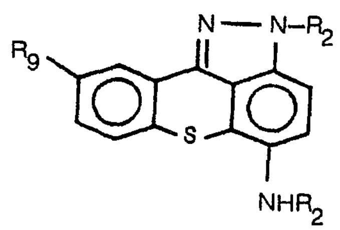

26

et leurs sels pharmaceutiquement acceptables,
dans laquelle: $R_9$ représente un groupe hydroxy ou alkoxy renfermant de 1 à 4 atomes de carbone; et $R_2$ est défini comme à la revendication 1.

15. Procédé de préparation de dérivés du benzothiopyrano[4,3,2-cd]indazole de formule structurelle 1:

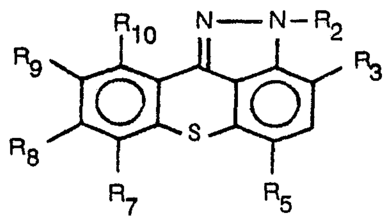

1

selon la revendication 1, qui consiste:

— à faire réagir une 1-chloro-4-nitro-9H-thioxanthen-9-one et une hydrazine substituée par le radical $R_2$ pour former les composés selon la revendication 1 dans lesquels $R_5$ représente un groupe nitro et, si on le souhaite,

— à transformer ces composés par réduction en composés selon la revendication 1 dans lesquels $R_5$ représente $NH_2$; et si on le souhaite encore,

— à transformer les composés qui en résulte dans lesquels $R_5$ représente $NH_2$ en composés selon la revendication 1 dans lesquels $R_5$ représente $NHR_2$:

. par alkylation à l'aide d'une halogénoalkylamine de formule $XR_2$ éventuellement après protection des groupes sensibles par des groupes protecteurs; ou

. par réaction avec un aldéhyde ou un acétal, une cétone ou un cétal et réduction de la base de Schiff qui en résulte, ou

. par monoacylation à l'aide d'un dérivé réactif d'un agent d'acylation de formule $R^aCOOH$ et réduction du produit acylé, éventuellement après protection des groupes sensibles par des groupes protecteurs; et si on le souhaite encore,

— à transformer les composés dans lesquels $R_5$ représente $NHR_2$ en composés dans lesquels $R_5$ représente $NR'R_2$ par acylation et réduction; et si on le souhaite,

— à éliminer les groupes protecteurs par hydrolyse ou réduction; et

— à isoler le produit sous sa forme de base libre ou sous sa forme de sels d'addition d'acides;

X représentant un atome de chlore ou de brome et $R_2$, $R_3$, R', $R_5$, $R_7$, $R_8$, $R_9$ et $R_{10}$ étant définis comme à la revendication 1 et $R^a$ représentant un radical alkyle à chaîne droite ou ramifiée renfermant de 1 à 3 atomes de carbone.

16. Procédé de préparation de dérivés du benzothiopyrano[4,3,2-cd]indazole selon la revendication 1, ayant sous leur forme de base libre la formule structurelle:

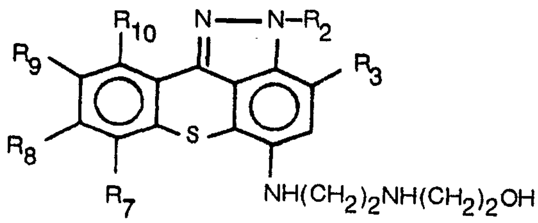

par réaction d'un composé de formule structurelle:

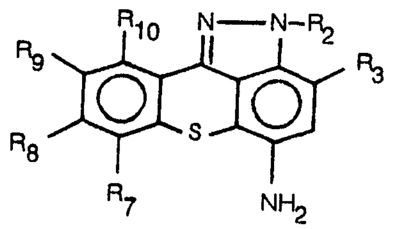

dans laquelle $R_3$, $R_7$, $R_8$, $R_9$ et $R_{10}$ représentent un atome d'hydrogène, un groupe hydroxyle, un radical alkoxy ayant de 1 à 4 atomes de carbone, benzyloxy ou benzyloxy substitué en para par un atome d'halogène ou par un groupe méthoxy, avec la 3-(β-halogénoéthyl)-2-oxazolidinone pour obtenir une oxazolidinone de formule structurelle:

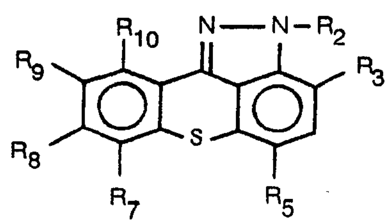

dans laquelle $R_5$ représente un groupe N-[2-(2-oxo-3-oxazolidinyl)éthyl]amino, et à soumettre ce dernier composé à une hydrolyse alcaline pour obtenir un composé de formule structurelle:

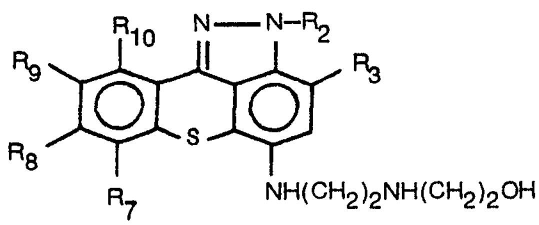

si nécessaire, à éliminer les groupes benzyloxy par hydrogénolyse; et à isoler le produit sous sa forme de base libre ou de sels d'addition d'acides.

17. Procédé selon la revendication 16, dans lequel un ou plusieurs des symboles $R_3$, $R_7$, $R_8$, $R_9$ et $R_{10}$ représentent un groupe benzyloxy ou alkoxy et dans lequel les groupes benzyloxy et alkoxy sont éliminés par hydrolyse ou par traitement au tribromure de bore.

18. Composition pharmaceutique comprenant un composé de formule structurelle 1 selon la revendication 1, en combinaison avec un support pharmaceutiquement acceptable.

19. Composition pharmaceutique comprenant un composé de formule structurelle 2 selon la revendication 2, en combinaison avec un support pharmaceutiquement acceptable.

20. Composition pharmaceutique comprenant un composé de formule structurelle 4a selon la revendication 3, en combinaison avec un support pharmaceutiquement acceptable.

21. Composition pharmaceutique comprenant un composé de formule structurelle 6 selon la revendication 7, en combinaison avec un support pharmaceutiquement acceptable.

**Revendications pour l'Etat contractant: AT:**

1. Procédé de préparation de dérivés du benzothiopyrano[4,3,2-cd]indazole de formule structurelle 1:

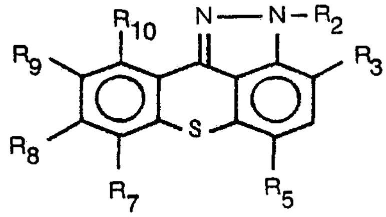

1

et leurs sels pharmaceutiquement acceptables;
dans laquelle: les substituants $R_3$, $R_7$, $R_8$, $R_9$ et $R_{10}$ représentent un atome d'hydrogène, un groupe hydroxy ou un radical alkoxy renfermant de 1 à 4 atomes de carbone;

$R_2$ représente ANR'R'' A représentant une chaîne alkylène droite ou ramifiée renfermant de 2 à 5 atomes de carbone, éventuellement substituée par un hydroxyle; R' et R'' représentent un atome d'hydrogène ou un radical alkyle à chaîne droite ou ramifiée renfermant de 1 à 4 atomes de carbone, éventuellement substitué par un hydroxyle, R' et R'' pris ensemble représentant également:

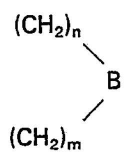

où: n et m sont chacun égaux à 2 ou 3; et B représente une liaison directe ou O, S, ou NR''', dans lequel R''' représente un atome d'hydrogène ou un radical alkyle à chaîne droite ou ramifiée renfermant de 1 à 4 atomes de carbone, éventuellement substitué par un groupe hydroxyle; et $R_5$ représente un groupe nitro, $NH_2$,

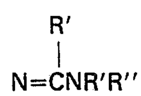

NHR', ou $NR'R_2$ ou NR'R'''' où R'''' représente un radical acyle renfermant de 1 à 4 atomes de carbone, un radical chloroacétyle, ou

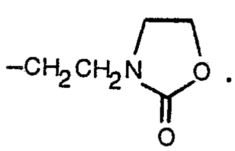

ce procédé consistant à:

— faire réagir une 1-chloro-4-nitro-9H-thioxanthèn-9-one et une hydrazine substituée par le radical $R_2$ pour former les composés dans lesquels $R_5$ représente un groupe nitro; et, si on le souhaite,

— à transformer ces composés, par réduction, en composés dans lesquels $R_5$ représente $NH_2$; et, si on le souhaite encore,

— à transformer les composés qui en résultent dans lesquels $R_5$ représente $NH_2$ en composés dans lesquels $R_5$ représente $NHR_2$ par alkylation à l'aide d'une halogénoalkylamine de formule $XR_2$, éventuellement après protection des groupes sensibles par des groupes protecteurs; ou par réaction avec un aldéhyde, un acétal, une cétone ou un cétal et réduction de la base de Schiff qui en résulte, ou par monoacylation à l'aide d'un dérivé réactif d'un agent d'acylation de formule $R^aCOOH$ et réduction du produit acylé, éventuellement après protection des groupes sensibles par des groupes protecteurs; et si on le souhaite encore,

— à transformer les composés dans lesquels $R_5$ représente $NHR_2$ en composés dans lesquels $R_5$ représente $NR'R_2$ par acylation et réduction; et si on le souhaite,

— à éliminer les groupes protecteurs par hydrolyse ou réduction; et

— à isoler le produit sous sa forme de base libre ou sous sa forme de sels d'addition d'acides;

X représentant un atome de chlore ou de brome et $R^a$ représentant un radical alkyle à chaîne droite ou ramifiée renfermant de 1 à 3 atomes de carbone.

2. Procédé de préparation de dérivés du benzothiopyrano[4,3,2-cd]indazole ayant sous leur forme de base libre la formule structurelle:

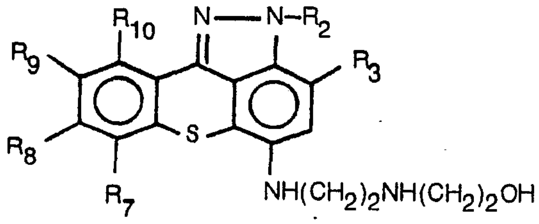

et leurs sels pharmaceutiquement acceptables,
dans laquelle $R_2$, $R_3$, $R_7$, $R_8$, $R_9$ et $R_{10}$ sont tels que définis à la revendication 1, ce procédé consistant:

— à faire réagir un composé de formule structurelle:

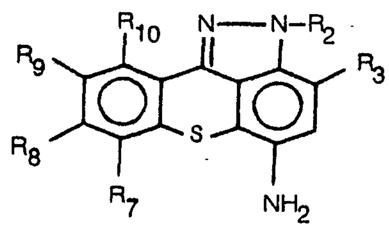

dans laquelle $R_3$, $R_7$, $R_8$, $R_9$ et $R_{10}$ représentent un atome d'hydrogène, un groupe hydroxyle, un radical alkoxy inférieur, benzyloxy ou benzyloxy substitué en para par un atome d'halogène ou par un radical méthoxy,
avec une 3-(β-halogénoéthyl)-2-oxazolidinone pour obtenir une oxazolidinone de formule structurelle:

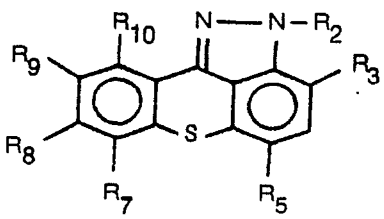

dans laquelle $R_5$ représente un groupe N-[2-(2-oxo-3-oxazolidinyl)éthyl]amino; et

— à soumettre ce dernier composé à une hydrolyse alcaline pour obtenir un composé de formule structurelle:

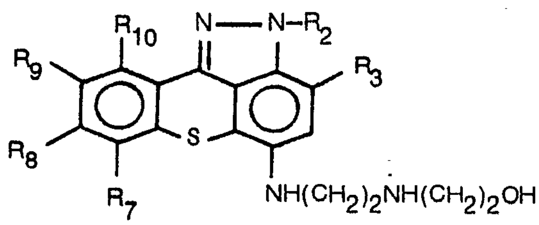

et, si cela est nécessaire,

— à éliminer les groupes benzyloxy par hydrogénolyse; et

— à isoler le produit sous sa forme de base libre ou de sels d'addition d'acides.

3. Procédé selon la revendication 2, dans lequel un ou plusieurs des symboles $R_3$, $R_7$, $R_8$, $R_9$ et $R_{10}$ représentent un radical benzyloxy ou alkoxy et dans lequel les groupes benzyloxy et alkoxy sont éliminés par hydrolyse ou par traitement avec du tribromure de bore.

4. Procédé selon la revendication 1 pour la préparation de dérivés du benzothiopyrano[4,3,2-cd]indazole ayant sous leur forme de base libre la formule structurelle 2:

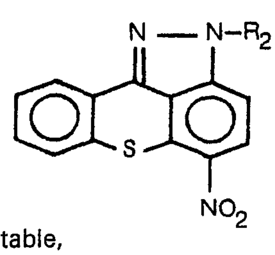

2

et son sel pharmaceutiquement acceptable,

dans laquelle: $R_2$ représente ANR'R'', A représente une chaîne alkylène droite ou ramifiée renfermant de 2 à 5 atomes de carbone, éventuellement substituée par un hydroxyle; R' et R'' représentant un atome d'hydrogène ou un radical alkyle à chaîne droite ou ramifiée renfermant de 1 à 4 atomes de carbone, éventuellement substitué par un hydroxyle, R' et R'' pris ensemble représentant également:

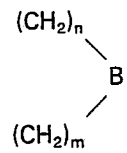

dans lequel : n et m sont chacun égaux à 2 ou 3; et B représente une liaison directe ou O, S, ou NR''', dans lequel R''' représente un atome d'hydrogène ou un radical alkyle à chaîne droite ou ramifiée renfermant de 1 à 4 atomes de carbone, éventuellement substitué par un groupe hydroxyle.

5. Procédé selon la revendication 1, 2 ou 3 pour la préparation de dérivés du benzothiopyrano[4,3,2-cd]indazole ayant sous leur forme de base libre la formule structurelle 4a:

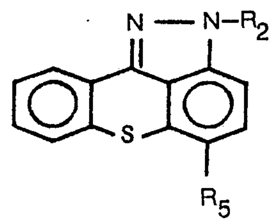

4a

et leurs sels pharmaceutiquement acceptables;
dans laquelle: $R_2$ représente ANR'R'', A représentant une chaîne alkylène droite ou ramifiée renfermant de 2 à 5 atomes de carbone, éventuellement substituée par un hydroxyle; R' et R'' représentant un atome d'hydrogène ou un radical alkyle à chaîne droite ou ramifiée renfermant de 1 à 4 atomes de carbone, éventuellement substitué par un hydroxyle, R' et R'' pris ensemble représentant également:

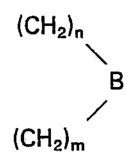

dans lequel: n et m sont chacun égaux à 2 ou 3; et B représente une liaison directe ou O, S, ou NR''', R''' représentant un atome d'hydrogène ou un radical alkyle à chaîne droite ou ramifiée renfermant de 1 à 4 atomes de carbone, éventuellement substitué par un hydroxyle; et dans laquelle: $R_5$ représente un groupe nitro, $NH_2$,

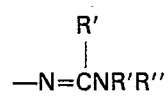

NHR', ou $NR'R_2$ ou NR'R'''' où R'''' représente un radical acyle renfermant de 1 à 4 atomes de carbone, un radical chloroacétyle, ou

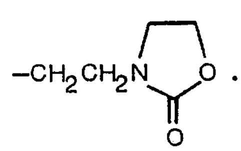

6. Procédé selon la revendication 1, 2, 3 ou 5, dans lequel $R_2$ représente le radical diéthylaminoéthyle et $R_5$ représente $NHCH_2CH_2NHCH_2CH_2OH$.

7. Procédé selon la revendication 1, 2, 3 ou 5, dans lequel $R_2$ représente $CH_2CH_2NHCH_2CH_2OH$ et $R_5$ représente $NHCH_2CH_2NHCH_2CH_2OH$.

8. Procédé selon la revendication 1 ou 5, dans lequel $R_2$ représente le radical diéthylaminoéthyle et $R_5$ représente le radical aminoéthylamino.

9. Procédé selon la revendication 1, 2, 3 ou 5, pour la préparation de dérivés du benzothiopyrano[4,3,2-cd]indazole ayant sous leur forme de base libre la formule structurelle 6:

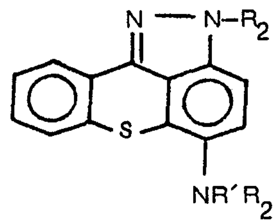

6

et leurs sels pharmaceutiquement acceptables,
dans laquelle: $R_2$ représente ANR'R'', A représentant une chaîne alkylène droite ou ramifiée renfermant de 2 à 5 atomes de carbone, éventuellement substituée par un hydroxyle; R' et R'' représentant un atome d'hydrogène ou un radical alkyle à chaîne droite ou ramifiée renfermant de 1 à 4 atomes de carbone, éventuellement substitué par un hydroxyle, R' et R'' pris ensemble représentant également:

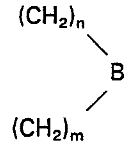

dans lequel: n et m sont chacun égaux à 2 ou 3; et B représente une liaison directe ou O, S, ou NR''', dans lequel R''' représente un atome d'hydrogène ou un radical alkyle à chaîne droite ou ramifiée renfermant de 1 à 4 atomes de carbone, éventuellement substitué par un hydroxyle.

10. Procédé selon la revendication 1, 2, 3 ou 5 pour la préparation de:
. 2-{[2-[[2-[2-(diéthylamino)éthyl]-2H(1)benzothiopyrano[4,3,2-cd]indazol-5-yl]amino]éthyl]amino}éthanol;
. 2-{[2-[5-[[2-[(2-hydroxyéthyl)amino]éthyl]amino]-2H(1)benzothiopyrano[4,3,2-cd]indazol-2-yl]éthyl]amino}éthanol;
. N-[2-[2-(diéthylamino)éthylamino)éthyl]-2H(1)benzothiopyrano[4,3,2-cd]indazol-5-yl]-1,2-éthanediamine;
. N,N-diéthyl-5-nitro-2H(1)benzothiopyrano[4,3,2-cd]indazole-2-éthanamine;
. 5-nitro-2H(1)benzothiopyrano[4,3,2-cd]indazole-2-éthanamine;
. 2-{[2-(5-nitro-2H(1)benzothiopyrano[4,3,2-cd]indazol-2-yl)éthyl]amino}éthanol;
. 5-amino-N,N-diéthyl-2H(1)benzothiopyrano[4,3,2-cd]indazole-2-éthanamine;
. N,N-diéthyl-9-méthoxy-5-nitro-2H(1)benzothiopyrano[4,3,2-cd]indazole-2-éthanamine;
. N-{2-[2-(diéthylamino)éthyl]-2H(1)benzothiopyrano[4,3,2-cd]indazol-5-yl}-1,3-propanediamine;
. 5-amino-N,N-diméthyl-2H(1)benzothiopyrano[4,3,2-cd]indazole-2-propanamine;
. N'-{2-[2-(diéthylamino)éthyl]-2H(1)benzothiopyrano[4,3,2-cd]indazol-5-yl}-N,N-diméthylméthanimidamide;
. 3-{2-[[2-[2-(diéthylamino)éthyl]-2H(1)benzothiopyrano[4,3,2-cd]indazol-5-yl]amino]éthyl}-2-oxazolidinone;
. N,N-diméthyl-5-nitro-2H(1)benzothiopyrano[4,3,2-cd]indazole-2-propanamine;
. 2-{[2-(5-amino-2H(1)benzothiopyrano[4,3,2-cd]indazol-2-yl)éthyl]amino}éthanol;
. 2-{[2-[[2-(2-aminoéthyl)-2H(1)benzothiopyrano[4,3,2-cd]indazol-5-yl]amino]éthyl]amino}éthanol;
. 5-amino-2-{2-(diethylamino)éthyl}-2H(1)benzothiopyrano[4,3,2-cd]indazol-9-ol;
. 2-{2-(diéthylamino)éthyl}-5-{[2-[(2-hydroxyéthyl)amino]éthyl]amino}-2H(1)benzothiopyrano[4,3,2-cd]indazol-9-ol;
. N-{2-[3-(diméthylamino)propyl]-2H(1)benzothiopyrano[4,3,2-cd]indazol-5-yl}-1,2-éthanediamine;
. 3-{2-[[2-[2-(diéthylamino)éthyl]-9-hydroxy-2H(1)benzothiopyrano[4,3,2-cd]indazol-5-yl];
. 2-{2-(diéthylamino)éthyl}-5-{[2-[(2-hydroxyéthyl)amino]éthyl]amino}-2H(1)benzothiopyrano[4,3,2-cd]indazol-9-ol;
. N,N-diéthyl-7-méthoxy-5-nitro-2H(1)benzothiopyrano[4,3,2-cd]indazole-2-éthanamine;
. 5-amino-N,N-diéthyl-7-méthoxy-2H(1)benzothiopyrano[4,3,2-cd]indazole-2-éthanamine;
. bromhydrate de N-{2-[2-(diéthylamino)éthyl]-7-méthoxy-2H(1)benzothiopyrano[4,3,2-cd]indazol-5-yl}-1,2-éthanediamine;
. bromhydrate de 5-{2-(aminoéthyl)amino}-2-[2-(diéthylamino)éthyl]-2H(1)benzothiopyrano[4,3,2-cd]indazol-7-ol;
. 5-amino-2-{2-(diéthylamino)éthyl}-2H(1)benzothiopyrano[4,3,2-cd]indazol-7-ol;
. N,N-diéthyl-8-méthoxy-5-nitro-2H(1)benzothiopyrano[4,3,2-cd]indazole-2-éthanamine;
. 5-amino-N,N-diéthyl-8-méthoxy-2H(1)benzothiopyrano[4,3,2-cd]indazole-2-éthanamine;
. bromhydrate de N-{2-[2-(diéthylamino)éthyl]-8-méthoxy-2H(1)benzothiopyrano[4,3,2-cd]indazol-5-yl}-1,2-éthanediamine;

. bromhydrate de 5-{2-(aminoéthyl)amino}-2-[2-(diéthylamino)éthyl]-2H(1)benzothiopyrano[4,3,2-cd]indazol-8-ol;

. 5-amino-2-{2-(diéthylamino)éthyl}-2H(1)benzothiopyrano[4,3,2-cd]indazol-8-ol;

. N,N-diéthyl-10-méthoxy-5-nitro-2H(1)benzothiopyrano[4,3,2-cd]indazole-2-éthanamine;

. 5-amino-N,N-diéthyl-10-méthoxy-2H(1)benzothiopyrano[4,3,2-cd]indazole-2-éthanamine;

. bromhydrate de N-{2-[2-(diéthylamino)éthyl]-10-méthoxy-2H(1)benzothiopyrano[4,3,2-cd]indazol-5-yl}-1,2-éthanediamine;

. bromhydrate de 5-{2-(aminoéthyl)amino}-2-[2-(diéthylamino)éthyl]-2H(1)benzothiopyrano[4,3,2-cd]indazol-10-ol;

. 5-amino-2-{2-(diéthylamino)éthyl}-2H(1)benzothiopyrano[4,3,2-cd]indazol-10-ol;

. N,N-diéthyl-3-méthoxy-5-nitro-2H(1)benzothiopyrano[4,3,2-cd]indazole-2-éthanamine;

. 5-amino-N,N-diéthyl-3-méthoxy-2H(1)benzothiopyrano[4,3,2-cd]indazole-2-éthanamine;

. bromhydrate de N-{2-[2-(diéthylamino)éthyl]-3-méthoxy-2H(1)benzothiopyrano[4,3,2-cd]indazol-5-yl}-1,2-éthanediamine;

. bromhydrate de 5-{2-(aminoéthyl)amino}-2-[2-(diéthylamino)éthyl]-2H(1)benzothiopyrano[4,3,2-cd]indazol-3-ol;

. 5-amino-2-{2-(diéthylamino)éthyl}-2H(1)benzothiopyrano[4,3,2-cd]indazol-3-ol;

. N,N-diéthyl-3,9-diméthoxy-5-nitro-2H(1)benzothiopyrano[4,3,2-cd]indazole-2-éthanamine;

. 5-amino-N,N-diéthyl-3,9-diméthoxy-2H(1)benzothiopyrano[4,3,2-cd]indazole-2-éthanamine;

. bromhydrate de N-{2-[2-(diéthylamino)éthyl]-3,9-diméthoxy-2H(1)benzothiopyrano[4,3,2-cd]indazol-5-yl}-1,2-éthanediamine;

. bromhydrate de 5-{2-(aminoéthyl)amino}-2-[2-(diéthylamino)éthyl]-2H(1)benzothiopyrano[4,3,2-cd]indazol-3,9-diol;

5-amino-2-{2-(diethylamino)éthyl}-2H(1)benzothiopyrano[4,3,2-cd]indazol-3,9-diol;

. le 2- {[2-[5-[[2-(diethylamino)éthyl]amino]-2H(1)benzothiopyrano[4,3,2-cd]indazol-2-yl]éthyl]amino}éthanol;

et les sels pharmaceutiquement acceptables de ces composés.

11. Procédé selon la revendication 1 pour la préparation de dérivés du benzothiopyrano[4,3,2-cd]indazole ayant sous leur forme de base libre la formule structurelle 16:

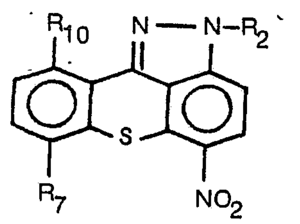

16

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

12. Procédé selon la revendication 1 pour la préparation de dérivés du benzothiopyrano[4,3,2-cd]indazole, ayant sous leur forme de base libre la formule structurelle 17:

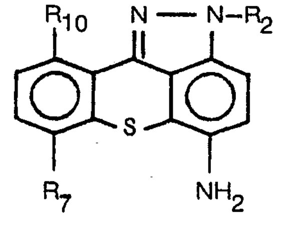

17

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

13. Procédé selon la revendication 1 ou 2 pour la préparation de dérivés du benzothiopyrano[4,3,2-cd]indazole ayant sous leur forme de base libre la formule structurelle 18:

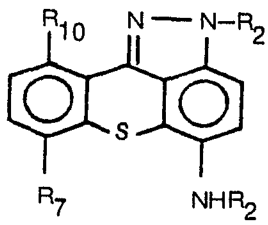

18

et leurs sels pharmaceutiquement acceptables;
dans laquelle: $R_2$ est défini comme à la revendication 1; et $R_7$ et $R_{10}$ représentent un atome d'hydrogène, un groupe hydroxy ou alkoxy ayant de 1 à 4 atomes de carbone, au moins l'un des symboles $R_7$ et $R_{10}$ représentant un groupe hydroxy ou alkoxy ayant de 1 à 4 atomes de carbone.

14. Procédé selon la revendication 1 pour la préparation de dérivés du benzothiopyrano[4,3,2-cd]indazole ayant sous leur forme de base libre la formule structurelle 24:

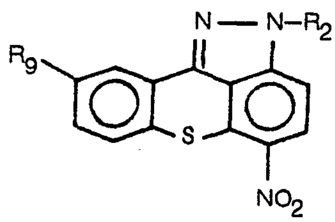

24

et leurs sels pharmaceutiquement acceptables,
dans laquelle: $R_9$ représente un groupe ou alkoxy renfermant de 1 à 4 atomes de carbone; et $R_2$ est défini comme à la revendication 1.

15. Procédé selon la revendication 1 pour la préparation de dérivés du benzothiopyrano[4,3,2-cd]indazole ayant sous leur forme de base libre la formule structurelle 25:

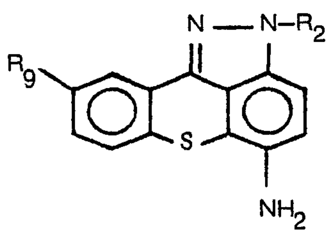

25

et leurs sels pharmaceutiquement acceptables,
dans laquelle: $R_9$ représente un groupe ou alkoxy renfermant de 1 à 4 atomes de carbone; et $R_2$ est défini comme à la revendication 1.

16. Procédé selon la revendication 1 ou 2 pour la préparation de dérivés du benzothiopyrano[4,3,2-cd]indazole ayant sous leur forme de base libre la formule structurelle 26:

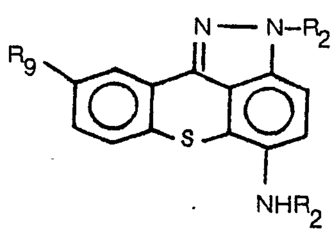

26

et leurs sels pharmaceutiquement acceptables,
dans laquelle: $R_9$ représente un groupe hydroxy ou alkoxy renfermant de 1 à 4 atomes de carbone; et $R_2$ est défini comme à la revendication 1.

17. Procédé de préparation d'une composition pharmaceutique, ce procédé consistant à combiner un composé de formule structurelle 1 selon la revendication 1 avec un support pharmaceutiquement acceptable.

18. Procédé de préparation d'une composition pharmaceutique, ce procédé consistant à combiner un composé de formule structurelle 2 selon la revendication 4 avec un support pharmaceutiquement acceptable.

19. Procédé de préparation d'une composition pharmaceutique, ce procédé consistant à combiner un composé de formule structurelle 4a selon la revendication 5 avec un support pharmaceutiquement acceptable.

20. Procédé de préparation d'une composition pharmaceutique, ce procédé consistant à combiner un composé de formule structurelle 6 selon la revendication 9 avec un support pharmaceutiquement acceptable.